# EUROPEAN PATENT SPECIFICATION

(11) **EP 2 627 673 B1**
(45) Date of publication and mention of the grant of the patent: **26.07.2017**
(21) Application number: 11769881.1
(22) Date of filing: 14.10.2011
(51) Int. Cl.: C07K 16/24, G01N 33/577, A61P 37/08, A61K 39/395

(54) **THERAPIES FOR IMPROVING PULMONARY FUNCTION**
THERAPIEN ZUR VERBESSERUNG DER LUNGENFUNKTION
THÉRAPIES CONÇUES POUR AMÉLIORER LA FONCTION PULMONAIRE

(30) Priority: 18.07.2011 US 201161508723 P; 15.10.2010 US 393677 P
(43) Date of publication of application: 21.08.2013
(73) Proprietor: Medimmune Limited, Cambridge, Cambridgeshire CB21 6GH (GB)
(72) Inventor: FAGGIONI, Raffaella, Hayward California 94566 (US); MAY, Richard, Cambridge Cambridgeshire CB21 6GH (GB); KELL, Chris, Cambridge Cambridgeshire CB21 6GH (GB); MOLFINO, Nestor, Potomac Maryland 20854 (US); ROSKOS, Lorin, Gaithersburg Maryland 20878 (US)
(74) Representative: Meyrial, Valérie Marie-Noëlle
(86) International application number: PCT/EP2011/067947
(87) International publication number: WO 2012/049278

(56) References cited:
- WO-A2-2006/055638
- WO-A2-2007/080174
- GB-A- 2 403 952
- BLEASE KATE: "Therapeutics targeting IL-13 for the treatment of pulmonary inflammation and airway remodeling", CURRENT OPINION IN INVESTIGATIONAL DRUGS, PHARMAPRESS, US, vol. 9, no. 11, 1 November 2008 (2008-11-01), pages 1180-1184, XP008122454, ISSN: 1472-4472
- SINGH DAVE ET AL: "A phase 1 study evaluating the pharmacokinetics, safety and tolerability of repeat dosing with a human IL-13 antibody (CAT-354) in subjects with asthma", BMC PULMONARY MEDICINE, BIOMED CENTRAL, LONDON, GB, vol. 10, no. 1, 8 January 2010 (2010-01-08), page 3, XP021067289, ISSN: 1471-2466
- MedImmune,NCT00873860: "A Phase 2a, Randomized, Double-blind, Placebo-Controlled, Parallel-Arm, Multicenter Study to Evaluate the Efficacy and Safety of CAT-354, on Asthma Control in Adults", clinical trials.gov , 24 August 2010 (2010-08-24), pages 1-5, XP002665050, usa Retrieved from the Internet: URL:http://clinicaltrials.gov/archive/NCT0 0873860/2010_08_24 [retrieved on 2011-11-29]
- MedImmune: "A Phase 2b, Randomized, Double-blind Study to Evaluate the Efficacy of Tralokinumab in Adults With Uncontrolled, Severe Asthma", clinical trials.gov,NCT01402986 , 26 July 2011 (2011-07-26), pages 1-4, XP002665051, usa Retrieved from the Internet: URL:http://clinicaltrials.gov/archive/NCT0 1402986/2011_07_26 [retrieved on 2011-12-05]
- GAOYUN YANG ET AL: "Therapeutic dosing with anti-interleukin-13 monoclonal antibody inhibits asthma progression in mice", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 313, no. 1, 1 April 2005 (2005-04-01) , pages 8-15, XP002644930, ISSN: 0022-3565, DOI: 10.1124/JPET.104.076133 [retrieved on 2005-01-11]
- YANG G ET AL: "Anti-IL-13 monoclonal antibody inhibits airway hyperresponsiveness, inflammation and airway remodeling", CYTOKINE, ACADEMIC PRESS LTD, PHILADELPHIA, PA, US, vol. 28, no. 6, 21 December 2004 (2004-12-21), pages 224-232, XP004654755, ISSN: 1043-4666, DOI: 10.1016/J.CYTO.2004.08.007
- LACHMAN H ET AL: "Efficacy and Safety of Canakinumab (ACZ885), a Fully Human Anti-Interleukin-1b-Antibody, in Cryopyrin Associated Periodic Fever Syndrome Results of a Multicenter, Randomized, Double-blind, Phase III Study", JOURNAL OF ALLERGY AND CLINICAL IMMUNOLOGY, MOSBY, INC, US, vol. 123, no. 3, 1 March 2009 (2009-03-01) , page 732, XP026033133, ISSN: 0091-6749, DOI: 10.1016/J.JACI.2009.01.038 [retrieved on 2009-03-01]

## Description

### Field of the invention.

The invention relates to the use of an IL-13 antagonist, in particular an anti-IL-13 antibody, for improving, maintaining or reducing the rate of decline of pulmonary function, as assessed by a spirometric method. The invention further relates to the use of an IL-13 antagonist for the treatment of asthma in a subject with detectable IL-13 in sputum. The invention further relates to a method for identification of a subject with asthma likely to benefit from treatment with an IL-13 antagonist, said subject having detectable IL-13 in sputum.

### Background to the invention

Respiratory disorders, such as asthma, chronic obstructive pulmonary disease (COPD) and idiopathic pulmonary fibrosis are associated with a decrease in pulmonary function; this impairment of pulmonary function can be measured by spirometry. Measurement of lung function provides an assessment of the severity of airflow limitation, its reversibility and its variability in diseases such as asthma, COPD, IPF and bronchiectasis important measures of pulmonary function these diseases are FEV₁ (forced expiratory volume in the first second), this is the volume of air that can be forced out in one second after taking a deep breath, and FVC (forced vital capacity).

Asthma is a chronic airway disorder that is characterized by variable and reversible airway obstruction, airway inflammation, excessive mucus production and airway hyper-responsiveness (AHR) that leads to episodes of wheezing, breathlessness, chest tightness and coughing. Progressive pathological airway remodelling and scarring may occur in persistent asthma resulting in partially reversible or irreversible airway obstruction.

Airways hyper-responsiveness (AHR) is the exaggerated constriction of the airways to non-specific stimuli such as cold air. Both AHR and mucus overproduction are thought to be responsible for the variable airway obstruction that leads to the shortness of breath characteristic of asthma attacks (exacerbations) and which is responsible for the mortality associated with this disease (around 2000 deaths/year in the United Kingdom).

Current British Thoracic Society (BTS) and Global Initiative for Asthma (GINA) guidelines suggest a stepwise approach to the treatment of asthma. Most patients with asthma have mild-to-moderate disease that is controlled at present by "standard of care" therapies: inhaled corticosteroids combined with β₂-adrenoceptor agonists or leukotriene inhibitors. However, patients may not comply with the treatment regime which reduces the effectiveness of treatment. In more severe disease, long-term treatment with oral corticosteroids may be required, but is associated with side effects such as osteoporosis, diabetes and oral candidiasis.

Although conventional therapies alleviate mild-to-moderate disease in many patients, in a subset of asthmatic patients (approximately 25% of the moderate-to-severe population), asthma continues to be poorly controlled in terms of ongoing symptoms, frequent exacerbations, persistent and variable airway obstruction and frequent requirement for β₂-agonists, despite adequate treatment. This results in considerable impact on quality of life, disproportionate use of healthcare resources, and adverse effects from regular systemic steroid use. Hospitalization of these patients, who represent around 6% of the UK asthma population, as a result of severe exacerbations accounts for the majority of the significant economic burden of asthma on healthcare authorities. Thus, there is a clear need for new treatments for asthma, particularly in subjects with more severe asthmatic disease, who often gain very limited benefit from either higher doses of inhaled or oral corticosteroids recommended by asthma guidelines. Additionally, there is a need for new treatments for children with asthma, as treatment with high dose corticosteroids is associated with reduced growth rates.

It is believed that the pathology of asthma is caused by ongoing Th2 lymphocyte mediated inflammation that results from inappropriate responses of the immune system to harmless antigens. Interleukin-13 (IL-13) has emerged as a key pleiotropic Th2 cytokine that contributes to the development and maintenance of all aspects of the asthma phenotype. Human Interleukin (IL) -13 is a 114 amino acid protein with an unmodified molecular mass of approximately 12 kDa. IL-13 is most closely related to IL-4 with which it shares 30% sequence similarity at the amino acid level. IL-13 shares receptor components and many biological properties with interleukin 4 (IL-4). IL-13 receptors (heterodimers of IL-13Rα1 and IL-4Rα) are expressed on the leukocyte subsets (eosinophils, neutrophils, B cells, NKT cells) and structural cells (epithelial cells, fibroblasts, smooth muscle) considered relevant in asthma. The action of IL-13 on these cells is to stimulate processes that promote airway hyperresponsiveness, inflammation, and underlying airway structural changes including fibrosis and mucus hypersecretion that are cardinal features of asthma (Hershey, 2003).

IL-13 mRNA or protein levels are elevated in bronchial biopsies, sputum, and BAL fluid from asthmatics compared to control subjects (Humbert et al., 1997; Kotsimbos et al., 1996; Komai-Koma et al., 2001; Naseer et al., 1997) and further increased in BAL samples from allergen challenged asthmatics (Huang et al., 1995; Kroegel et al., 1996). IL-13 protein expression in the lung is also correlated with both asthma severity and control. Mild steroid-naive asthmatics have upregulated IL-13 in the sputum compared to "normals", as do severe asthmatics who have poor control despite standard of care up to, and including, oral corticosteroids; moderate asthmatics (well controlled by inhaled corticosteroids) are not reported to have upregulated IL-13 expression (Saha et al., 2008).

Genetic polymorphisms in the IL-13 pathway are also associated with both atopy and asthma; IL-13 Q130R has been associated with asthma, atopy, and raised serum IgE (Heinzmann et al., 2000; Liu et al., 2000; Kauppi et al., 2001) activating the signaling IL-13 receptor (Rα1) and downstream functions more efficiently, whilst binding to the decoy receptor (Ra2) less efficiently, than wild-type IL-13 (Vladich et al., 2005). Similar polymorphisms associated with risk of atopy and asthma have been observed in the IL-13 promoter (Howard et al., 2001 and van der Pouw Kraan et al., 1999), IL4Rα (Howard et al., 2002), and in the key signalling molecule downstream of the IL-13 receptor - STAT6 (Tamura et al., 2003). The IL-13 variant (Q130R) and antibodies to this variant are discussed in WO 01/62933.

Animal model data from models mimicking aspects of human asthma support the hypothesis that IL 13 is a key mediator in the development and maintenance of the asthma phenotype. Administration of recombinant IL 13 to the airway of allergen-naive mice results in AHR, airway inflammation, and mucus production (Wills-Karp et al., 1998; Grunig et al., 1998; Venkayya et al., 2002). Similar pathologies are seen in transgenic mice in which IL-13 is selectively overexpressed in the lung although longer-term exposure to IL-13 also results in fibrosis (Zhu et al., 1999). The same 'asthmatic' phenotype occurs following allergen challenge in sensitised mice and is also associated with IL-13. Soluble IL-13Rα2, a potent IL-13 neutraliser, has been shown to inhibit AHR, mucus hypersecretion, and pulmonary inflammation (Wills-Karp et al., 1998; Grunig et al., 1998). NHP segmental antigen challenge studies have also shown an anti-inflammatory effect for an IL-13 neutralising antibody (Bree et al., 2007).

Given the range of cells involved in asthma on which IL-13 is known to act, and the pathogenic functions ascribed to this interleukin, a significant role for IL-13 in asthma can be expected. Coupled with the evidence of a relationship between IL-13 expression and disease severity in patients (Saha et al., 2008), neutralisation of IL-13 is a credible approach to the treatment of asthma.

Chronic Obstructive Pulmonary Disease (COPD) includes patient populations with varying degrees of chronic bronchitis, small airway disease and emphysema and is characterized by progressive irreversible lung function decline that responds poorly to current asthma based therapies. The incidence of COPD has risen dramatically in recent years to become the fourth leading cause of death worldwide (World Health Organization). COPD therefore represents a large unmet medical need. The underlying causes of COPD remain poorly understood. It has been proposed that there is a common susceptibility to COPD and asthma and therefore, that similar mechanisms may contribute to the pathogenesis of both disorders. Over-expression of IL-13 in the mouse lung has been demonstrated to cause emphysema, elevated mucus production and inflammation, reflecting aspects of human COPD. Furthermore, AHR, an IL-13 dependent response in murine models of allergic inflammation, has been shown to be predictive of lung function decline in smokers. A link has also been established between an IL-13 promoter polymorphism and susceptibility to develop COPD.

The signs are, therefore, that IL-13 plays an important role in the pathogenesis of COPD, particularly in patients with asthma-like features, including AHR and eosinophilia. The mRNA levels of IL-13 have been shown to be higher in autopsy tissue samples from subjects with a history of COPD when compared to lung samples from subjects with no reported lung disease. In another study, raised levels of IL-13 were demonstrated by immunohistochemistry in peripheral lung sections from COPD patients.

IL-13 has been associated with other fibrotic conditions. Increased levels of IL-13 have been measured in the serum of patients with systemic sclerosis and in BAL samples from patients affected with other forms of pulmonary fibrosis. Correspondingly, over-expression of IL-13 in the mouse lung has been reported to result in pronounced fibrosis. The contribution of IL-13 to fibrosis in tissues other than the lung has been extensively studied in a mouse model of parasite-induced liver fibrosis.

Tralokinumab (an IgG4 monoclonal antibody) is a highly potent and specific IL-13-neutralising antibody, with a 165pM affinity for human IL-13, that neutralises human and cynomolgus IL-13 with near equivalent potency, but does not neutralise mouse IL-13, nor the most homologous cytokine to IL-13, IL-4. CAT-354 also neutralises the variant IL-13, Q130R, which has been associated with atopy and asthma as well as endogenous (mammalian glycosylated) human IL-13. Tralokinumab (BAK502G9) is described in International Patent Application Publication No. WO 2005/007699.

Blease (2008) describes blocking of IL-13 for the treatment of pulmonary inflammation and airway remodelling.

WO 2006/055638, WO 2007/080174 and GB 2403952 describe different IL-13 antibodies for the treatment of various pulmonary disorders.

Singh et al. (2010) describes a phase 1 study of IL-13 antibody CAT-354 in subjects with asthma.

A Phase 2a, randomized, double-blind, placebo-controlled, parallel-arm, multicentre study, number NCT00873860 (2010.08.24, p. 1-5), aimed at evaluating the efficacy and safety of CAT-354 on asthma control in adults (available from clinical trials.gov site).

It is an object of the invention to provide IL-13 antagonists, in particular anti-IL-13 antibodies such as tralokinumab, or IL-13 neutralizing fragments thereof, for improvement, maintenance or reduction in decline of pulmonary function, in particular as assessed by FEV₁, or FVC, in subjects with impaired pulmonary function, which may be due to asthma, COPD or pulmonary fibrosis. It is a further object of the invention to provide an IL-13 antagonist for the treatment of asthma in a subject, wherein said subject has upregulated airway IL-13, for example, based on detectable IL-13 in sputum. It is a further object of the invention to provide a method for identification of a subject with asthma likely to benefit from treatment with an IL-13 antagonist, wherein said subject has upregulated airway IL-13, for example, based on detectable IL-13 in sputum.

### Statement of Invention

The invention provides an IL-13 antagonist for use in improving pulmonary function in a subject with impaired pulmonary function.

An object of the invention is an IL-13 antagonist for use in improving pulmonary function in a subject with impaired pulmonary function, wherein the IL-13 antagonist is an anti-human-IL-13 antibody or a human-IL-13-binding fragment thereof, selected from the group consisting of:
(a) an anti-human-IL-13 antibody or a human-IL-13 binding fragment thereof comprising a VH domain comprising HCDR1, HCDR2 and HCDR3 and a VL domain comprising LCDR1, LCDR2 and LCDR3, wherein HCDR1 has the amino acid sequence of SEQ ID NO: 7, HCDR2 has the amino acid sequence of SEQ ID NO: 8, HCDR3 has the amino acid sequence of SEQ ID NO: 9, LCDR1 has the amino acid sequence of SEQ ID NO: 10, LCDR2 has the amino acid sequence of SEQ ID NO: 11, and LCDR3 has the amino acid sequence of SEQ ID NO: 12;
(b) an anti-human-IL-13 antibody, or a human-IL-13-binding fragment thereof comprising a VH domain having the amino acid sequence of SEQ ID NO: 15 and a VL domain having the amino acid sequence of SEQ ID NO: 16; and
(c) an anti-human-IL-13 antibody or a human-IL-13-binding fragment thereof according to (a) or (b), further comprising a human IgG4 constant domain;
wherein improvement in pulmonary function is an improvement in Forced Expiratory Volume in One Second (FEV1) score of between 5% and 18% over a baseline FEV1 measurement taken before administering the anti-human-IL-13 antibody or a human-IL-13-binding fragment thereof, and wherein the IL-13 antibody or IL-13 binding fragment thereof is administered subcutaneously at a dose between 100 and 600 mg.

Also provided is the IL-13 antagonist for use according to the invention, wherein said antagonist is selected from the group consisting of: an immunoglobulin molecule, a monoclonal antibody, a chimeric antibody, a CDR-grafted antibody, a humanized antibody, a Fab, a Fab', a F(ab')2, a Fv, a disulfide linked Fv, a scFv, a single domain antibody, a diabody, a multispecific antibody, a dual-specific antibody, and a bispecific antibody.

Also provided is the IL-13 antagonist for use according to the invention, wherein the subject has impaired pulmonary function associated with asthma, COPD, or idiopathic pulmonary fibrosis.

Provided herewith is the IL-13 antagonist for use according to the invention, wherein the subject has impaired pulmonary function associated with asthma selected from atopic asthma, non-atopic asthma, moderate to severe asthma, moderate to severe asthma uncontrolled by inhaled or oral corticosteroids and asthma at GINA score 5, 4, 3, 2, or 1.

The invention also provides the IL-13 antagonist for use according to the invention, wherein the subject has impaired pulmonary function associated with asthma and is undergoing concomitant therapy with one or more further therapeutic selected from an inhaled corticosteroid, long-acting beta2 antagonist (LABA), theophylline, a leukotriene antagonist, and an oral corticosteroid.

Also provided is the IL-13 antagonist for use according to the invention, wherein the IL-13 antibody or IL-13 binding fragment thereof is administered subcutaneously at a dose selected from the group consisting of 150, 300, and 600 mg.

Also provided is the IL-13 antagonist for use according to the invention, wherein the IL-13 antibody or IL-13-binding fragment thereof is administered at a frequency selected from every 1, 2, 3, 4, 6, 8, 10 and 12 weeks.

Provided herewith is the IL-13 antagonist for use according to the invention, wherein an improvement in pulmonary function in asthma subjects can be detected by 15 days following initiation of treatment.

An embodiment of the invention provides the IL-13 antagonist for use according to the invention, wherein an improvement in pulmonary function in asthma subjects can be detected at least 12 weeks after cessation of treatment.

Also provided herewith is the IL-13 antagonist for use according to any one of the preceding embodiments of the invention, wherein the IL-13 antibody or IL-13-binding fragment thereof is administered by subcutaneous administration, at a dose of 300mg and at dosing intervals of 2 weeks or 4 weeks.

Also provided by the invention is the IL-13 antagonist for use according to any one of the preceding embodiments of the invention, wherein the improvement in pulmonary function is an improvement in FEV1 score of between 8% and 16% over a baseline FEV1 measurement taken before subcutaneously administering the anti-human-IL-13 antibody or a human-IL-13-binding fragment thereof at two week or four week intervals.

Also provided herewith is the IL-13 antagonist for use according to any one of the preceding embodiments of the invention, wherein the improvement in pulmonary function is an improvement in FEV1 score of between 12% and 18% over a baseline FEV1 measurement taken before administering the anti-human-IL-13 antibody or a human-IL-13-binding fragment thereof.

Provided herewith is the IL-13 antagonist for use according to any one of the preceding embodiments of the invention, wherein the improvement in pulmonary function is an improvement in FEV1 score of 8% over a baseline FEV1 measurement taken before subcutaneously administering 150mg of the anti-human-IL-13 antibody or a human-IL-13-binding fragment thereof at two week intervals; or
wherein the improvement in pulmonary function is an improvement in FEV1 score of 13.3% over a baseline FEV1 measurement taken before subcutaneously administering 300mg of the anti-human-IL-13 antibody or a human-IL-13-binding fragment thereof at two week intervals; or
wherein the improvement in pulmonary function is an improvement in FEV1 score of 15.2% over a baseline FEV1 measurement taken before subcutaneously administering 600mg of the anti-human-IL-13 antibody or a human-IL-13-binding fragment thereof at two week intervals.

Provided herewith is the IL-13 antagonist for use according to any one of the preceding embodiments of the invention, wherein the IL-13 antagonist is an anti-human-IL-13 antibody or a human-IL-13 binding fragment thereof comprising a VH domain comprising HCDR1, HCDR2 and HCDR3 and a VL domain comprising LCDR1, LCDR2 and LCDR3, wherein HCDR1 has the amino acid sequence of SEQ ID NO: 7, HCDR2 has the amino acid sequence of SEQ ID NO: 8, HCDR3 has the amino acid sequence of SEQ ID NO: 9, LCDR1 has the amino acid sequence of SEQ ID NO: 10, LCDR2 has the amino acid sequence of SEQ ID NO: 11, and LCDR3 has the amino acid sequence of SEQ ID NO: 12.

Also provided herewith is the IL-13 antagonist for use according to any one of the preceding embodiments of the invention, wherein the IL-13 antagonist is an anti-human-IL-13 antibody or a human-IL-13 binding fragment thereof comprising a VH domain having the amino acid sequence of SEQ ID NO: 15 and a VL domain having the amino acid sequence of SEQ ID NO: 16.

Also provided herewith is the IL-13 antagonist for use according to any one of the preceding embodiments of the invention, wherein the improvement in pulmonary function is an improvement in FEV1 score of between 5% and 12% over a baseline FEV1 measurement taken before administering the anti-human-IL-13 antibody or a human-IL-13-binding fragment thereof.

Disclosed herewith is a method of treating a subject with impaired pulmonary function, said method comprising administering to said subject an IL-13 antagonist in an amount sufficient to improve, maintain or reduce the rate of decline of pulmonary function.

Disclosed herewith is the use of an IL-13 antagonist in the manufacture of a medicament for the treatment of impaired pulmonary function to improve, maintain or reduce the rate of decline of pulmonary function in a subject in need thereof.

Disclosed herewith is a method of improving, maintaining or reducing the rate of decline in pulmonary function in a subject with impaired pulmonary function, said method comprising administering to said subject an IL-13 antagonist.

Additionally disclosed herewith is a composition for the treatment of impaired pulmonary function to improve, maintain or reduce the rate of decline of pulmonary function in a subject with impaired pulmonary function, said composition comprising an IL-13 antagonist and a pharmaceutically acceptable excipient.

The invention provides an IL-13 antagonist for use in the treatment of asthma in a subject, wherein said subject has upregulated airway IL-13, for example, based on detectable IL-13 in sputum.

Disclosed herewith is a method for treating asthma in a subject, wherein said subject has detectable IL-13 in sputum, said method comprising administering to said subject an IL-13 antagonist in an amount sufficient to ameliorate asthma symptoms.

Disclosed is the use of an IL-13 antagonist in the manufacture of a medicament for the treatment of asthma in a subject, wherein said subject has detectable IL-13 in sputum.

Also disclosed herewith is a method for the identification of a subject with asthma likely to benefit from treatment with an IL-13 antagonist, said method comprising testing for the presence of IL-13 in a sample of sputum from said subject, wherein a subject with detectable IL-13 in sputum is likely to benefit from treatment with an IL-13 antagonist.

A beneficial response to treatment of asthma may be assessed by consideration of asthma symptoms, e.g. by an ACQ method, such as ACQ-6, and / or by spirometric assessments as described herein. Subjects that benefit from treatment with an IL-13 antagonist experience an amelioration of their asthma symptoms. As described herein, subjects with asthma who had detectable IL-13 in their sputum experienced an amelioration of asthma symptoms following treatment with an IL-13 antagonist. Methods of treatment and uses described herein may comprise a step, performed prior to treatment of a subject with an IL-13 antagonist, whereby a sample of sputum from the subject is tested for the presence of IL-13. The inventors have found that presence of IL-13 in sputum defines a sub-set of asthmatics in which IL-13 antagonist therapy is particularly beneficial, as described herein.

Disclosed herewith is a composition for the treatment of asthma in a subject, wherein said subject has detectable IL-13 in sputum, said composition comprising an IL-13 antagonist and a pharmaceutically acceptable excipient.

Thus pharmaceutical compositions disclosed herewith, and for use in accordance with the present invention, may comprise, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. intravenous. For injection, the active ingredient will be in the form of a parenterally acceptable aqueous solution which is pyrogen-free and has suitable pH, isotonicity and stability. Preservatives, stabilisers, buffers, antioxidants and/or other additives may be included, as required.

Said compositions may be formulated in liquid or solid forms. Solid forms may be provided for reconstitution prior to administration by intravenous or subcutaneous injection, or for inhalation. The composition employed will depend on the physicochemical properties of the molecule and the route of delivery. Formulations may include excipients, or combinations of excipients, for example: sugars, amino acids and surfactants. Liquid formulations may include a wide range of antibody concentrations and pH. Solid formulations may be produced by lyophilisation, spray drying, or drying by supercritical fluid technology, for example. Formulations will depend upon the intended route of delivery: for example, formulations for pulmonary delivery may consist of particles with physical properties that ensure penetration into the deep lung upon inhalation.

In a particularly preferred embodiment, tralokinumab is formulated at 150 mg/mL in 50 mM sodium acetate, 85 mM sodium chloride, 0.01% (w/v) plant-derived polysorbate 80 at pH 5.5.

A composition for use in accordance with the invention may be administered alone or in combination with other treatments, either simultaneously or sequentially dependent upon the condition to be treated.

Treatment may be given by injection, for example, subcutaneously, or intravenously, or by inhalation. It is envisaged that treatment will not be restricted to use in the clinic. Therefore, subcutaneous injection using an administration device is envisaged.

Accordingly, also disclosed herewith is an administration device for the treatment of impaired pulmonary function to improve, maintain or reduce the rate of decline of pulmonary function in a subject in need thereof, said device comprising an IL-13 antagonist. An administration device may comprise a composition for the treatment of impaired pulmonary function to improve, maintain or reduce the rate of decline of pulmonary function in a subject in need thereof, said composition comprising an IL-13 antagonist and a pharmaceutically acceptable excipient.

Also disclosed herewith is an administration device for the treatment of asthma in a subject, wherein said subject has detectable IL-13 in sputum, said device comprising an IL-13 antagonist. An administration device may comprise a composition for the treatment of asthma in a subject, wherein said subject has detectable IL-13 in sputum, said composition comprising an IL-13 antagonist and a pharmaceutically acceptable excipient.

A detectable level of IL-13 in sputum may be the presence of ≥1pg/mL IL-13. The presence of IL-13 in sputum can be detected using an ELISA method. The limit of quantification using ELISA methods is about 1pg/mL, hence subjects with a concentration of IL-13 in sputum that is <1 pg/ may be deemed to not have detectable IL-13 in sputum. Methods suitable for detection of IL-13 in sputum include: western blot, proteomic techniques such as mass spectrometry, MALDI-TOF and 2D-DIGE; antibody- or receptor-based protein identification techniques, which include ELISAs in the format of Luminex® assays (Luminex Corp., Austin, TX, USA) based on xMAP technology (multi-analyte profiling beads) enabling the detection and quantitation of multiple RNA or protein targets simultaneously; the xMAP system combines a flow cytometer, fluorescent-dyed microspheres (beads), lasers and digital signal processing to efficiently allow multiplexing of up to 100 unique assays within a single sample and in the format of MSD® cytokine assays (Meso Scale Discovery, Gaithersburg, MD, USA) that employ electrochemiluminescent detection; sputum-driven cell-based assays to identify IL-13 in sputum, such as TF1 proliferation blocked with an IL-13 neutralising reagent or STAT-6 phosphorylation and measurement of downstream mediators, e.g. eotaxin, TARC, MCP4. The amount corresponding to a detectable level of IL-13 in sputum may vary due to the known limit of quantitation of a method known in the art such as any of those described above, or may be further refined based on improvement in the limits of quantitation of methods known in the art such as those described above, or other methods suitable to detect levels of a molecule such as IL-13.

An administration device in accordance with the invention may be selected from a pre-filled syringe and an auto-injector device for sub-cutaneous administration, which may be designed to permit "home" administration.

In respiratory diseases such as asthma, COPD, and IPF, pulmonary function tests (PFT) are used to assess lung function and quantify the amount of damage to the lungs; PFT can also be used to assess the progression of lung disease and the effectiveness of treatment. Spirometry testing is the most commonly used of all pulmonary function tests. It is a convenient non-invasive procedure, generally performed with a hand-held spirometer. It is normally the clinician's first choice when attempting to diagnose a respiratory problem. Spirometry requires the patient, after all air has been expelled, to inhale deeply; this is then followed by a rapid exhalation, so that all the air is expelled from the lungs. Results of spirometry tests vary, but are based on predicted values of a standardized, healthy population. Common Terminology in spirometry testing is as follows:
VC-Vital Capacity, this is the amount of air that can be forcibly exhaled from the lungs after a full inhalation.
FVC-Forced Vital Capacity, this is the amount of air that can be forcibly exhaled from the lungs after taking the deepest breath possible. FEV₁-Forced Expiratory Volume in One Second, this is the amount of air which can be forcibly exhaled from the lungs in the first second of a forced exhalation.
FEV₁/FVC, the ratio of FEV₁ to FVC expressed as a fraction (previously this was expressed as a percentage), this indicates what fraction of the total amount of air is exhaled from the lungs during the first second of forced exhalation.
PEF Peak Expiratory Flow, this is used as a measure to determine if treatment is effective in improving airway diseases, in particular in asthma and COPD.
FEF Forced Expiratory Flow, this is a measure of how much air can be exhaled from the lungs; it is an indicator of large airway obstruction.
MW Maximal Voluntary Ventilation, a value for this is determined by having the patient inhale and exhale as rapidly and fully as possible in 12 seconds. The results reflect the status of the muscles used for breathing, how stiff the lungs are and if there is any resistance in the airways when breathing. This test tells indicates how strong a patient's lungs are prior to surgery. If patients demonstrate poor performance on this test, it suggests that respiratory complications may occur after surgery.

In asthma, measurement of lung function using spirometry, in particular measurement of FEV₁ and / or Peak expiratory flow is used in both diagnosis and monitoring to confirm airflow limitation, and to demonstrate reversibility of lung function abnormalities following administration of a bronchodilator. The degree of reversibility in forced expiratory volume in one second (FEV₁) indicative of a diagnosis of asthma is usually accepted as ≥12% and ≥200 mL from the pre-bronchodilator value. As many lung diseases may result in reduced FEV₁, a useful assessment of airflow limitation in asthma is the ratio of FEV₁ to forced vital capacity (FVC). The FEV₁/FVC ratio is normally >0.75-0.80, and possibly >0.90 in children. Lower values suggest airflow limitation. PEF measurements are made using a peak flow meter are also a useful aid to diagnosis and monitoring of asthma. The terms "reversibility" and "variability" refer to changes in symptoms accompanied by changes in airflow limitation that occur spontaneously or in response to treatment. The term reversibility is generally applied to rapid improvements in FEV₁ (or PEF) measured within minutes after inhalation of a rapid-acting bronchodilator, e.g. after 200-400 µg salbutamol (albuterol), or more sustained improvement over days or weeks after the introduction of effective controller treatment, such as inhaled glucocorticosteroids. Variability refers to improvement or deterioration in symptoms and lung function occurring over time. Variability may be experienced over the course of 1 day (when it is called diurnal variability), from day to day, from month to month, or seasonally.

In accordance with the invention, it is particularly preferred that pulmonary function is assessed by spirometry, e.g. by measuring FEV₁ (asthma) and / or FVC (COPD / IPF), using methods standard in the art.

FEV₁ can be measured using the Masterscope® CT Spirometer using a protocol following the ATS / ERS recommendations 2005 (Eur Respir J; 26: 319-338). FEV₁ is the maximal volume of air exhaled in the first second of a forced expiration from a position of full inspiration, expressed in litres at BTPS (Body temperature (i.e. 37°C), ambient pressure, saturated with water vapour). FVC is the maximal volume of air exhaled with maximally forced effort from a maximal inspiration, i.e. vital capacity performed with a maximally forced expiratory effort, expressed in litres at body temperature and ambient pressure saturated with water vapour.

In asthma, an IL-13 antagonist in accordance with the invention may be employed to treat impaired pulmonary function to improve pulmonary function. When assessed by FEV₁, the improvement can be expressed as mean percent change in FEV₁ from baseline, preferably in the range of from 5% to 18%; for example, 5, 6, 7, 8, 9, 10, 11, 12, 13, 14, 15, 16, 17, 18%; such as at least 5, 6, 7, 8, 9, 10, 11, 12, 13, 14, 15, 16, 17, 18%.

In COPD or IPF, an IL-13 antagonist in accordance with the invention may be employed to treat impaired pulmonary function to maintain or slow the rate of decline in pulmonary function.

In certain embodiments of the invention amelioration of asthma in a subject is assessed by a composite measure of one or more asthma symptoms selected from the group consisting of: night-time waking, symptoms on waking, activity limitation, shortness of breath, wheezing, and short-acting β₂-agonist use. Amelioration of asthma in a subject can be assessed by asthma control questionnaire ACQ-6 (see Juniper EF, O'Byrne PM, Guyatt GH, Ferrie PJ, King DR. Development and validation of a questionnaire to measure asthma control. Eur Respir J 1999; 14: 902-7). Amelioration of asthma in a subject can be assessed by reduction in ACQ-6 score compared to baseline ACQ-6 score before administration of the IL-13 antagonist. A reduction in mean ACQ-6 score of ≥0·5 relative to baseline score is considered to be indicative of a clinically meaningful of amelioration of asthma, whereas a score of ≥1·5 indicates uncontrolled asthma.

In preferred embodiments of the invention, the IL-13 antagonist is an anti-human-IL-13 antibody or a human-IL-13-binding fragment thereof. Preferably, the anti-IL-13 antagonist is an anti-human-IL-13 antibody or a human-IL-13-binding fragment thereof, selected from:
(a) tralokinumab (BAK502G9, CAT-354) antibody;
(b) a human-IL-13 binding fragment of tralokinumab comprising an antibody antigen-binding site which is composed of a human antibody VH domain (SEQ ID NO: 15) and a human antibody VL domain (SEQ ID NO:16) and which comprises a set of CDR's HCDR1, HCDR2, HCDR3, LCDR1, LCDR2 and LCDR3, wherein the VH domain comprises HCDR1, HCDR2 and HCDR3 and the VL domain comprises LCDR1, LCDR2 and LCDR3, wherein the HCDR1 has the amino acid sequence of SEQ ID NO: 7, the HCDR2 has the amino acid sequence of SEQ ID NO: 8, the HCDR3 has the amino acid sequence of SEQ ID NO: 9, the LCDR1 has the amino acid sequence of SEQ ID NO: 10, the LCDR2 has the amino acid sequence of SEQ ID NO: 11, and the LCDR3 has the amino acid sequence of SEQ ID NO: 12;
(c) a human-IL-13 binding fragment of tralokinumab comprising an antibody antigen-binding site which is composed of a human antibody VH domain and a human antibody VL domain which comprises a set of CDR's HCDR1, HCDR2, HCDR3, LCDR1, LCDR2 and LCDR3, wherein the VH domain comprises HCDR1, HCDR2 and HCDR3 and the VL domain comprises LCDR1, LCDR2 and LCDR3, wherein the HCDR1 has the amino acid sequence of SEQ ID NO: 7, the HCDR2 has the amino acid sequence of SEQ ID NO: 8, the HCDR3 has the amino acid sequence of SEQ ID NO: 9, the LCDR1 has the amino acid sequence of SEQ ID NO: 10, the LCDR2 has the amino acid sequence of SEQ ID NO: 11, and the LCDR3 has the amino acid sequence of SEQ ID NO: 12;
(d) an anti-IL-13 antibody, or a human-IL-13 binding fragment thereof comprising an antibody antigen-binding site which is composed of a human antibody VH domain (SEQ ID NO: 15) and/or a human antibody VL domain (SEQ ID NO:16);

Also disclosed herewith is an anti-human-IL-13 antibody or a human-IL-13-binding fragment thereof, selected from:
(a) an antibody or an IL-13 binding fragment thereof that competes with tralokinumab for binding to human IL-13;
(b) an antibody or an IL-13 binding fragment thereof capable of binding an epitope within the human IL-13 amino acid sequence from aspartic acid at position 99 to C-terminal asparagine at position 132 (FSSLHVRDTKIEVAQFVKDLLLHLKKLFREGRFN) of human IL-13 protein, or capable of binding an epitope within the human IL-13 sequence from aspartic acid at position 106 to C-terminal asparagine at position 132 (DTKIEVAQFVKDLLLHLKKLFREGRFN) of human IL-13 protein; and
(c) an antibody or an IL-13 binding fragment thereof that has one, two, three, four, five, or six CDRs (SEQ ID NOs: 7, 8, 9, 10, 11, 12) in common with tralokinumab.

In a preferred aspect of the anti-human-IL-13 antibody or a human-IL-13 binding fragment thereof described above, the whole antibody is an IgG4.

Disclosed herewith is an anti-human-IL-13 antibody or a human-IL-13 binding fragment thereof that may comprise one or more CDRs, or a set of all 6 CDRs (HCDR1, HCDR2, HCDR3, LCDR1, LCDR2, LCDR3), selected from the CDRs of:
BAK278D6 (SEQ ID NOS: 1, 2, 3, 4, 5, 6),
BAK1183H4 (SEQ ID NOS: 97, 98, 99, 100, 101, 102),
BAK1167F02 (SEQ ID NOS: 64, 65, 66, 67, 68, 69),
BAK1111D 10 (SEQ ID NOS: 91, 92, 93, 94, 95, 96),
BAK1166G02 (SEQ ID NOS: 67, 68, 69, 70, 71, 72),
BAK1167F04 (SEQ ID NOS: 85, 86, 87, 88, 89, 90),
BAK1184C8 (SEQ ID NOS: 73, 74, 75, 76, 77, 78),
BAK1185E1 (SEQ ID NOS: 79, 80, 81, 82, 83, 84), and
BAK1185F8 (SEQ ID NOS: 103, 104, 105, 106, 107, 108).

A method, use, composition or device as described herewith may comprise an anti-human-IL-13 antibody or a human-IL-13 binding fragment thereof comprising a VH domain comprising a set of CDRs and/or a VL domain comprising a set of CDRs selected from the set of HCDRs and / or set of LCDRs of BAK278D6, BAK1183H4, BAK1167F02, BAK1111D 10, BAK1166G02, BAK1167F04, BAK1184C8, BAK1185E1, and BAK1185F8.

An anti-human-IL-13 antibody or a human-IL-13 binding fragment thereof, may comprise a VH and VL of a clone selected from:
BAK278D6 (VH SEQ ID NO: 13, VL SEQ ID NO: 14),
BAK1183H4 (VH SEQ ID NO: 37, VL SEQ ID NO: 38),
BAK1167F02 (VH SEQ ID NO: 35, VL SEQ ID NO: 36),
BAK1111D 10 (VH SEQ ID NO: 41, VL SEQ ID NO: 42),
BAK1166G02 (VH SEQ ID NO: 53, VL SEQ ID NO: 54),
BAK1167F04 (VH SEQ ID NO: 43, VL SEQ ID NO: 44),
BAK1184C8 (VH SEQ ID NO: 45, VL SEQ ID NO: 46),
BAK1185E1 (VH SEQ ID NO: 47, VL SEQ ID NO: 48), and
BAK1185F8 (VH SEQ ID NO: 49, VL SEQ ID NO: 50).

An anti-human-IL-13 antibody or a human-IL-13 binding fragment thereof may have HCDR1, HCDR2 and HCDR3 of the VH domain within a germ-line framework and/or LCDR1, LCDR2 and LCDR3 of the VL domain within a germ-line framework. The VH germ-line framework can be VH1 DP14. The VL germ-line framework can be VL y3 3H.

An anti-human-IL-13 antibody or a human-IL-13 binding fragment thereof may bind a human IL-13 variant in which arginine at position 130 is replaced by glutamine. An anti-human-IL-13 antibody or a human-IL-13 binding fragment thereof may bind a non-human primate IL-13, such as rhesus or cynomolgus IL-13.

Other IL-13 antagonists that may be employed in accordance with the invention include:
(a) Anti-human-IL-13 antibodies such as Lebrikizumab (MILR1444A / RG3637, Roche / Genentech), ABT-308 (Abbott), GSK679586 (GlaxoSmithkline); QAX576 (Novartis),
(b) Anti-human-IL-13Rα1 antibodies such as Merck MK6105;
(c) IL-13 - toxin conjugates, such as IL-13 PE38QQR ;
(d) IL-4 muteins, such as Aerovant ™ (Aerovance) (modified IL-4);
(e) anti-IL-4Rα antibodies, such as Regeneron-668 (Regeneron);
(f) nucleic acids such as AIR645 (a double-stranded oligonucleotide directed against IL-4Rα); and,
(g) IL-4 / IL-13 bispecific antibodies, such as Domantis' 09/10 and DOM-1000P.

In some aspects of the invention, it is preferred that the anti-IL-13 antagonist is not also an antagonist of the interaction between IL-4 and IL4Rα, preferably the IL-13 antagonist is not an anti-IL-4Rα antibody, such as AMG317 (Amgen).

Preferably the anti-IL-13 antagonist is an anti-human-IL-13 antibody or a human-IL-13-binding fragment thereof.

Preferably, an antibody or a binding fragment thereof employed in the invention is selected from the group consisting of; an immunoglobulin molecule, a monoclonal antibody, a chimeric antibody, a CDR-grafted antibody, a humanized antibody, a Fab, a Fab', a F(ab')2, a Fv, a disulfide linked Fv, a scFv, a single domain antibody, a diabody, a multispecific antibody, a dual-specific antibody, and a bispecific antibody.

The subject to be treated in accordance with the invention may have impaired pulmonary function associated with asthma, COPD, or idiopathic pulmonary fibrosis (IPF). Asthma may be classified as atopic asthma non-atopic asthma, moderate to severe asthma, moderate to severe asthma uncontrolled by inhaled or oral corticosteroids and asthma at GINA score 5, 4, 3, 2, or 1. Subjects with asthma and may be undergoing concomitant therapy with one or more further therapeutic selected from an inhaled corticosteroid, long-acting beta2 antagonist (LABA), theophylline, a leukotriene antagonist, and an oral corticosteroid.

Impaired pulmonary function may be associated with COPD, such as moderate to severe COPD defined by GOLD classification II, II and IV. The spirometric criterion required for a diagnosis of COPD is an FEV1 /FVC ratio below 0.7 after Bronchodilator). The GOLD spirometric criteria for COPD severity is as follows

| | | | |
|---|---|---|---|
| I: Mild COPD | FEV1 / FVC < 0.7 | FEV1 ≥ 80% predicted | At this stage, the patient may not be aware that their lung function is abnormal. |
| II: Moderate COPD | FEV1 / FVC < 0.7 | 50% ≤ FEV1 < 80% predicted | Symptoms usually progress at this stage, with shortness of breath typically developing on exertion. |
| III: Severe COPD | FEV1 / FVC < 0.7 | 30% ≤ FEV1 < 50% predicted | Shortness of breath typically worsens at this stage and often limits patients' daily activities. Exacerbations are especially seen beginning at this stage. |
| IV: Very Severe COPD | FEV1 / FVC < 0.7 | FEV1 < 30% predicted or FEV1 < 50% predicted plus chronic respiratory failure | At this stage, quality of life is very appreciably impaired and exacerbations may be life-threatening. |

Subjects with COPD may be undergoing concomitant therapy with one or more further therapeutic selected from an inhaled corticosteroid, LABA, theophylline, an oral corticosteroid, tiotropium and roflumilast.

Impaired pulmonary function may be associated with idiopathic pulmonary fibrosis; subjects with IPF may be undergoing concomitant therapy with one or more further therapeutic selected from an inhaled corticosteroid, LABA, theophylline, a leukotriene antagonist, an oral corticosteroid and pirfenidone.

Accordingly, an IL-13 antagonist or composition comprising an IL-13 antagonist as described herein may be administered alone or in combination with other treatments, either simultaneously or sequentially dependent upon the condition to be treated.

In some aspect of the invention the subject with impaired pulmonary function is steroid naive.

In preferred embodiments, the IL-13 antagonist, which is an anti-human-IL-13 antibody or a human-IL-13-binding fragment thereof, is administered subcutaneously at a dose in the range of from 100 to 600 mg. It could also be administered intravenously at a dose in the range of from 100 to 1800 mg, or at a dose in the range of from 200 to 1600 mg, or at about 1500 mg. Preferably the anti-human IL-13 antibody is tralokinumab or a human-IL-13 binding fragment thereof, as described herein. Suitable unit doses for sub-cutaneous administration may be selected from 150, 300, and 600 mg, preferably administered at 2 or 4 week intervals. Suitable unit doses for intravenous administration may be selected from 1000, 1200, 1400, 1500, 1600 1700 and 1800 mg, preferably administered at 2 or 4 week intervals, most preferably administered at 4 week intervals. Preferably, the IL-13 antagonist is an anti-human-IL-13 antibody or human-IL-13-binding fragment thereof, more preferably the anti-human IL-13 antibody is tralokinumab or a human-IL-13 binding fragment thereof, as described herein, administered at a mg/kg dose in the range of from 1 mg / Kg to 30 mg /Kg.

The subject may be an adult, or a young adult (12 - 18 years' old) however the invention may also be employed to improve, maintain or reduce the rate of decline of pulmonary function in paediatric subjects, aged less than 12 years' old.

An IL-13 antagonist, preferably an anti-human-IL-13 antibody or human-IL-13-binding fragment thereof, more preferably the anti-human IL-13 antibody is tralokinumab or a human-IL-13 binding fragment thereof, as described herein, may be administered in accordance with the invention at a frequency selected from every 1, 2, 3, 4, 6, 8, 10 and 12 weeks, preferred dosing intervals are every two or every four weeks.

Generally, an improvement in pulmonary function in asthma subjects can be detected by 15 days following initiation of treatment in accordance with the invention.

Surprisingly, the inventors found an improvement in pulmonary function as assessed by spirometry by measuring FEV₁ in moderate to severe asthma patients (GINA 4 or 5) to whom tralokinumab had been administered by subcutaneous administration, at doses of 150 mg, 300 mg or 600 mg, at dosing intervals of 2 weeks. It is believed that hitherto anti-human IL-13 antibodies have not been reported to improve pulmonary function in asthmatic patients. An early onset of improvement in pulmonary function, as assessed by FEV₁ was seen in the asthmatic subjects treated with tralokinumab, with an improvement in FEV₁ score being detectable by 15 days after the first administration of tralokinumab. Furthermore, it was found that the improvement in FEV₁ score correlated with the dose of the tralokinumab that was administered. The average improvement in FEV₁ score over baseline measurement taken before initiation of treatment was 8% in the 150 mg treatment group, 13.3% in the 300 mg treatment group and 15.2 % in the 600 mg treatment group. The mean improvement in FEV₁ for all tralokinumab treatment groups combined was 12.2%.

Accordingly, the inventors have found that an improvement in pulmonary function, as assessed by spirometry, e.g., by measuring FEV₁, can be used as an indicator of a subjects' response to treatment of asthma using an anti-IL-13 antagonist, preferably using an anti-human-IL-13 antibody or human-IL-13-binding fragment thereof, e.g. tralokinumab or a human-IL-13 binding fragment thereof as described herein.

Thus, also disclosed herewith is a method for detecting a subject's response to an IL-13 antagonist treatment in asthma, comprising:
(a) assessing pulmonary function by a spirometric method prior to treatment,
(b) assessing pulmonary function by a spirometric method after treatment,
(c) wherein an improvement in pulmonary function is indicative of a beneficial response to treatment.

Pulmonary function may be assessed by measuring FEV₁ and/or PEF. Assessment of pulmonary function after treatment can be performed at least 15 days after initiation of treatment.

In preferred aspects of the invention the IL-13 antagonist is an anti-human-IL-13 antibody, more preferably the anti-human IL-13 antibody is tralukinumab or a human-IL-13 binding fragment thereof, as described herein, preferably administered by subcutaneous administration, preferred doses being 150 mg, 300mg or 600 mg, with the preferred dosing interval being 2 or 4 weeks. Preferably, the improvement in pulmonary function following treatment compared to baseline is in the range of from 8% to 16%, measured at least 15 days after initiation of treatment and / or up to 12 weeks after cessation of treatment.

Preferably, the improvement in pulmonary function from baseline, as assessed by FEV₁ measurement, following subcutaneous administration at two week intervals of 150 mg tralokinumab or a human-IL-13 binding fragment thereof, as described herein, is in the range of from about 6 to about 10%, e.g., about 8%. Preferably, assessment of pulmonary function after treatment is performed at least 15 days after initiation of treatment and / or up to 12 weeks after cessation of treatment.

Preferably, the improvement in pulmonary function from baseline, as assessed by FEV₁ measurement, following subcutaneous administration at two week intervals of 300 mg tralokinumab or a human-IL-13 binding fragment thereof, as described herein, is in the range of from about 12 to about 14%, e.g., about 13.3 %. Preferably, assessment of pulmonary function after treatment is performed at least 15 days after initiation of treatment and / or up to 12 weeks after cessation of treatment.

Preferably, the improvement in pulmonary function from baseline, as assessed by FEV₁ measurement, following subcutaneous administration at two week intervals of 600 mg tralokinumab or a human-IL-13 binding fragment thereof, as described herein, is in the range of from about 14 to about 16%, e.g., about 15.2%. Preferably, assessment of pulmonary function after treatment is performed at least 15 days after initiation of treatment and / or up to 12 weeks after cessation of treatment.

All subjects were followed for 12 weeks after the last dose of tralokinumab and underwent spirometry testing at days 127 and day 169. Surprisingly, the increases in FEV₁ that were observed in tralokinumab-treated subjects at day 92 were maintained at both these post-dosing time points. The mean change from baseline in FEV₁ at day 169 was 0.101 L in the placebo group and 0.218 L in the combined subcutaneous tralokinumab treatment group (p = 0.096). The numerical increases in FVC and PEF observed at day 92 were also maintained to day 169. This finding suggests that the beneficial effects of tralokinumab on lung function may be sustained for a prolonged period of at least 12 weeks after cessation of treatment.

This sustained effect on FEV₁ permits a dosing regimen of the invention that provides an initial "loading" first dosing period, followed by a subsequent "maintenance" second dosing period. In the loading first dosing period the dose may be administered at a higher dose than in the second maintenance period. In the loading first dosing period the dose may be administered more frequently than in the maintenance second dosing period. Accordingly, the the invention provides a dosing regimen wherein the IL-13 antagonist (which is preferably an anti-human-IL-13 antibody, more preferably the anti-human IL-13 antibody is tralukinumab or a human-IL-13 binding fragment thereof, as described herein) is administered:
(a) at a first dose at a first dosing interval for a first period of time, then at the first dose for a second dosing interval (which is longer than the first dosing interval) for a second period of time, for example by subcutaneous administration at 300 mg every two weeks (Q2W) for 8, 9, 10, 11, 12, 13, 14, 15, or 16 weeks followed by subcutaneous administration at 300 mg every 3, 4, 5, or 6 weeks (Q4W); preferably at 300 mg every two weeks (Q2W) for 12 weeks followed by subcutaneous administration at 300 mg every 4 weeks (Q4W); or,
(b) at a first dose at a first dosing interval for a first period of time, then at a second dose (which is lower than the first dose) at the first dosing interval, for example by subcutaneous administration at 300 mg every two weeks (Q2W) for 8, 9, 10, 11, 12, 13, 14, 15, or 16 weeks followed by subcutaneous administration at 150 mg every 2 weeks (Q2W); preferably at 300 mg every two weeks (Q2W) for 12 weeks followed by subcutaneous administration at 150 mg every 2 weeks (Q2W);
(c) at a first dose at a first dosing interval, then at a second dose (lower than the first dose) for a second dosing interval (which is longer than the first dosing interval), for example by subcutaneous administration at 300 mg every two weeks (Q2W) for 8, 9, 10, 11, 12, 13, 14, 15, or 16 weeks followed by subcutaneous administration at 150 mg every 3, 4, 5, or 6 weeks; preferably at 300 mg every two weeks (Q2W) for 12 weeks followed by subcutaneous administration at 150 mg every 4 weeks (Q4W).

When the IL-13 antagonist is Tralokinumab or a variant thereof or a human-IL-13 binding fragment thereof, the dosing regimen may be such that it is administered at 300 mg (e.g., as 2 x 150 mg subcutaneous injections every 2 weeks (Q2W) for 12 weeks followed by every 4 weeks (Q4W). Formulations of Tralokinumab suitable for use in such dosing regimens are described herein.

### Figure Legends

Figure 1: Study Flow Diagram: A Phase 2a, randomized, double-blinded placebo-controlled, parallel -arm study to evaluate the efficacy and safety of three subcutaneous treatment regimes of tralokinumab (CAT-354) in adult subjects with uncontrolled, moderate-to-severe, persistent asthma
Figure 2a: Mean Change from Baseline in FEV₁ (Evaluable Population). Change from baseline in pre-bronchodilator FEV₁ to study day 92 for combined tralokinumab group and for placebo.
Figure 2b: Change from baseline in FEV₁. Change from baseline in pre-bronchodilator FEV₁ to study day 92 for individual tralokinumab treatment groups (150 mg, 300 mg, or 600 mg administered subcutaneously every 2 weeks) and placebo.

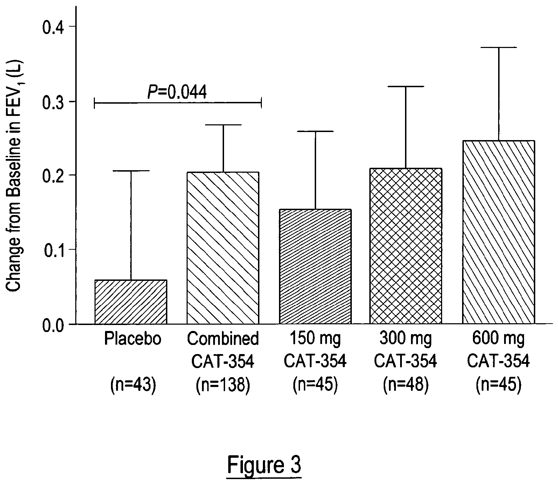

Figure 3: Change from baseline in pre-bronchodilator FEV₁ (L) at study day 92

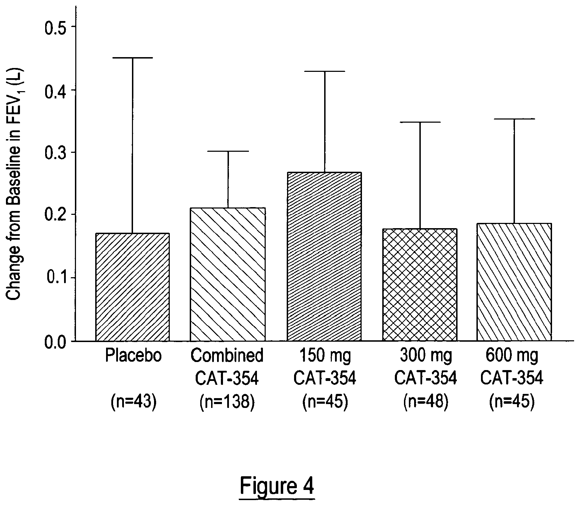

Figure 4: Change from baseline in pre-bronchodilator FEV₁ (L) at study day day 92 in subjects classified as atopic at baseline.

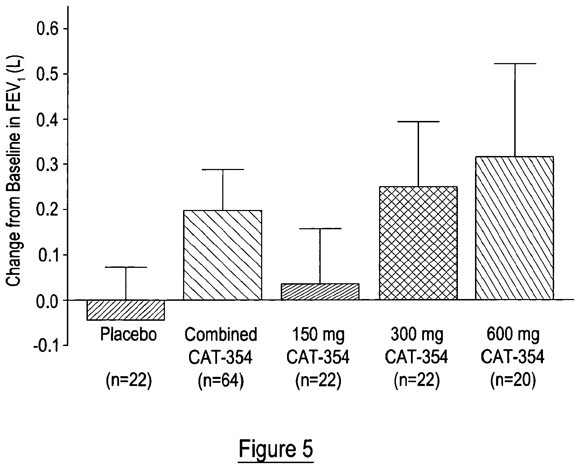

Figure 5: Change from baseline in pre-bronchodilator FEV₁ at study day 92 in subjects classified as non-atopic at baseline.

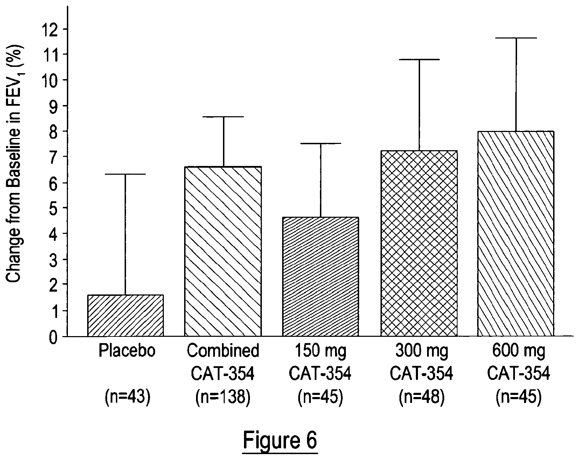

Figure 6: Change from baseline in % predicted pre-bronchodilator FEV₁ at study day 92

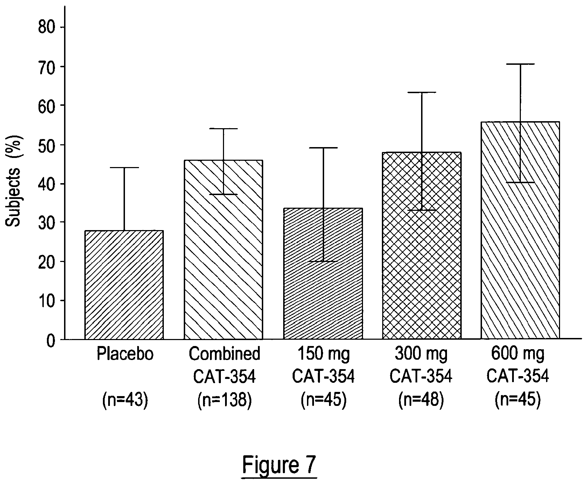

Figure 7: Percentage of subjects showing a greater than, or equal to 10% change from baseline in pre-bronchodilator FEV₁

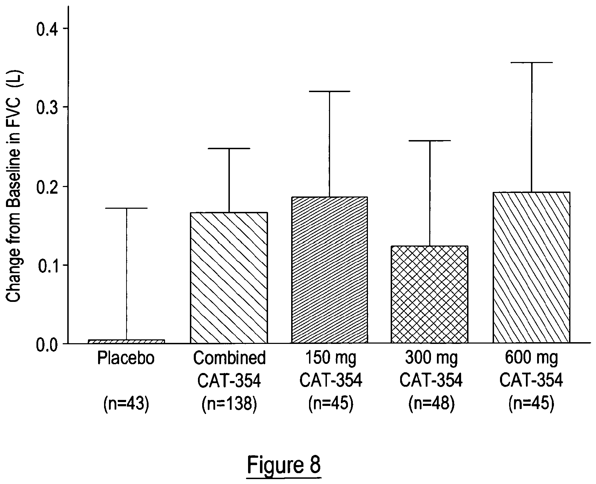

Figure 8: Change from baseline in FVC at study day 92

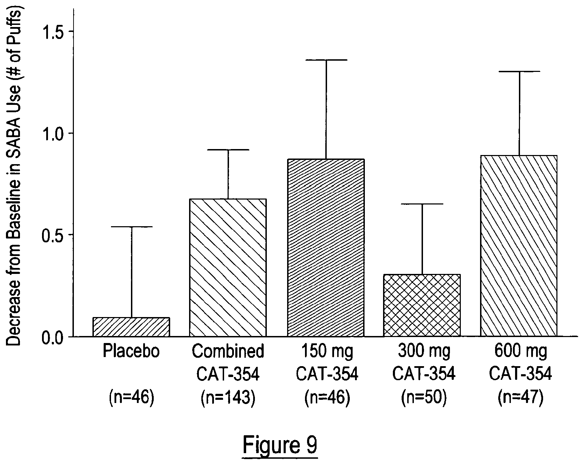

Figure 9: Decrease from baseline in SABA use at study day 92. Change in the mean number of "puffs" of short acting beta agonist (rescue treatment) taken daily comparing
Figure 10a: Mean change from baseline in FEV₁ (Evaluable Population), change from baseline in pre-bronchodilator FEV₁ to study day 169 for combined tralokinumab group (150 mg, 300 mg, or 600 mg administered subcutaneously every 2 weeks) and for placebo.
Figure 10b: Change from baseline in FEV₁, change from baseline in pre-bronchodilator FEV₁ to study day 169 for individual tralokinumab treatment groups (150 mg, 300 mg, or 600 mg administered subcutaneously every 2 weeks) and placebo.
Figure 11: Mean (SD) change from baseline in ACQ-6 score over time (evaluable population) ACQ-6=Asthma Control Questionnaire, mean of 6 individual item scores.
Figure 12: Mean (SD) change from baseline in pre-bronchodilator FEV₁ over time (evaluable population) FEV₁=forced expiratory volume in 1 s.
Figure 13: Mean (SD) change from baseline in ACQ-6 score over time (evaluable population) in the subpopulation (n=56) who supplied a sputum sample at baseline. Placebo (sputum: all) is the subjects treated with placebo (n=17). Tralokinumab (sputum: all) is the subjects treated with tralokinumab (n=39). The tralokinumab-treated group (n=39) is subdivided into those without measurable IL-13 [tralokinumab (sputum IL-13 <1 pg/mL)] and those with measurable IL-13 [tralokinumab (sputum IL-13 ≥1 pg/mL)]. ACQ-6=Asthma Control Questionnaire, mean of 6 individual item scores.
Figure 14: Mean (SD) change from baseline in pre-bronchodilator FEV₁ over time (evaluable population) in the subpopulation (n=56) who supplied a sputum sample at baseline. Placebo (sputum: all) is the subjects treated with placebo (n=17). Tralokinumab (sputum: all) is the subjects treated with tralokinumab (n=39). The tralokinumab-treated group (n=39) is subdivided into those without measurable IL-13 [tralokinumab (sputum IL-13 <1 pg/mL)] and those with measurable IL-13 [tralokinumab (sputum IL-13 ≥1 pg/mL)]. FEV₁=forced expiratory volume in 1 s.

### Examples

### Example 1:

### Study Design

It was proposed that IL-13 blockade may reduce exacerbations and improve disease control (as defined by improvements in asthma symptoms coupled with reduced need for rescue medications) in patients whose asthma is uncontrolled, moderate-to-severe, and persistent despite maximal therapy at GINA step 4 or 5.

A Phase 2a, randomized, double-blind, placebo-controlled, parallel-arm study was performed to evaluate the efficacy and safety of three subcutaneous (SC) treatment regimens of tralokinumab, a recombinant human monoclonal antibody directed against interleukin 13 (IL-13), in adult subjects with uncontrolled, moderate-to-severe, persistent asthma. The study comprised a 2-week run-in period, a 12-week treatment period, and a 12-week follow-up period. Selection of dose regimen and treatment duration was based on pharmacokinetic modelling and simulations using data from previous clinical studies. This study is registered with ClinicalTrials.gov, number NCT00873860.

A total of 192 subjects were planned to be randomized. Prior to being randomized, subjects were stratified according to atopic asthma status using the Phadiatop test (Laboratoire Pasteur Cerba, France, a fluorescence enzyme immunoassay (FEIA) for allergen-specific immunoglobulin E (IgE) to the most frequent aero-allergens (mites, moulds, animals, grasses, weeds and trees) which determines presence of specific IgE antibodies to multiple inhaled allergens). Within each stratum, subjects were randomised according to a computer-generated randomisation list (1:1:1) to one of three arms (150 mg tralokinumab or placebo, 300 mg tralokinumab or placebo, 600 mg tralokinumab or placebo). Within each arm, subjects were then randomised according to a computer-generated randomisation list to active treatment or placebo (3:1). Study treatment was administered every 2 weeks by SC injection in the anterior thigh. Subjects received one, two or four SC injections (150 mg/l mL per injection) of tralokinumab at 7 visits over 12 weeks. All subjects were followed for 12 weeks after the last dose of investigational product (Study Day 169). Study medication was dispensed and administered by unblinded personnel, not otherwise involved in the study. Subjects continued their pre-study asthma controller therapy, as prescribed by their physician, during the study. Subjects and physicians were blinded to treatment allocation throughout.

An objective of this study was to evaluate the effect of the three (SC) treatment regimens of tralokinumab on asthma control at Study Day 92 versus placebo in adults with uncontrolled, moderate-to-severe, persistent asthma. The primary endpoint of the study was the Asthma Control Questionnaire (ACQ-6) (Juniper *et al.* (1999). Measures of pulmonary function, rescue medication use, asthma exacerbation rate, patient-reported outcomes (PROs), and safety were secondary endpoints. The secondary objectives included evaluating the effect of SC tralokinumab on the variability of airflow obstruction using the parameters of forced expiratory volume within 1 second (FEV₁) and peak expiratory flow (PEF).

### Major inclusion criteria

Male or female subjects.

Age 18 to 65 years at the time of screening.

Subjects must have a body mass index (BMI) between 18 and 40 kg/m². Written informed consent obtained from the subject prior to performing any protocol-related procedures, including screening evaluations. Physician-diagnosed moderate-to-severe, persistent asthma requiring treatment with appropriate asthma controller medication.

Shows FEV₁ reversibility post-bronchodilator of ≥ 12% and ≥ 200 mL or have shown such values in a previous test within the last year, or have a positive airway hyper-responsiveness (AHR) test result in the last year. Pre-bronchodilator FEV₁ value ≥ 40% of individual predicted value at Visits 1 and 3.

Uncontrolled asthma consistent with Expert Panel Report (EPR)-3. In the 2 to 4 weeks preceding the screening visit, subjects should have a history of one or more of the following:
Daytime asthma symptoms ≥ 2 days/week
Night-time awakening ≥ 1 night/week
Salbutanol use ≥ 2 dayst/week.
An ACQ score ≥ 1.5 at Visits 1 and 3.

At least one occurrence of asthma exacerbation in the past year that required an unscheduled medical encounter.

Women of childbearing potential, unless surgically sterile (including tubal ligation) and/or at least 2 years postmenopausal must have a negative pregnancy test prior to the first dose of investigational product, and must agree to use 2 effective methods of avoiding pregnancy (including oral, injectable, transdermal, or implanted contraceptives, intrauterine device, female condom with spermicide, diaphragm with spermicide, cervical cap, use of condom by male sexual partner); or abstinence or sterile sexual partner from screening through Study Day 169. Women of childbearing potential must continue to practice birth control during the study and for at least 2 months after completing the study.

Men, unless surgically sterile, must likewise practice 2 effective methods of birth control (condom with spermicide) and must use such precautions from Study Day 1 through Study Day 169.

Otherwise healthy by medical history and physical examination for that age group.

A chest x-ray within the previous 12 months with no findings suggestive of acute or chronic respiratory pathology other than asthma.

Ability and willingness to complete the follow-up period until Study Day 169 as required by the protocol.

### Major exclusion criteria

Known history of allergy or reaction to any component of the investigational product formulation.

Acute illness other than asthma at the start of the study.

History of an active infection within 4 weeks prior to screening, or evidence of clinically significant active infection, including ongoing chronic infection. History of ingestion of untreated water in a location known to be infected with parasites, resulting in acute or chronic diarrhea; or a diagnosis of parasitic infection within 6 months prior to screening.

Use of immunosuppressive medication (except oral prednisone up to 10 mg/day and inhaled and topical corticosteroids) within 30 days before randomization into the study.

Receipt of immunoglobulin or blood products within 30 days before randomization into the study.

Receipt of any investigational drug therapy or use of any biologicals including omalizumab within 6 months before the first dose of investigational product in this study or within 5 half-lives of an investigational agent or biologic, whichever is longer.

History of any known immunodeficiency disorder.

A positive hepatitis B surface antigen, or hepatitis C virus antibody, as determined by medical history and/or subject's verbal report.

A positive human immunodeficiency virus test or is taking antiretroviral medications, as determined by medical history and/or subject's verbal report.

A live or attenuated vaccination received within 4 weeks prior to screening Previous medical history, or evidence, of an intercurrent illness that may compromise the safety of the subject in the study.

History of clinically significant abnormality on electrocardiogram (ECG) in the opinion of the investigator.

### Lactation (women)

History of treatment for alcohol or drug abuse within the past year.

History suggestive of chronic obstructive pulmonary disease (COPD) and of cigarette smoking ≥ 10 pack-years.

Evidence of any systemic disease on physical examination.

History of cancer, apart from basal cell carcinoma or in situ carcinoma of the cervix treated with apparent success with curative therapy ≤ 1 year prior to Study Day 1 or other malignancies treated with apparent success with curative therapy ≤ 5 years prior to entry.

Known exposure to inhaled occupational agents or fumes.

Any condition (eg, cystic fibrosis [CF] or COPD) that, in the opinion of the investigator, would interfere with evaluation of the investigational product or interpretation of study results.

Individuals who are legally institutionalized

Employees of the clinical study site or any other individuals involved with the conduct of the study, or family members of such individuals.

### Target patient population

The subjects in this study were adult male and female subjects, 18 to 65 years of age, with uncontrolled, moderate-to-severe, persistent asthma.

### Primary endpoint

The ACQ-6, a composite measure of asthma symptoms (night-time waking, symptoms on waking, activity limitation, shortness of breath, wheezing, and short-acting β₂-agonist use), was completed weekly using an electronic diary (eDiary). The primary endpoint was the change from baseline to week 13 in mean ACQ-6 score. Reductions in mean ACQ-6 score of ≥0·5 are considered to be clinically meaningful whilst a score of ≥1·5 indicates uncontrolled asthma (Juniper *et al.* (2006)).

### Secondary endpoints

Spirometry was performed at every study visit in the morning; pre-bronchodilator FEV₁, forced vital capacity (FVC) and peak expiratory flow (PEF) were measured. Subjects also performed peak flow testing every morning at home.

For the purposes of the study, an asthma exacerbation was defined as a deterioration that required the administration of a systemic corticosteroid burst; specifically in a more detailed definition for the purpose of this study an asthma exacerbation (relapse or *de novo*) was defined as either a progressive increase of asthma symptoms (cough, wheeze, chest tightness, and/or shortness of breath) or a reduction of ≥20% in peak expiratory flow (PEF) or forced expiratory volume in 1 s (FEV₁) from baseline that did not resolve after the initiation of rescue medications and resulted in an administration of systemic corticosteroids by the investigator or healthcare provider. For those receiving maintenance oral corticosteroids (OCS) for the control of asthma, an asthma exacerbation was defined as occurring if OCS burst therapy or intravenous corticosteroids were initiated. Asthma exacerbations, if any, were reported at each study visit.

PROs recorded included a 4-item Daily Asthma Symptom Score and the Asthma Quality of Life Questionnaire with standardised activities (AQLQ(S)). Adverse events (AEs), if any, were recorded at every study visit; routine laboratory evaluations were done at screening, after the first, third, and fifth doses, and at weeks 13 and 24.

Sputum was induced and collected at selected study centres at baseline, and weeks 8 and 14. Sputum IL-13 content was assessed using an ELISA system (Luminex® Corp).

### Pharmacokinetic assessments

Blood samples were collected prior to each dose and during the follow-up period for pharmacokinetic assessment. Trough serum concentrations (Cₘᵢₙ) of tralokinumab were determined using an immunoassay performed on the Gyrolab assay platform that detects free tralokinumab. This method has a lower limit of quantification in serum of 0·300 µg/mL, and exhibits accuracy of <25% absolute relative error and precision of <20% CV (coefficient of variation).

Based on trough serum concentrations observed during the 12-week treatment period, exposure to tralokinumab increased approximately proportionally to dose over the range 150-600 mg. Mean (SD) serum concentrations at week 13 were 42·0 (16·3), 86·8 (33·1), and 156 (46·6) µg/mL for 150, 300, and 600 mg tralokinumab, respectively. Within each dose group, serum concentrations increased approximately twofold between weeks 2 and 10, consistent with moderate accumulation. Comparable serum concentrations were observed at weeks 10 and 12, indicating that steady-state had been attained by week 10.

### Immunogenicity

Blood samples were collected before the first dose and at weeks 13 and 24 for measurement of serum anti-drug antibodies using validated homogeneous bridging immunoassays with detection by electrochemiluminescence. No serum anti-drug antibodies were detected during the course of the study.

### Investigational product, dosage

A total of 192 subjects were planned to be randomized into this study, with 144 subjects to receive SC administration of Tralokinumab and 48 subjects to receive placebo. Tralokinumab was formulated at 150 mg/mL in 50 mM sodium acetate, 85 mM sodium chloride, 0.01% (w/v) plant-derived polysorbate 80 at pH 5.5)
Treatment Arm 1: 150 mg SC Tralokinumab (n=48) or 1 mL placebo (n=16) once every 2 weeks on Study Days 1,15, 29, 43, 57, 71, and 85
Treatment Arm 2: 300 mg Tralokinumab (n=48) or 2 mL placebo (n=16) once every 2 weeks on Study Days 1, 15, 29, 43, 57, 71, and 85
Treatment Arm 3: 600 mg Tralokinumab (n=48) or 4 mL placebo (n=16) once every 2 weeks on Study Days 1, 15, 29, 43, 57, 71, and 85.

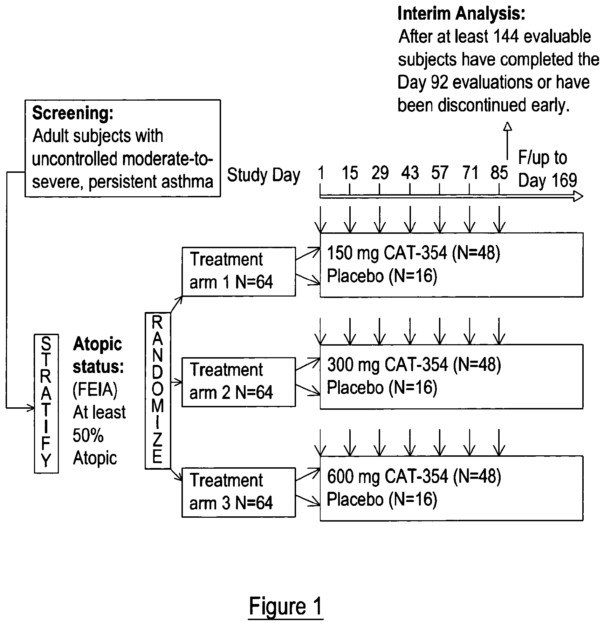

The study flow diagram is shown in Figure 1. Five subject populations were analyzed in this study as follows.
1. Intent-to-treat (ITT) Population included all subjects who were randomized into the study.
2. Evaluable Population included all subjects who received at least 4 doses of investigational product as well as subjects who received at least one dose of investigational product but discontinued treatment prior to receiving 4 doses due to safety reasons. The primary efficacy analysis was based on the Evaluable Population.
3. According-to-Protocol (ATP) Population included all subjects who received all doses of investigational product and had an ACQ score at Study Day 92.
4. The Safety Population included all subjects who received at least one dose of investigational product.
5. The PK Population included all subjects in the Safety Population who received SC tralokinumab and had a sufficient number of serum concentration measurements.

**Table 1 Summary of Subject Populations**

| Subject Populations | Placebo | SC TRALOKINUMAB | | | | Total |
|---|---|---|---|---|---|---|
| | | 150 mg | 300 mg | 600 mg | Combined | |
| All Subjects Randomized | 48 | 47 | 51 | 48 | 146 | 194 |
| ITT Population | 48 | 47 | 51 | 48 | 146 | 194 |
| Evaluable | 46 | 46 | 51 | 47 | 144 | 190 |
| Population | | | | | | |
| ATP Population | 44 | 45 | 47 | 44 | 136 | 180 |
| Safety Population | 47 | 47 | 51 | 48 | 146 | 193 |
| PK Population | 47 | 47 | 51 | 48 | 146 | 193 |

| | | | | | | |
|---|---|---|---|---|---|---|
| ATP = According-to-Protocol; ITT = Intent-to-Treat; PK = Pharmacokinetic. | | | | | | |

FEV1 was measured using the Masterscope® CT Spirometer using a protocol following the ATS / ERS recommendations 2005 (Eur Respir J; 26: 319-338).

### Statistical methods

Sample size calculations based on the primary endpoint were conducted using statistical software nQuery Advisor^{®} 6·01. Change from baseline in mean ACQ-6 score at week 13 was assumed to be -0·5 and -1·0 for the placebo and combined tralokinumab (150, 300, and 600 mg) groups, respectively, with a common SD of 0·9. A sample size of 144 was required to detect a statistically significant difference between combined tralokinumab groups and placebo at a 0·05 significance level with 80% power. To allow for dropouts it was planned to randomise 192 subjects.

The evaluable population included all subjects who received at least four doses of study medication and those who received at least one dose but discontinued prior to receiving four doses for safety reasons; the efficacy analyses reported are based on this population. The intent-to-treat (ITT) population was defined as all randomised subjects; confirmatory efficacy analysis was undertaken on this population. The safety population comprised all subjects who received at least one dose of study medication.

The primary endpoint was the comparison of the change from baseline to week 13 in mean ACQ-6 score between the combined tralokinumab group (i.e., 150, 300, and 600 mg) and placebo using analysis of variance. The primary efficacy analysis was undertaken at an interim analysis after all subjects had completed the week 13 visit. Two-sample t tests were used to compare between treatment group changes from baseline to week 13 in spirometry parameters measured during study visits. Efficacy endpoints were collected through to week 24 and were also analysed as described above (both study team and sites remained blinded to week 24). Exploratory efficacy analyses to compare each tralokinumab dose with placebo were undertaken and no multiple comparison adjustment was performed. Post-hoc analyses were conducted on the change from baseline to weeks 13 and 24 in rescue β₂-agonist use in the combined tralokinumab group and for each dose compared with placebo (two-sample t test). A post-hoc subgroup analysis defined by atopic asthma status was conducted on the change from baseline to week 13 in FEV₁.

A descriptive post-hoc analysis of the change from baseline in ACQ-6 and FEV₁ was undertaken in a subgroup of subjects with sputum samples obtained at baseline; subjects were analysed according to the presence or absence of detectable sputum IL-13.

Safety analysis was conducted after all subjects had completed the study using the safety population. All safety parameters were summarised descriptively.

### Results

The study was conducted between June 2009 and August 2010. 194 subjects were randomised at 27 study centres in Europe. Most subjects received all seven doses of study medication (placebo 95·7%; tralokinumab 93·8%).

### Disposition of Subjects

A total of 194 subjects was randomized in this Study at 27 sites in the European Union; 10 sites in Romania (n = 77), 6 sites in Bulgaria (n = 52), 6 sites in Poland (n = 37), 4 sites in Germany (n = 21), and one site in the United Kingdom (n = 7).

Of the 194 subjects randomized in the study, 48 subjects were randomized to the placebo group and 146 subjects were randomized to one of three SC Tralokinumab treatment groups (47 subjects in the 150 mg SC Tralokinumab treatment group, 51 subjects in the 300 mg SC Tralokinumab treatment group, and 48 subjects in the 600 mg SC Tralokinumab treatment group).

### Demographic and Other Baseline Characteristics

Baseline demographic characteristics are summarized in Table 2. The mean age of the subject population was 47.3 years (18-65 years), with similar mean ages (43.4-49.8 years) seen across the placebo and SC tralokinumab treatment groups. The majority of subjects was female (59.8%), with the exception of the 150 mg SC tralokinumab treatment group (40.4%). Most subjects were White (91.8%). Mean BMI was 27.082 kg/m² (18.52-35.29 kg/m²), with similar mean BMI (26.548-27.536 kg/m²) seen across the placebo and SC tralokinumab treatment groups.

**Table 2 Baseline Demographic Characteristics (ITT Population)**

| Parameters | Placebo (N=48) | SC TRALOKINUMAB | | | | Total (N=194) |
|---|---|---|---|---|---|---|
| | | 150 mg (N=47) | 300 mg (N=51) | 600 mg (N=48) | Combined (N=146) | |
| Age (years) | | | | | | |
| Mean | 47.2 | 43.4 | 48.7 | 49.8 | 47.4 | 47.3 |
| SD | 9.8 | 11.1 | 11 | 10.4 | 11.1 | 10.8 |
| Median | 48 | 46 | 51 | 52 | 50 | 50 |
| Min-Max | (23 - 65) | (18 - 64) | (19 - 65) | (25 - 65) | (18-65) | (18-65) |
| Gender | | | | | | |
| Male | 15 (31.3%) | 28 (59.6%) | 15 (29.4%) | 20 (41.7%) | 63 (43.2%) | 78 (40.2%) |
| Female | 33 (68.8%) | 19 (40.4%) | 36 (70.6%) | 28 (58.3%) | 83 (56.8%) | 116 (59.8%) |
| Ethnicity | | | | | | |
| Hispanic or Latino | 1 (2.1%) | 4 (8.5%) | 0 (0.0%) | 1 (2.1%) | 5 (3.4%) | 6 (3.1%) |
| Not Hispanic or Latino | 47 (97.9%) | 43 (91.5%) | 51 (100%) | 47 (97.9%) | 141 (96.6%) | 188 (96.9%) |
| Race | | | | | | |
| Asian | 1 (2.1%) | 0 (0.0%) | 1 (2.0%) | 0 (0.0%) | 1 (0.7%) | 2 (1.0%) |
| White | 42 | 46 | 46 | 44 | 136 | 178 |
| | (87.5%) | (97.9%) | (90.2%) | (91.7%) | (93.2%) | (91.8%) |
| Other | 5 (10.4%) | 1 (2.1%) | 4 (7.8%) | 4 (8.3%) | 9 (6.2%) | 14 (7.2%) |
| BMI (kg/m²) | | | | | | |
| Mean | 26.983 | 26.548 | 27.536 | 27.22 | 27.114 | 27.082 |
| SD | 4.001 | 3.777 | 4.181 | 3.876 | 3.949 | 3.952 |
| Median | 27.29 | 26.43 | 27.44 | 27.43 | 27.175 | 27.2 |
| Min-Max | (18.87 - 34.81) | (18.87 - 35.29) | (18.52 - 34.89) | (19.20 - 34.63) | (18.52 - 35.29) | (18.52 - 35.29) |

| | | | | | | |
|---|---|---|---|---|---|---|
| BMI = body mass index; ITT = intent-to-treat; Max = maximum; Min = minimum; SD = standard deviation. | | | | | | |

Subjects with physician-diagnosed moderate-to-severe, persistent asthma requiring treatment with appropriate asthma controller medication were randomized in this study. Baseline disease characteristics and asthma medication history are summarized in Table 3 and Table 4, respectively.

At baseline, the study population had moderate-to-severe asthma that was not fully controlled (overall mean FEV₁ 2·00 L [61·2% predicted]; FVC 3·26 L; ACQ-6 score 2·66). Overall, mean FEV₁ was 61.2% (41%-103%) predicted, which was consistent across the placebo and SC tralokinumab treatment groups. Mean Asthma Symptom Score was 4.42 (0.0-8.1), with the majority (59.8%) of subjects (59.8%) having an average of > 2 night-time awakenings per week. The majority (61.9%) of subjects also had at least one asthma exacerbation requiring oral corticosteroids (OCS) within the past 12 months. Stratification indicated that 47.9% of subjects had non-atopic asthma, whereas 52.1% of subjects were classified as atopic (defined by the presence of allergen-specific immunoglobulin E at the screening visit). Mean ACQ score was 2.66, which is consistent with an uncontrolled asthma population.

Subjects were taking a range of asthma controller medications at baseline and the mean inhaled corticosteroid (ICS) dose, expressed as beclomethasone dipropionate (BDP) equivalent, ranged from 1058.9-1254.3 mcg per day across the placebo and SC Tralokinumab treatment groups, the median daily ICS dose, expressed as beclomethasone diproprionate equivalent, was 1000 µg/day, 32% of subjects received high dose ICS (per GINA guideline 2009) and/or a regular daily dose of oral corticosteroid. Most subjects required rescue β2 agonists (mean 2.5 puffs/day) and short-acting beta agonists (90.7% using SABAs at least 3 days per week).

**Table 3 Baseline Disease Characteristics (ITT Population)**

| Parameters | Placebo (N=48) | SC TRALOKINUMAB | | | | Total (N=194) |
|---|---|---|---|---|---|---|
| | | 150 mg (N=47) | 300 mg (N=51) | 600 mg (N=48) | Combined (N=146) | |
| Office Spirometry (FEV₁ % Predicted) | | | | | | |
| N | 47 | 47 | 51 | 48 | 146 | 193 |
| Mean | 61.4 | 61.9 | 61.1 | 60.5 | 61.2 | 61.2 |
| SD | 12.3 | 12.7 | 12.6 | 11.8 | 12.3 | 12.3 |
| Median | 63.0 | 61.0 | 62.0 | 59.5 | 61.0 | 61.0 |
| Min-Max | (41 - 90) | (42 - 94) | (43 - 98) | (43 - 103) | (42 - 103) | (41 - 103) |
| FEV_{1,} L, mean (SD) | 1·94 (0·48)* | 2·20 (0·67) | 1·90 (0·59) | 1·96 (0·66) | 2·01 (0·65) | |
| FVC, L, mean (SD) | 3·25 (0·80)* | 3·56 (0·95) | 3·08 (1·01) | 3·15 (1·04) | 3·26 (1·02) | |
| Asthma Status | | | | | | |
| Non-atopic | 25 (52.1%) | 22 (46.8%) | 24 (47.1%) | 22 (45.8%) | 68 (46.6%) | 93 (47.9%) |
| Atopic | 23 (47.9%) | 25 (53.2%) | 27 (52.9%) | 26 (54.2%) | 78 (53.4%) | 101 (52.1%) |
| Asthma Exacerbations Requiring OCS | | | | | | |
| in Past 12 Months | | | | | | |
| 0 | 18 (37.5%) | 18 (38.3%) | 23 (45.1%) | 15 (31.3%) | 56 (38.4%) | 74 (38.1%) |
| 1 | 23 (47.9%) | 24 (51.1%) | 23 (45.1%) | 23 (47.9%) | 70 (47.9%) | 93 (47.9%) |
| 2 | 6 (12.5%) | 4 (8.5%) | 3 (5.9%) | 6 (12.5%) | 13 (8.9%) | 19 (9.8%) |
| 3 | 1 (2.1%) | 1 (2.1%) | 1 (2.0%) | 2 (4.2%) | 4 (2.7%) | 5 (2.6%) |
| 5 | 0 (0.0%) | 0 (0.0%) | 0 (0.0%) | 1 (2.1%) | 1 (0.7%) | 1 (0.5%) |
| | | | | | | |
| PEF, L, mean (SD) | 284·9 (79·6)* | 341·2 (118·6) | 290·5 (97·0) | 309·9 (117·5) | 313·2 (112·3) | |
| Rescue β₂-agonist use, puffs / day, mean (SD) | 2·60 (1·68)* | 2·80 (2·03) | 2·04 (1·51) | 2·60 (1·95) | 2-47 (1·85) | |
| ACQ-6 score, mean (SD) | 2·60 (0·54)* | 2·68 (0·62) | 2·63 (0·50) | 2·72 (0·75) | 2·68 (0·63) | |
| Overall AQLQ(S) score, mean (SD) | 4-15 (0·83)† | 3·90 (0·88)‡ | 4-15 (0·80)‡ | 4-16 (0·94)† | 4-07 (0·87)§ | |

| | | | | | | |
|---|---|---|---|---|---|---|
| ACQ = Asthma Control Questionnaire; FEV₁ = Forced expiratory volume within 1 second; Max = maximum; Min = minimum; N = number; NA = not applicable; SD = standard deviation. | | | | | | |

**Table 4 Summary of Asthma Medication History (ITT Population)**

| Parameters | Placebo (N=48) | SC TRALOKINUMAB | | | | Total (N=194) |
|---|---|---|---|---|---|---|
| | | 150 mg (N=47) | 300 mg (N=51) | 600 mg (N=48) | Combined (N=146) | |
| ICS Dose (µg/day) | | | | | | |
| N | 45 | 46 | 50 | 48 | 144 | 189 |
| Mean | 1058.9 | 1254.3 | 1112.0 | 1254.2 | 1204.9 | 1170.1 |
| SD | 729.7 | 785.6 | 799.7 | 866.9 | 815.4 | 796.4 |
| Median | 1000.0 | 1000.0 | 1000.0 | 1000.0 | 1000.0 | 1000.0 |
| Min-Max | (200-4000) | (200-4000) | (100-4000) | (200-4000) | (100-4000) | (100-4000) |
| Subjects Who Received High-dose ICS/OCS | 11 (22.9%) | 18 (38.3%) | 15 (29.4%) | 18 (37.5%) | 51 (34.9%) | 62 (32.0%) |
| Rescue β2 Agonist Use (Average Number of Puffs/Day) | | | | | | |
| N | 47 | 47 | 51 | 48 | 146 | 193 |
| Mean | 2.60 | 2.80 | 2.04 | 2.60 | 2.47 | 2.50 |
| SD | 1.68 | 2.03 | 1.51 | 1.95 | 1.85 | 1.81 |
| Median | 2.14 | 2.00 | 1.86 | 2.00 | 2.00 | 2.00 |
| Min-Max | (0.0 - 7.4) | (0.0 - 7.7) | (0.0 - 6.6) | (0.0 - 9.1) | (0.0 - 9.1) | (0.0 - 9.1) |
| SABA Use in Last 3 Months | | | | | | |
| 0-2 Days/Week | 5 (10.4%) | 3 (6.4%) | 5 (9.8%) | 4 (8.3%) | 12 (8.2%) | 17 (8.8%) |
| 3-6 Days/Week | 28 (58.3%) | 26 (55.3%) | 28 (54.9%) | 29 (60.4%) | 83 (56.8%) | 111 (57.2%) |
| 7 Days/Week | 15 (31.3%) | 18 (38.3%) | 17 (33.3%) | 15 (31.3%) | 50 (34.2%) | 65 (33.5%) |
| NA | 0 (0.0%) | 0 (0.0%) | 1 (2.0%) | 0 (0.0%) | 1 (0.7%) | 1 (0.5%) |

| | | | | | | |
|---|---|---|---|---|---|---|
| ICS = inhaled corticosteroids; Max = maximum; Min = minimum; N = number; NA = not applicable; OCS = oral corticosteroids; SABA = short-acting beta agonist; SD = standard deviation. | | | | | | |

### Efficacy Evaluation

This study was a Phase 2a study designed to establish whether blockade of IL-13 by SC Tralokinumab resulted in improved asthma control in subjects with uncontrolled, moderate-to-severe, persistent asthma. Efficacy results are reported for the evaluable population; results based on the ITT population were consistent with those for the evaluable population.

A 2-stage approach was used to analyze the data. Stage 1 (Study Day 92) of the analysis corresponded to the interim analysis specified by the protocol, i.e., the analysis of the primary endpoint was conducted after all subjects completed the Study Day 92 visit, with selected secondary endpoints included. Stage 2 (Study Day 169) corresponded to the final analysis specified in the protocol, i.e., the analysis of the overall study safety endpoints together with the remaining efficacy endpoints.

### Spirometry

Spirometry was performed at the study site during each visit. Multiple forced expiratory efforts (at least 3, but no more than 8) were performed for each spirometry session. The highest FEV₁ and FVC of all accepted efforts were used for the analysis. In addition, the PEF associated with the best effort of FEV₁ was used for analysis. The changes in airflow obstruction (FEV₁ and FVC at site and PEF at home) during the study were assessed.

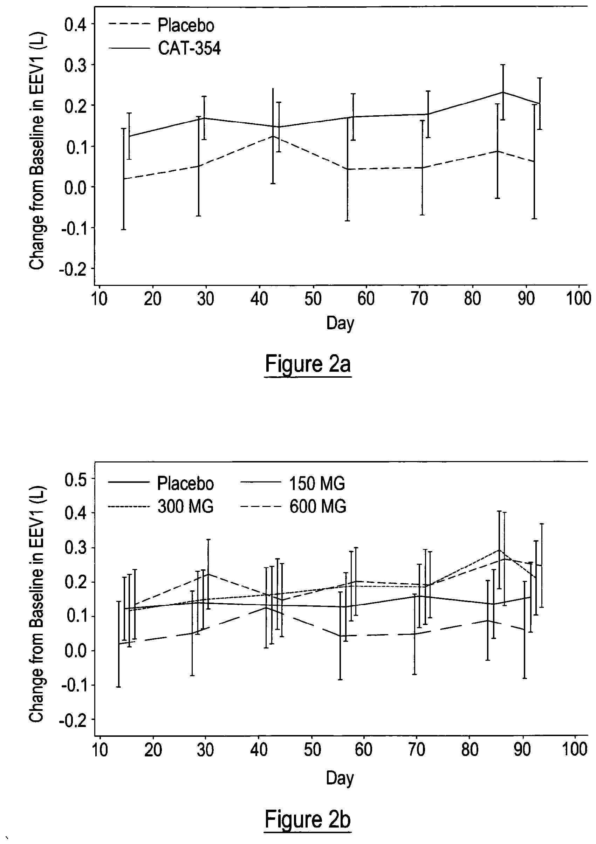

Table 5 summarizes the change from baseline in spirometry parameters to Study Day 92. The mean change from baseline in FEV₁ at day 92 was 0.061 L in the placebo group and 0.204 L in the combined SC tralokinumab treatment group; this difference was statistically significant (p = 0.044). This is shown graphically in Figure 2. The increases in FEV1 in the combined SC tralokinumab treatment group compared to placebo were observed as early as day 15 after the start of dosing, at this time point, the mean change from baseline in FEV₁ was 0.019 L in the placebo group and 0.124 L in the combined SC tralokinumab treatment group. At day 92, a dose response was seen across the SC tralokinumab treatment groups (0.155, 0.210, and 0.246 L, respectively). The mean percent changes in FEV₁ from baseline at Study Day 92 were 4.1% for the placebo group, 8.0% for the 150 mg SC tralokinumab treatment group, 13.3% for the 300 mg SC tralokinumab treatment group, 15.2% for the 600 mg SC tralokinumab treatment group, and 12.2% for the combined SC tralokinumab treatment group.

Sub-analyses were conducted to examine the effect of tralokinumab on FEV₁ in sub-populations within the study (Table 6). The magnitude of the mean change from baseline in FEV₁ in the combined SC tralokinumab treatment group was similar in subjects irrespective of their atopic status at baseline. For subjects with non-atopic asthma, the mean change from baseline in FEV₁ was 0.197 L in the combined SC tralokinumab treatment group (this difference was statistically significant compared to the placebo group (p = 0.002). For subjects with atopic asthma, the mean change from baseline in FEV₁ was 0.210 L in the combined SC tralokinumab treatment group; (this difference was not statistically significant compared to the placebo group).

**Table 5 Summary of change from baseline in spirometry parameters from baseline office visit evaluable population**

| PARAMETER (UNIT) | TREATMENT GROUP | | | BASELINE | VISIT | | CHANGE FROM BASELINE [1] | | | |
|---|---|---|---|---|---|---|---|---|---|---|
| | | VISIT | N | MEAN | MEAN | MEAN | SD | MED | MIN | MAX |
| FEV1 (L) | PLACEBO (N=46) | DAY 15 | 44 | 1.931 | 1.950 | 0.019 | 0.420 | -0.015 | -0.89 | 1.91 |
| | | DAY 29 | 46 | 1.947 | 1.998 | 0.051 | 0.424 | -0.005 | -0.48 | 2.03 |
| | | DAY 43 | 45 | 1.950 | 2.075 | 0.125 | 0.399 | 0.100 | -0.50 | 1.85 |
| | | DAY 57 | 45 | 1.956 | 2.000 | 0.043 | 0.436 | -0.060 | -0.93 | 1.87 |
| | | DAY 71 | 45 | 1.956 | 2.003 | 0.047 | 0.397 | 0.070 | -0.91 | 1.55 |
| | | DAY 85 | 45 | 1.956 | 2.043 | 0.087 | 0.397 | 0.020 | -0.59 | 1.86 |
| | | DAY 92 | 43 | 1.937 | 1.998 | 0.061 | 0.470 | 0.000 | -0.73 | 2.10 |
| | | | | | | | | | | |
| | CAI-354 150 MG (N=46) | DAY 15 | 46 | 2.178 | 2.300 | 0.122 | 0.326 | 0.065 | -0.40 | 1.07 |
| | | DAY 29 | 46 | 2.178 | 2.317 | 0.133 | 0.315 | 0.075 | -0.49 | 0.99 |
| | | DAY 43 | 44 | 2.205 | 2.336 | 0.131 | 0.380 | 0.075 | -0.45 | 1.92 |
| | | DAY 57 | 46 | 2.178 | 2.305 | 0.127 | 0.339 | 0.075 | -0.53 | 0.95 |
| | | DAY 71 | 45 | 2.191 | 2.349 | 0.153 | 0.314 | 0.090 | -0.32 | 0.80 |
| | | DAY 35 | 46 | 2.178 | 2.313 | 0.135 | 0.342 | 0.030 | -0.43 | 0.83 |
| | | DAY 92 | 45 | 2.168 | 2.322 | 0.155 | 0.345 | 0.130 | -0.39 | 1.21 |
| | | | | | | | | | | |
| | CAI-354 300 MG (N=51) | DAY 15 | 50 | 1.898 | 2.014 | 0.116 | 0.378 | 0.025 | -0.89 | 1.26 |
| | | DAY 29 | 49 | 1.907 | 2.056 | 0.149 | 0.303 | 0.110 | -0.46 | 1.09 |
| | | DAY 43 | 47 | 1.896 | 2.059 | 0.164 | 0.358 | 0.130 | -0.54 | 0.95 |
| | | DAY 57 | 43 | 1.890 | 2.078 | 0.188 | 0.353 | 0.120 | -0.44 | 1.04 |
| | | DAY 71 | 48 | 1.890 | 2.075 | 0.185 | 0.383 | 0.090 | -0.44 | 1.15 |
| | | DAY 85 | 47 | 1.890 | 2.181 | 0.291 | 0.395 | 0.230 | -0.31 | 1.28 |
| | | DAY 92 | 43 | 1.908 | 2.118 | 0.210 | 0.378 | 0.165 | -0.73 | 1.19 |
| | | | | | | | | | | |
| | CAI-354 600 MG (N=47) | DAY 15 | 46 | 1.996 | 2.130 | 0.134 | 0.349 | 0.080 | -0.51 | 1.52 |
| | | DAY 29 | 47 | 1.977 | 2.199 | 0.222 | 0.354 | 0.190 | -0.76 | 1.27 |
| | | DAY 43 | 47 | 1.977 | 2.124 | 0.147 | 0.373 | 0.070 | -0.43 | 1.53 |
| | | DAY 57 | 47 | 1.977 | 2.178 | 0.201 | 0.344 | 0.230 | -0.51 | 1.08 |
| | | DAY 71 | 47 | 1.977 | 2.167 | 0.190 | 0.335 | 0.130 | -0.65 | 0.99 |
| | | DAY 85 | 47 | 1.977 | 2.242 | 0.265 | 0.473 | 0.220 | -0.60 | 2.37 |
| | | DAY 92 | 45 | 1.952 | 2.198 | 0.246 | 0.418 | 0.260 | -0.55 | 1.74 |

| PARAMETER (UNIT) | TREATMENT GROUP | | | BASELINE | VISIT | CHANGE FROM BASELINE [1] | | | | |
|---|---|---|---|---|---|---|---|---|---|---|
| | | VISIT | N | MEAN | MEAN | MEAN | SD | MED | MIN | MAX |
| FEV1 (L) | CAT-354 TOTAL (N=144) | DAY 15 | 142 | 2.020 | 2.144 | 0.124 | 0.347 | 0.065 | -0.89 | 1.52 |
| | | DAY 29 | 142 | 2.018 | 2.188 | 0.170 | 0.324 | 0.120 | -0.76 | 1.27 |
| | | DAY 43 | 133 | 2.022 | 2.170 | 0.148 | 0.368 | 0.090 | -0.54 | 1.92 |
| | | DAY 57 | 141 | 2.013 | 2.185 | 0.172 | 0.344 | 0.160 | -0.53 | 1.08 |
| | | DAY 71 | 140 | 2.016 | 2.194 | 0.178 | 0.344 | 0.120 | -0.65 | 1.15 |
| | | DAY 85 | 140 | 2.014 | 2.245 | 0.231 | 0.410 | 0.175 | -0.60 | 2.37 |
| | | DAY 92 | 133 | 2.007 | 2.211 | 0.204 | 0.380 | 0.150 | -0.73 | 1.74 |
| | | | | | | | | | | |
| FVC (L) | PLACEBO (N=46) | DAY 15 | 44 | 3.233 | 3.237 | 0.005 | 0.472 | -0.050 | -0.81 | 1.86 |
| | | DAY 29 | 46 | 3.261 | 3.260 | -0.001 | 0.504 | -0.090 | -0.63 | 2.11 |
| | | DAY 43 | 45 | 3.279 | 3.330 | 0.051 | 0.452 | 0.010 | -0.84 | 1.50 |
| | | DAY 57 | 45 | 3.264 | 3.279 | 0.015 | 0.531 | 0.000 | -0.92 | 2.15 |
| | | DAY 71 | 45 | 3.264 | 3.299 | 0.035 | 0.500 | -0.030 | -1.15 | 1.75 |
| | | DAY 85 | 45 | 3.264 | 3.320 | 0.056 | 0.493 | -0.050 | -0.70 | 2.03 |
| | | DAY 92 | 43 | 3.269 | 3.273 | 0.005 | 0.541 | -0.050 | -1.02 | 2.19 |
| | | | | | | | | | | |
| | CAT=354 150 MG (N=46) | DAY 15 | 46 | 3.534 | 3.626 | 0.092 | 0.371 | 0.100 | -0.65 | 0.99 |
| | | DAY 29 | 46 | 3.534 | 3.646 | 0.112 | 0.384 | 0.060 | -0.90 | 1.12 |
| | | DAY 43 | 44 | 3.559 | 3.710 | 0.152 | 0.503 | 0.115 | -0.61 | 2.54 |
| | | DAY 57 | 46 | 3.534 | 3.726 | 0.192 | 0.389 | 0.135 | -0.51 | 1.10 |
| | | DAY 71 | 45 | 3.535 | 3.694 | 0.158 | 0.432 | 0.040 | -0.62 | 1.15 |
| | | DAY 85 | 46 | 3.534 | 3.655 | 0.121 | 0.451 | 0.020 | -0.69 | 1.48 |
| | | DAY 92 | 45 | 3.536 | 3.723 | 0.187 | 0.448 | 0.140 | -0.71 | 1.81 |
| FVC (L) | CAT-354 300 MG (N=51) | DAY 15 | 50 | 3.082 | 3.159 | 0.076 | 0.422 | 0.060 | -0.71 | 1.09 |
| | | DAY 29 | 49 | 3.107 | 3.185 | 0.077 | 0.300 | 0.080 | -0.66 | 0.87 |
| | | DAY 43 | 47 | 3.119 | 3.238 | 0.119 | 0.350 | 0.050 | -0.66 | 0.99 |
| | | DAY 57 | 48 | 3.094 | 3.210 | 0.116 | 0.309 | 0.065 | -0.52 | 0.83 |
| | | DAY 71 | 48 | 3.094 | 3.218 | 0.125 | 0.389 | 0.090 | -0.94 | 1.22 |
| | | DAY 55 | 47 | 3.106 | 3.316 | 0.210 | 0.431 | 0.170 | -0.73 | 1.57 |
| | | DAY 92 | 48 | 3.113 | 3.235 | 0.122 | 0.463 | 0.070 | -0.82 | 1.74 |
| | | | | | | | | | | |
| | CAT-354 600 MG (N=47) | DAY 15 | 46 | 3.206 | 3.310 | 0.105 | 0.482 | 0.040 | -0.92 | 1.40 |
| | | DAY 29 | 47 | 3.176 | 3.371 | 0.196 | 0.483 | 0.130 | -0.81 | 1.36 |
| | | DAY 43 | 47 | 3.176 | 3.341 | 0.166 | 0.553 | 0.050 | -1.14 | 1.82 |
| | | DAY 57 | 47 | 3.176 | 3.324 | 0.148 | 0.516 | 0.170 | -1.25 | 1.23 |
| | | DAY 71 | 47 | 3.176 | 3.344 | 0.168 | 0.544 | 0.040 | -1.59 | 1.47 |
| | | DAY 85 | 47 | 3.176 | 3.387 | 0.211 | 0.571 | 0.190 | -0.95 | 2.43 |
| | | DAY 92 | 45 | 3.139 | 3.330 | 0.191 | 0.554 | 0.220 | -1.71 | 1.71 |
| | | | | | | | | | | |
| | CAI-354 TOTAL (N=144) | DAY 15 | 142 | 3.269 | 3.359 | 0.090 | 0.424 | 0.070 | -0.92 | 1.40 |
| | | DAY 29 | 142 | 3.268 | 3.396 | 0.128 | 0.396 | 0.080 | -0.90 | 1.36 |
| | | DAY 43 | 138 | 3.278 | 3.424 | 0.145 | 0.473 | 0.065 | -1.14 | 2.54 |
| | | PAY 57 | 141 | 3.265 | 3.416 | 0.152 | 0.411 | 0.120 | -1.25 | 1.23 |
| | | DAY 71 | 140 | 3.263 | 3.413 | 0.150 | 0.457 | 0.055 | -1.59 | 1.47 |
| | | DAY 85 | 140 | 3.270 | 3.451 | 0.181 | 0.487 | 0.150 | -0.95 | 2.43 |
| | | DAY 92 | 136 | 3.259 | 3.425 | 0.166 | 0.487 | 0.135 | -1.71 | 1.81 |
| PEF (L/MIN) | PLACEBO (N=46) | DAY 15 | 44 | 283.5 | 289.9 | 6.3 | 53.2 | -1.5 | -112 | 168 |
| | | DAY 29 | 46 | 284.4 | 291.8 | 7.4 | 49.0 | -4.5 | -58 | 181 |
| | | DAY 43 | 45 | 286.2 | 305.2 | 19.0 | 49.5 | 12.0 | -79 | 153 |
| | | DAY 57 | 45 | 287.2 | 294.5 | 7.2 | 53.6 | -4.0 | -122 | 172 |
| | | DAY 71 | 45 | 287.2 | 299.9 | 12.7 | 60.0 | 13.0 | -123 | 174 |
| | | DAY 85 | 45 | 287.2 | 381.7 | 14.5 | 47.6 | 7.0 | -89 | 171 |
| | | DAY 92 | 43 | 285.9 | 299.2 | 13.4 | 59.7 | 1.0 | -100 | 282 |
| | | | | | | | | | | |
| | CAI-354 150 MG (N=46) | DAY 15 | 46 | 338.3 | 353.5 | 15.2 | 51.0 | 7.0 | -82 | 129 |
| | | DAY 29 | 46 | 338.3 | 357.4 | 19.1 | 54.3 | 13.5 | -90 | 136 |
| | | DAY 43 | 44 | 345.5 | 364.3 | 18.8 | 59.1 | 11.5 | -89 | 221 |
| | | DAY 57 | 46 | 338.3 | 361.0 | 22.7 | 57.3 | 16.5 | -72 | 164 |
| | | DAY 71 | 45 | 341.5 | 372.8 | 31.3 | 59.8 | 17.0 | -97 | 157 |
| | | | | | | | | | | |
| | | DAY 85 | 46 | 338.3 | 365.5 | 27.2 | 65.0 | 17.0 | -101 | 160 |
| | | DAY 92 | 45 | 339.2 | 367.4 | 28.2 | 60.6 | 34.0 | -89 | 149 |
| | CAI-354 300 MG (N=51) | DAY 15 | 50 | 289.7 | 387.0 | 17.2 | 58.1 | 7.5 | -170 | 183 |
| | | DAY 29 | 49 | 293.5 | 311.4 | 17.9 | 61.7 | 13.0 | -123 | 206 |
| | | DAY 43 | 47 | 292.1 | 388.4 | 16.3 | 58.7 | 10.0 | -105 | 223 |
| | | DAY 57 | 48 | 289.0 | 310.3 | 21.3 | 60.3 | 5.0 | -93 | 201 |
| | | DAY 71 | 48 | 289.0 | 313.1 | 24.1 | 62.8 | 23.0 | -78 | 211 |
| | | DAY 85 | 47 | 289.3 | 332.7 | 43.4 | 63.7 | 23.0 | -56 | 237 |
| | | DAY 92 | 48 | 292.1 | 322.4 | 30.3 | 62.1 | 20.5 | -127 | 194 |
| | | | | | | | | | | |
| | CAI-354 600 MG (N=47) | DAY 15 | 46 | 316.8 | 340.7 | 23.8 | 57.8 | 25.5 | -81 | 214 |
| | | DAY 29 | 47 | 314.0 | 349.0 | 35.0 | 61.1 | 27.0 | -94 | 197 |
| | | DAY 43 | 47 | 314.0 | 331.8 | 17.8 | 58.8 | 16.0 | -98 | 181 |
| | | DAY 57 | 47 | 314.0 | 340.1 | 26.1 | 57.0 | 32.0 | -102 | 141 |
| | | DAY 71 | 47 | 314.0 | 347.7 | 33.7 | 59.9 | 31.0 | -94 | 164 |
| | | DAY 85 | 47 | 314.0 | 350.3 | 36.3 | 77.5 | 26.0 | -114 | 313 |
| | | DAY 92 | 45 | 311.8 | 347.0 | 35.2 | 66.7 | 29.0 | -97 | 237 |

| | | | | | | | | | | |
|---|---|---|---|---|---|---|---|---|---|---|
| [I] CHANGE FROM BASELINE = CURRENT VISIT VALUE - BASELINE VALUE. | | | | | | | | | | |

Table 6

**TABLE 6**

| CHANGE FROM BASELINE IN FEV1 AND PEF FROM OFFICE VISIT AT DAY 92[1] EVALUABLE POPULATION | | | | | | | |
|---|---|---|---|---|---|---|---|
| | | | PLACEBO (N=46) | CAT-354 150 MG (N=46) | CAT-354 300 MG (N=51) | CAT-354 600 MG (N=47) | CAT-354 TOTAL (N=144) |
| FEV1 (L) | ALL | N | 43 | 45 | 48 | 45 | 138 |
| | | MEAN | 0.061 | 0.155 | 0.210 | 0.246 | 0.204 |
| | | SD | 0.470 | 0.345 | 0.378 | 0.418 | 0.380 |
| | | MEDIAN | 0.000 | 0.130 | 0.165 | 0.260 | 0.150 |
| | | MIN - MAX | (-0.73-2.10) | (-0.39-1.21) | (-0.73-1.19) | (-0.55-1.74) | (-0.73-1.74) |
| | | P-VALUE [2] | | 0.289 | 0.097 | 0.055 | 0.044 |
| | SUBJECTS WITH ATOPIC ASTHMA | N | 21 | 23 | 26 | 25 | 74 |
| | | MEAN | 0.172 | 0.268 | 0.178 | 0.189 | 0.210 |
| | | SD | 0.607 | 0.371 | 0.414 | 0.400 | 0.393 |
| | | MEDIAN | 0.060 | 0.150 | 0.140 | 0.170 | 0.155 |
| | | MIN - MAX | (-0.65-2.10) | (-0.30-1.21) | (-0.73-1.19) | (-0.55-1.07) | (-0.73-1.21) |
| | | P-VALUE [2] | | 0.527 | 0.970 | 0.913 | 0.737 |
| | SUBJECTS WITH NON-ATOPIC ASTHMA | N | 22 | 22 | 22 | 20 | 64 |
| | | MEAN | -0.045 | 0.036 | 0.249 | 0.317 | 0.197 |
| | | SD | 0.259 | 0.277 | 0.335 | 0.439 | 0.368 |
| | | MEDIAN | -0.020 | 0.000 | 0.180 | 0.290 | 0.135 |
| | | MIN - MAX | (-0.73-0.54) | (-0.39-0.89) | (-0.28-1.04) | (-0.43-1.74) | (-0.43-1.74) |
| | | P-VALUE [2] | | 0.323 | 0.002 | 0.002 | 0.006 |
| | SUBJECTS IN LOWER TERTILE OF BASELINE MEAN ACQ | N | 23 | 19 | 24 | 20 | 63 |
| | | MEAN | 0.127 | 0.182 | 0.168 | 0.266 | 0.203 |
| | | SD | 0.569 | 0.301 | 0.339 | 0.308 | 0.316 |
| | | MEDIAN | 0.040 | 0.150 | 0.140 | 0.370 | 0.210 |
| | | MIN - MAX | (-0.73-2.10) | (-0.35-0.89) | (-0.73-0.97) | (-0.43-0.67) | (-0.73-0.97) |
| | | P-VALUE [2] | | 0.708 | 0.764 | 0.335 | 0.434 |
| | SUBJECTS IN MIDDLE TERTILE OF BASELINE MEAN ACQ | N | 8 | 10 | 12 | 13 | 35 |
| | | MEAN | 0.074 | 0.165 | 0.269 | 0.141 | 0.192 |
| | | SD | 0.260 | 0.299 | 0.457 | 0.357 | 0.374 |
| FEV1 (L) | SUBJECTS IN MIDDLE TERTILE OF BASELINE MEAN ACQ | MEDIAN | 0.005 | 0.125 | 0.200 | 0.130 | 0.140 |
| | | MIN - MAX | (-0.23-0.54) | (-0.27-0.71) | (-0.57-1.04) | (-0.46-1.07) | (-0.57-1.07) |
| | | P-VALUE [2] | | 0.506 | 0.290 | 0.652 | 0.404 |
| | SUBJECTS IN UPPER TERTILE OF BASELINE MEAN ACQ | N | 12 | 16 | 12 | 12 | 40 |
| | | MEAN | -0.073 | 0.116 | 0.237 | 0.326 | 0.215 |
| | | SD | 0.358 | 0.429 | 0.390 | 0.611 | 0.476 |
| | | MEDIAN | -0.140 | 0.030 | 0.135 | 0.140 | 0.075 |
| | | MIN - MAX | (-0.65-0.66) | (-0.39-1.21) | (-0.12-1.19) | (-0.55-1.74) | (-0.55-1.74) |
| | | P-VALUE [2] | | 0.226 | 0.055 | 0.064 | 0.059 |
| | SUBJECTS WITH % OF PREDICTED BASELINE FEV1 <= 60% | N | 19 | 22 | 22 | 24 | 68 |
| | | MEAN | 0.154 | 0.128 | 0.289 | 0.378 | 0.268 |
| | | SD | 0.596 | 0.326 | 0.322 | 0.446 | 0.381 |
| | | MEDIAN | 0.030 | 0.130 | 0.210 | 0.285 | 0.200 |
| | | MIN - MAX | (-0.65-2.10) | (-0.35-0.89) | (-0.22-1.04) | (-0.46-1.74) | (-0.46-1.74) |
| | | P-VALUE [2] | | 0.858 | 0.364 | 0.166 | 0.316 |
| | SUBJECTS WITH HIGH DOSE OF ICS OR OCS AT BASELINE | N | 11 | 17 | 13 | 16 | 46 |
| | | MEAN | | 0.112 | 0.128 | 0.313 | 0.186 |
| | | SD | 0.209 0.756 | 0.455 | 0.334 | 0.481 | 0.435 |
| | | MEDIAN | -0.030 | 0.000 | 0.090 | 0.370 | 0.115 |
| | | MIN - MAX | (-0.73-2.10) | (-0.39-1.21) | (-0.28-1.04) | (-0.55-1.74) | (-0.55-1.74) |
| | | P-VALUE [2] | | 0.675 | 0.729 | 0.667 | 0.894 |

| | | | | | | | |
|---|---|---|---|---|---|---|---|
| [1] CHANGE FROM BASELINE = CURRENT VISIT VALUE - BASELINE VALUE. [2] P-VALUES ARE CALCULATED USING AN ANALYSIS OF VARIANCE. REFER TO SUPPORTING DATA LISTING(S) 16. | | | | | | | |

The magnitude of the mean change from baseline in FEV₁ of 0.186 L in subjects receiving high dose inhaled corticosteroids and/or oral corticosteroids as background asthma controller therapy within the combined SC tralokinumab treatment group was similar compared to the overall study population (this difference was not statistically significant compared to the placebo group).

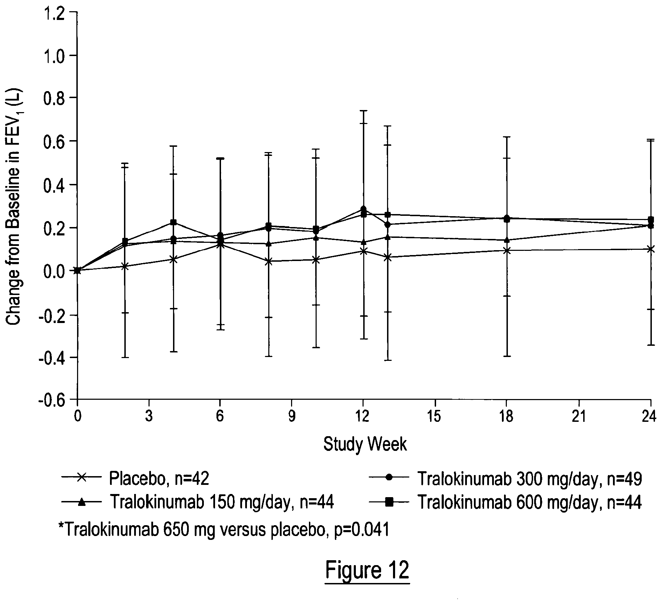

Pre-bronchodilator FEV₁, FVC, and PEF improved in all tralokinumab groups over the 12-week treatment period compared with placebo (table 8). The mean [SD] percent increases from baseline to week 13 in FEV₁ (12·5% [20·7%] *vs* 4·3% [29·4%]) and FVC (7·1% [16·8%] *vs* 1·5% [21·0%]) were greater for tralokinumab versus placebo. The effect on FEV₁ was evident at 2 weeks and persisted until week 24 (figure 12). At week 13 a dose response was evident, though qualitatively similar FEV₁ increases were observed in the tralokinumab 300 and 600 mg groups during the last 6 weeks of treatment. Similar mean [SD] percent increases in FEV₁ were seen at week 13 in subjects with atopic (12·9% [21·2%]) and non-atopic (12·2% [20·2%]) asthma who received tralokinumab.

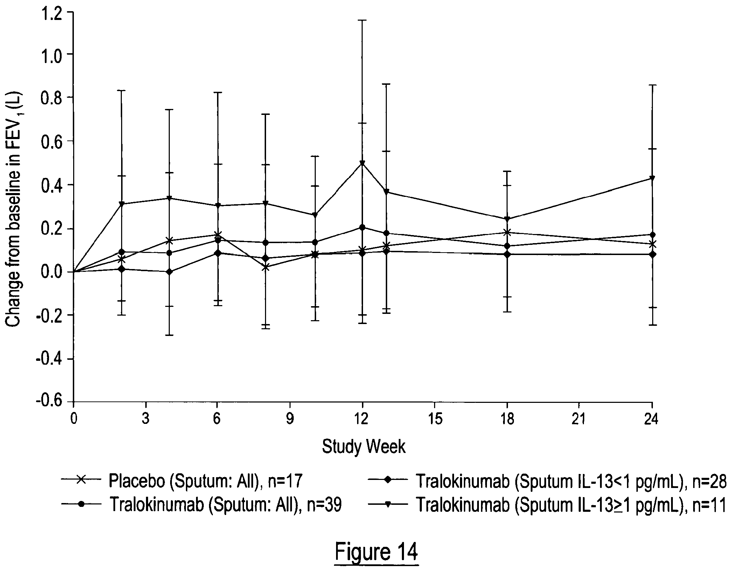

Changes in FEV₁ in those subjects providing a sputum sample at baseline, stratified by the presence or absence of IL-13, are presented in figure 14. The week 13 mean [SD] improvement in the sputum IL-13-positive (≥1pg/mL) tralokinumab group (9.9 [11.4] % n=11) was greater than placebo (3.9 [9.0] % n=15) or sputum IL-13-negative (<1 pg/mL) tralokinumab group (2.9 [8.9] % n=26).

### Rescue β2 Agonist Use

Table 7 summarizes the use of rescue beta2 agonist and the proportion of reliever-free days comparing the week preceding baseline and the week preceding Study Day 92 across the placebo and SC tralokinumab treatment groups. The mean number of rescue beta2 agonist puffs was similar at both time points in the placebo group (2.58 puffs at baseline and 2.5 puffs at Study Day 92). In contrast, the mean number of rescue beta2 agonist puffs was lower at Study Day 92 as compared to baseline in the combined SC tralokinumab treatment group (2.48 puffs at baseline and 1.8 puffs at Study Day 92).

The mean proportion of reliever-free days in a week in the placebo group was 13.2% at baseline and 22.0% at Study Day 92 for a difference of 8.8%. In the combined SC tralokinumab treatment group, the mean proportion of reliever-free days in a week was 17.4% at baseline and 38.7% at Study Day 92 for a difference of 21.3%.

Subjects in the combined tralokinumab group showed a significantly greater mean [SD] reduction in β₂-agonist use compared with placebo at week 13 (-0-68 [1·45] *vs* -0·10 [1·49] puffs/day; p=0·020) and week 24 (-0·73 [1·57] *vs* - 0-05 [1·87] puffs/day; p=0·025). The mean proportion of reliever-free days/week increased during the study in the combined tralokinumab versus placebo groups (17·4% *vs* 13·2%, 38·7% *vs* 22·0%, 38·6% *vs* 28·6% at baseline, week 13, week 24, respectively).

**Table 7 Summary of Rescue Beta2 Agonist Use and Reliever-free Days at Baseline and at Study Day 92 (Evaluable Population)**

| Parameter | Placebo (N=46) | SC TRALOKINUMAB | | | |
|---|---|---|---|---|---|
| | | 150 mg (N=46) | 300 mg (N=51) | 600 mg (N=47) | Combined (N= 144) |
| Rescue Beta2 Agonist Use (Average Number of Puffs) | | | | | |
| Baseline | | | | | |
| N | 46 | 46 | 51 | 47 | 144 |
| Mean | 2.58 | 2.84 | 2.04 | 2.62 | 2.48 |
| SD | 1.70 | 2.03 | 1.51 | 1.96 | 1.86 |
| Median | 2.14 | 2.07 | 1.86 | 2.00 | 2.00 |
| Min-Max | (0.0-7.4) | (0.0-7.7) | (0.0-6.6) | (0.0-9.1) | (0.0-9.1) |
| Study Days 86-92 | | | | | |
| N | 46 | 46 | 50 | 47 | 143 |
| Mean | 2.5 | 2.0 | 1.8 | 1.7 | 1.8 |
| SD | 2.3 | 2.1 | 2.0 | 1.7 | 2.0 |
| Median | 2.0 | 1.8 | 1.3 | 1.7 | 1.4 |
| Min-Max | (0-8) | (0-8) | (0-10) | (0-6) | (0-10) |
| Proportion of Reliever-free Days in a Week (%) | | | | | |
| Baseline | | | | | |
| N | 46 | 46 | 51 | 47 | 144 |
| Mean | 13.2 | 12.1 | 24.1 | 15.3 | 17.4 |
| SD | 24.8 | 25.5 | 34.6 | 28.1 | 30.1 |
| Median | 0.0 | 0.0 | 0.0 | 0.0 | 0.0 |
| Min-Max | (0-100) | (0-100) | (0-100) | (0-100) | (0-100) |
| Study Days 86-92 | | | | | |
| N | 46 | 46 | 50 | 47 | 143 |
| Mean | 22.0 | 37.3 | 40.6 | 38.0 | 38.7 |
| SD | 36.5 | 43.3 | 43.6 | 45.2 | 43.8 |
| Median | 0.0 | 0.0 | 28.6 | 0.0 | 0.0 |
| Min-Max | (0-100) | (0-100) | (0-100) | (0-100) | (0-100) |

| | | | | | |
|---|---|---|---|---|---|
| Max = maximum; Min = minimum; N = number; PEF = peak expiratory flow; SD = standard deviation. | | | | | |

### Efficacy Conclusions

A statistically significant increase in FEV₁ from baseline was seen in the combined SC tralokinumab treatment group compared to placebo at Study Day 92. The magnitude of this change (0.204 L or 12.2%) is clinically important, particularly as it was observed in addition to currently available asthma controller medications. The treatment effect was observed irrespective of atopic status and also in subjects receiving high doses of inhaled corticosteroids and/or oral steroids.

The increase in FEV₁ appeared to be dose dependant, with the greatest increases seen in the 600 and 300 mg SC tralokinumab treatment groups. Increases from baseline in PEF and FVC were also observed in the combined SC tralokinumab treatment group.

A reduction in the requirement for the use of additional short acting beta2 agonist was observed in the combined SC tralokinumab treatment group that is consistent with the improvement in FEV₁ described above.

### Primary endpoint - change in ACQ-6 score from baseline to week 13

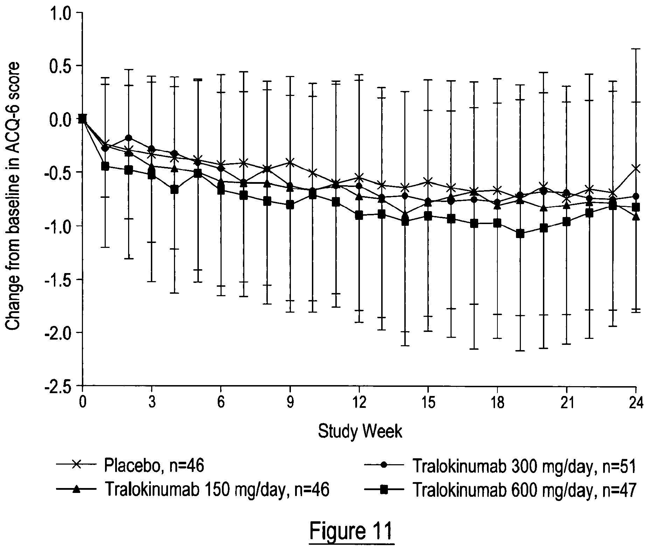

Mean ACQ-6 score improved from baseline to week 13 in all treatment groups (table 8; figure 11), with 84/144 (58·3%) tralokinumab and 24/46 (52·2%) placebo subjects showing improvement of ≥0·5. The mean [SD] reduction in the combined tralokinumab group (-0·76 [1·04]) at week 13 was not different from placebo (-0·61 [0·90]; p=0·375). The difference (95% CI) between treatment groups in change from baseline was -0·15 (-0-49, 0·19). Reductions in ACQ-6 score were maintained through week 24 in both groups although there was no difference in the mean [SD] between the tralokinumab (-0·79 [0·98]) and placebo (-0·60 [1·00]; p=0·256) groups.

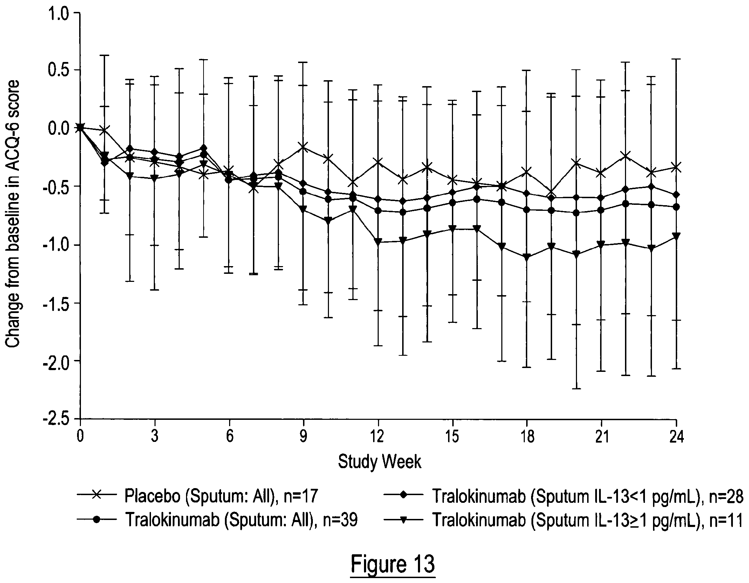

However, a difference was observed in ACQ-6 score in those subjects providing a sputum sample at baseline, stratified by the presence or absence of IL-13, this is presented in figure 13; the week 13 mean [SD] reduction in ACQ-6 in the sputum IL-13-positive (≥1pg/mL) tralokinumab group (-0·97 [0.98] n=11) was greater than that in the placebo (-0·43 [0·70] n=17) or sputum IL-13-negative (<1pg/mL) tralokinumab group (-0.62 [0.85] n=28).

**Table 8 Summary of change from baseline in mean ACQ-6 score and spirometry parameters at office visits at week 13 (evaluable population)**

| | **Placebo** | **Tralokinumab** | | | |
|---|---|---|---|---|---|
| | | **150 mg** | **300 mg** | **600 mg** | **Combined tralokinumab** |
| **Mean ACQ-6 score change from baseline** | | | | | |
| n | 46 | 46 | 51 | 47 | 144 |
| Mean (SD) | -0·61 (0·90) | -0·73 (1·12) | -0·70 (0·93) | -0·86 (1·09) | -0·76 (1·04) |
| Difference (95% CI)* | - | -0·12 (-0·54, 0·30) | -0·09 (-0-46, 0·28) | -0·25 (-0·66, 0·16) | -0·15 (-0-49, 0·19) |
| p value† | - | 0·573 | 0·640 | 0·224 | 0·375 |

| **FEV₁ (L) change from baseline** | | | | | |
|---|---|---|---|---|---|
| n | 42 | 44 | 49 | 44 | 137 |
| Mean (SD) | 0·06 (0-48) | 0·16 (0·35) | 0·21 (0·37) | 0·26 (0·41) | 0·21 (0·38) |
| Difference (95% CI)* | - | 0·09 (-0·09, 0·27) | 0·15 (-0·03, 0·32) | 0·20 (0·01, 0·39) | 0·15 (-0-01, 0-31) |
| p value‡ | - | 0·299 | 0·102 | 0·041 | 0·072 |
| Mean (SD) % change§ | 4-3 (29·4) | 8·1 (17·5) | 13·3 (21·8) | 16·1 (22·0) | 12·5 (20·7) |

| **FVC (L) change from baseline** | | | | | |
|---|---|---|---|---|---|
| n | 42 | 44 | 49 | 44 | 137 |
| Mean (SD) | 0·00 (0·55) | 0·18 (0·45) | 0·12 (0·46) | 0·21 (0·54) | 0·17 (0-48) |
| Difference (95% CI)* | - | 0·18 (-0·04, 0·39) | 0·12 (-0·09, 0·33) | 0·21 (-0·03, 0·44) | 0·17 (-0·01, 0·34) |
| p-value‡ | - | 0·100 | 0·261 | 0·082 | 0·059 |
| Mean (SD) % change§ | 1·5 (21·0) | 6·4 (15·0) | 5·5 (16·4) | 9·6 (18·8) | 7·1 (16·8) |

| **PEF (L/min) change from baseline** | | | | | |
|---|---|---|---|---|---|
| n | 42 | 44 | 49 | 44 | 137 |
| Mean (SD) | 14-2 (60·1) | 27·5 (61·1) | 30·0 (61·5) | 38·2 (64·3) | 31·8 (62·0) |
| Difference (95% CI)* | - | 13·3 (-12·7, 39·3) | 15·8 (-9·7, 41·2) | 24·0 (-2·7, 50·7) | 17·6 (-3·8, 39.0) |
| p value‡ | - | 0·313 | 0·222 | 0·078 | 0·107 |
| Mean (SD) % change§ | 5·4 (23·6) | 11·0 (23·6) | 14·1 (27·5) | 16·5 (25·7) | 13·9 (25·6) |
| ACQ-6=Asthma Control Questionnaire, mean of 6 individual item scores. CI=confidence interval. FEV₁=forced expiratory volume in 1 s. FVC=forced vital capacity. PEF=peak expiratory flow. SD=standard deviation. *Difference in mean change from baseline: tralokinumab-placebo. †p values were based on analysis of variance. ‡p values were calculated using a 2-sample t test. §Change from baseline: current visit value-baseline value. | | | | | |

### Safety and Tolerability Conclusions

The majority of subjects received all 7 doses of investigational product and there was a low incidence of subjects dropping-out of the study. tralokinumab administered as a SC injection at doses of 150, 300, and 600 mg every other week for 12 weeks was generally well tolerated. No major safety issues were identified.

### Post-treatment follow-up

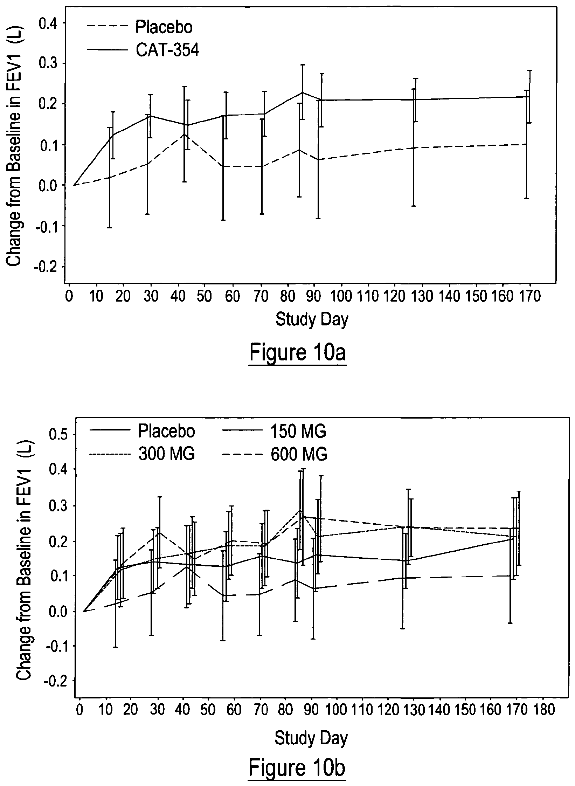

All subjects were followed for 12 weeks after the last dose of investigational product and underwent spirometry at days 127 and day 169. The increases in FEV₁ that were observed in tralokinumab-treated subjects at day 92 were maintained at both these post-doing time points. The mean change from baseline in FEV₁ at day 169 was 0.101 L in the placebo group and 0.218 L in the combined SC tralokinumab treatment group (p = 0.096). The numerical increases in FVC and PEF observed at day 92 were also maintained to day 169. This finding suggests that the beneficial effects of tralokinumab on lung function may be sustained for a prolonged period after cessation of treatment (see figures 10a & 10b).

### Summary

Evidence of clinical effect was observed following the addition of tralokinumab to standard asthma medications in subjects with uncontrolled moderate-to-severe persistent asthma. The most compelling signal was the statistically significant increase in FEV₁ at day 92 that was also supported by the improvements in FVC, PEF and reduction in the use of short-acting beta2 agonist. The improvement in airflow obstruction was observed as early as day 15 following randomisation. As regards differentiation between doses of tralokinumab, the largest treatment effects were observed in the 600 mg SC tralokinumab treatment group but clinically important changes in FEV₁ were also observed in the 300 mg SC tralokinumab treatment group. The improvement in FEV₁ in the tralokinumab treatment group was maintained in the 12-week post-treatment follow-up period. A sub-set of subjects stratified into sputum IL-13 positive or negative demonstrated an association between presence of IL-13 in sputum and response to tralokinumab as assessed by ACQ-6.

A growing body of evidence implicates IL-13 as a key mediator in the development and maintenance of asthma. This study investigated the additive effect of tralokinumab, a potent and specific IL-13-neutralising human IgG4 monoclonal antibody, to currently available asthma controller therapy in subjects with uncontrolled moderate-to-severe asthma. The increases in FEV₁ and FVC and reduction in the use of short-acting β₂-agonists in tralokinumab-treated subjects are indicative of a treatment effect. The magnitude of increase in FEV₁ approximates the minimal important difference of 10% (Reddel et al., (2009)) suggesting that this antibody is able to deliver a clinically important benefit.

The use of tralokinumab resulted in a reduction in the need for 'rescue' short acting β agonists (SABA) that is consistent with the improvement observed in FEV₁. The largest effect size was observed in 600 mg treatment group. The beneficial effect on FEV₁ was observed irrespective of atopic status at baseline and also in subjects receiving high doses of inhaled corticosteroids and/or oral steroids and persisted for 12 weeks following the last treatment.

As asthma is a heterogeneous disease, identification of patient subgroups or individual patient/disease characteristics is important in delivering optimal benefit with biotherapeutics that, by design, target specific mechanisms. We postulated that subjects who have upregulated airway IL-13 may benefit most from treatment with IL-13-neutralising therapy. We explored this concept in our study by examining 56 subjects who supplied a baseline sputum sample. When subjects were stratified into sputum IL-13 positive or negative there was an association between presence of IL-13 and response to tralokinumab (figures 13 and 14). Taken together these data demonstrate that subjects with upregulated lung IL-13 receive the greatest clinical benefits of IL-13 neutralising therapies such as tralokinumab.

### Example 2: Measurement of IL-13 in sputum

### Assay Description

Free IL-13 was measured using the Luminex technology (Millipore, Billerica, MA). Necessary reagents were supplied in the assay kit, all steps were conducted at room temperature, and the assay plate was sealed and placed on a plate shaker with moderate shaking for all incubation steps. Briefly, wells were pre-wet with 200µl/well of Assay Buffer for ≥ 10 minutes. Anti-IL-13 antibody-conjugated bead was prepared in Bead Dilution Buffer and added to the assay plate at 25µl/well; vacuum was applied to remove the Bead Dilution Buffer. Reference standards (RS), quality controls (QC), and negative control (NC) prepared in Assay Buffer were added to the assay plate at 25µl/well. Test samples (neat serum) were added at 25µl/well. The assay plate was incubated for 60 ± 10 minutes. Unbound analyte was removed by washing the plate twice with 1x Wash Buffer. To detect bound analyte, biotin-conjugated anti-IL-13 detection antibody was added and the assay plate was incubated for 60 ± 10 minutes. PE-conjugated Streptavidin dye (SA-PE) was then added at 25µl/well and the assay plate was incubated for an additional 30 ± 5 minutes. Excess detection antibody and dye was removed by washing the plate twice with 1x Wash Buffer. Luminex Sheath Fluid was added at 150µl/well and the plate was placed on a plate shaker for ≥ 5 minutes prior to reading. The fluorescent intensity, measured in the Luminex200 Plate reader, was proportional to the IL-13 concentration in the sample. The presence of ≥ 1pg/ml IL-13 in sputum from a subject with asthma was indicative that the subject had detectable IL-13 in sputum ("sputum IL-13 positive"). A value of <1 pg/ml IL-13 in sputum was indicative that the subject did not have detectable IL-13 in sputum and thus that the subject was "sputum IL-13-negative".

### Reagents

1. MPXHCYTO-60K (Luminex Kit Cat# MPXHCYTO-60K):
   Filter Assay Plate: Cat# MX-PLATE
   Anti-IL-13 antibody-conjugated beads: Cat# MXHIL-13, bead region 50, 50x stock
   Reference Standards, rh IL-13: Cat# MXH8060
   Quality Control # 1 & #2: MXH6060 & MXH6060-2
   Biotin-conjugated anti-IL-13 detection antibody: Cat# MXH1060-1, 1x stock
   Streptavidin-Phycoerythrin detection dye: Cat# L-SAPE9, 1x stock Buffers: Assay Buffer (1x), Bead Dilution Buffer (1x), Wash Buffer (25x)
2. Luminex Sheath Fluid Cat# 40-50000

### Assay Protocol

Assay reagents were equilibrated to room temperature. An aliquot of 200µl of Assay Buffer was transferred to each well of a 96-well filter assay plate, the plate was sealed and incubated on a plate shaker for ≥ 10 minutes. Eight levels of reference standard (RS) were prepared by diluting the recombinant human IL-13 reference standards in Assay Buffer. The following concentrations were used: 2500.0, 833.3, 277.8, 92.6, 30.9, 10.3, 3.4 and 1.1 pg/ml. Three 3 levels of quality control samples (QC) were prepared in Assay Buffer. The following concentrations were used: 1000.0, 10.0 pg/ml. The negative control (NC) (0.0 ng/ml) was prepared with the Assay Buffer. The test serum samples were thawed on ice and vortexed briefly. The anti-IL-13 antibody-conjugated bead was diluted 1:50 in Bead Dilution buffer. A vacuum was applied to remove the buffer, the bottom of the plate was blotted on absorbent paper. An aliquot of 25µl/well of the diluted bead solution was transferred to the plate. Vacuum was applied to the plate as above. Aliquots of 25µl/well of the RS, QC, NC, and serum samples were transferred to the plate; which was then sealed and incubated for 60 ± 10 minutes on a rotating platform with moderate shaking. 1X Wash Buffer was prepared from the 25X Wash Buffer stock. The plate was washed twice with 200µl/well of 1X Wash Buffer. An aliquot of 25µl/well of the biotin-conjugated anti-IL-13 detection antibody was transferred to the plate; the plate was sealed and incubated for 60 ± 10 minutes on a rotating platform with moderate shaking. An aliquot of 25µl/well of the Streptavidin dye (SA-PE) was transferred to the plate; the plate was sealed and incubated for 30 ± 5 minutes on a rotating platform with moderate shaking. The plate was washed twice with 200µl/well of 1X Wash Buffer. An aliquot of 150µl/well of the Luminex Sheath Fluid was transferred to the plate and the plate was shaken on a plate shaker for ≥ 5 minutes. The plate was read on the Luminex200 Plate reader plate.

### Example 3

This is a Phase 2b, randomized, double-blind, placebo-controlled, parallel-arm, multicenter study to evaluate the efficacy and safety of two SC treatment regimens of tralokinumab in adult subjects with uncontrolled, severe asthma requiring high-dose ICS and LABA with or without additional controller medications (high-dose ICS defined as a total daily dose > 500 µg fluticasone dry powder inhaler [DPI] or > 440 µg metered dose inhaler [MDI]; GINA, 2009; National Heart, Lung, and Blood Institute, 2007). Subjects will be randomized in a 1:1 ratio to one of 2 cohorts (Cohort 1 or Cohort 2). Within each cohort, subjects will be randomized in a 2:1 ratio to receive tralokinumab (300 mg) or placebo as follows:
Cohort 1: Tralokinumab 300 mg (n = 130) or Placebo (n = 65) as 2 SC injections every 2 weeks (Q2W) for 50 weeks for a total of 26 doses
Cohort 2: Tralokinumab 300 mg (n = 130) or Placebo (n = 65) as 2 SC injections Q2W for 12 weeks followed by every 4 weeks (Q4W) for 38 weeks for a total of 16 doses

Subjects will be stratified at screening by the number of asthma exacerbations in the past 12 months (2 versus > 2 but ≤ 6 exacerbations) and by chronic oral corticosteroid (OCS) use (presence versus absence).

A 5-week screening/run-in period (Week -5 to -1 [Day -1) will precede investigational product administration. Starting at Week -4 (Day -28), subjects will receive a fixed-dose combination product of fluticasone/salmeterol, either as an MDI (230 µg/21 µg) at a dose of 2 inhalations twice per day or as a DPI (500 µg/50 µg) at a dose of one inhalation twice per day. Sites/subjects will be permitted to use either presentation of fluticasone/salmeterol if approved and available for use in their country. If the subject is also taking additional asthma controller medications (including leukotriene modifiers, theophylline, cromones, or OCS ≤ 20 mg/day), then these medications should be continued at a stable dose during the screening/run-in period. During the study, subjects
may use inhaled reliever therapy (eg, short-acting β2 agonist or short-acting anticholinergic) on an as-required basis as documented in their Personalized Asthma Action Plan.

Subjects will continue to receive the same fixed-dose combination product of fluticasone/salmeterol with or without additional asthma controller medications at a stable dose during the treatment period and through Week 53. Subjects who experience an asthma exacerbation during the treatment period will be treated accordingly and will remain in the study. Subjects will return to the clinic at Week 53 for an assessment of the efficacy endpoints. Subjects will have 3 additional follow-up visits at Weeks 59, 67, and 75. After the Week 53 visit, background medications may be changed as deemed necessary by the investigator. As part of the study, selected sites will take part in a sub-study using HRCT scanning to measure potential airway wall structural changes including airway wall thickness. Sites will be selected on their ability and willingness to carry out the scans. Participation in this sub study is optional for subjects at the selected sites. An adequate number of sites will be identified in order to target recruitment of approximately 40 subjects into each of the 2 cohorts for the HRCT sub-study. Subjects will be in the study for 79 weeks, including a 5-week screening/run-in period (Week -5 to Week -1), a 48- or 50-week treatment period (Weeks 1-51 for Cohort 1 and Weeks 1-49 for Cohort 2), and a 24-week follow-up period (Weeks 51-75).

### Subject Population

The subjects in this study will be adults aged 18-75 years with uncontrolled, severe asthma requiring high-dose ICS and LABA with or without additional controller medications.

### Treatment Regimen

A total of 390 subjects will be randomized in a 1:1 ratio to one of 2 cohorts. Within each cohort, subjects will be randomized in a 2:1 ratio to receive SC tralokinumab (300 mg) or placebo. Investigational product (tralokinumab or placebo) will be administered as 2 SC injections of 1 mL either Q2W for 50 weeks for a total of 26 doses (Cohort 1) or Q2W for 12 weeks followed by Q4W for 38 weeks for a total of 16 doses (Cohort 2).

### References

Blease. Therapeutics targeting IL-13 for the treatment of pulmonary inflammation and airway remodelling. Current Opinion in Investigational Drugs. 2008, 9(11):1180-1184.
Bree A, Schlerman FJ, Wadanoli M, Tchistiakova L, Marquette K, Tan X-Y, Jacobson BA, Widom A, Cook TA, Wood N, Vunnum S, Krykbaev R, Xu X, Donaldson DD, Goldman SJ, Sypek J, Kasaian MT (2007) IL-13 blockade reduces lung inflammation after ascaris suum challenge in cynomolgus monkeys. J. Allergy Clin. Immunol. 119(5) 1251-7.
GINA Guidelines 2009: GINA report, Global strategy for asthma management and prevention. http://www.ginasthma.com/Guidelineitem.asp??l1=2&l2=1&intId=1561
Grunig G, Warnock M, Wakil AE, Venkayya R, Brombacher F, Rennick DM, Sheppard D, Mohrs M, Donaldson DD, Locksley RM, Corry DB. Requirement for IL-13 Independently of IL-4 in Experimental Asthma. Science 1998; 2261-3.
Heinzmann A, Mao XQ, Akaiwa M, Kreomer RT, Gao PS, Ohshima K, Umeshita R, Abe Y, Braun S, Yamashita T, Roberts MH, Sugimoto R, Arima K, Arinobu Y, Yu B, Kruse S, Enomoto T, Dake Y, Kawai M, Shimazu S, Sasaki S, Adra CN, Kitaichi M, Inoue H, Yamauchi K, Tomichi N, Kurimoto F, Hamasaki N, Hopkin JM, Izuhara K, Shirakawa T, Deichmann KA. Genetic variants of IL-13 signalling and human asthma and atopy. Hum Mol Genet. 2000; 9(4): 549-59.
Hershey GK. IL-13 receptors and signaling pathways: an evolving web. J Allergy Clin Immunol, 2003. 111(4): p. 677-90.
Howard TD, Whittaker PA, Zaiman AL, Koppelman GH, Xu J, Hanley MT, Meyers DA, Postma DS, Bleecker ER. Identification and association of polymorphisms in the interleukin-13 gene with asthma and atopy in a Dutch population. Am J Respir Cell Mol Biol. 2001; 25(3): 377-84.
Howard TD, Koppelman GH, Xu J, Zheng SL, Postma DS, Meyers DA, Bleecker ER. Gene-gene interaction in asthma: IL4RA and IL13 in a Dutch population with asthma. Am J Hum Genet. 2002; 70(1): 230-6.
Huang S-K, Xiao H-Q, Kleine-Tebbe J, Paciotti Gi, Marsh DG., Lichtenstein LM, and Liu MC. J Immunol. 1995; 155(5): 2688-2694
Humbert M, Durham SR, Kimmitt P, Powell N, Assoufi B, Pfister R, Menz G, Kay AB, Corrigan CJ. Elevated expression of messenger ribonucleic acid encoding IL-13 in the bronchial mucosa of atopic and nonatopic subjects with asthma. J Allergy Clin Immunol. 1997; 99(5): 657-65.
Juniper EF, O'Byrne PM, Guyatt GH, Ferrie PJ, King DR. Development and validation of a questionnaire to measure asthma control. Eur Respir J. 1999; 14: 902-7.
Juniper EF, Bousquet J, Abetz L, Bateman ED. Identifying 'well-controlled' and 'not well-controlled' asthma using the Asthma Control Questionnaire. Respir Med. 2006; 100: 616-21.
Kauppi P, Lindblad-Toh K, Sevon P, Toivonen HT, Rioux JD, Villapakkam A, Laitinen LA, Hudson TJ, Kere J, Laitinen T. A second-generation association study of the 5q31 cytokine gene cluster and the interleukin-4 receptor in asthma. Genomics. 2001 Sep;77(1-2):35-42.
Komai-Koma M, McKay A, Thomson L, McSharry C, Chalmers GW, Liew FY, Thomson NC. Immuno-regulatory cytokines in asthma: IL-15 and IL-13 in induced sputum. Clin Exp Allergy. 2001; 31(9): 1441-8.
Kotsimbos TC, Ernst P, Hamid QA. Interleukin-13 and interleukin-4 are coexpressed in atopic asthma. Proc Assoc Am Physicians. 1996; 108(5): 368-73.
Kroegel, Julius, Matthys, Virchow, Luttmann. Endobronchial secretion of interleukin-13 following local allergen challenge in atopic asthma: relationship to interleukin-4 and eosinophil counts. Eur Respir J. 1996; 9: 899.
Liu X, Nickel R, Beyer K, Wahn U, Ehrlich E, Freidhoff LR, Björkstén B, Beaty TH, Huang SK. An IL13 coding region variant is associated with a high total serum IgE level and atopic dermatitis in the German multicenter atopy study (MAS-90). J Allergy Clin Immunol. 2000; 106(1 Pt 1): 167-70.
Naseer T, Minshall EM, Leung DY, Laberge S, Ernst P, Martin RJ, Hamid Q. Expression of IL-12 and IL-13 mRNA in asthma and their modulation in response to steroid therapy. Am J Respir Crit Care Med. 1997; 155(3): 845-51.
Reddel HK, Taylor DR, Bateman ED, et al.. An official American Thoracic Society/European Respiratory Society statement: asthma control and exacerbations: standardizing endpoints for clinical asthma trials and clinical practice. Am J Respir Crit Care Med 2009; 180: 59-99.
Saha SK, Berry MA, Parker D, Siddiqui S, Morgan A, May R, Monk P, Bradding P, Wardlaw AJ, Pavord ID, Brightling CE. Increased sputum and bronchial biopsy IL-13 expression in severe asthma. J Allergy Clin Immunol. 2008 Mar;121(3):685-91.
Singh et al. A phase 1 study evaluating the pharmacokinetics, safety and tolerability of repeat dosing with a human IL-13 antibody (CAT-354) in subjects with asthma. BMC Pulmonary Medicine. 2010, 10(1):3.
Tamura K, Suzuki M, Arakawa H, Tokuyama K, Morikawa A. Linkage and association studies of STAT6 gene polymorphisms and allergic diseases. Int Arch Allergy Immunol. 2003 May;131(1):33-8.
van der Pouw Kraan TC, van Veen A, Boeije LC, van Tuyl SA, de Groot ER, Stapel SO, Bakker A, Verweij CL, Aarden LA, van der Zee JS. An IL-13 promoter polymorphism associated with increased risk of allergic asthma. Genes Immun. 1999; 1(1): 61-5.
Venkayya R, Lam M, Willkom M, Grünig G, Corry DB, Erle DJ. The Th2 lymphocyte products IL-4 and IL-13 rapidly induce airway hyperresponsiveness through direct effects on resident airway cells. Am J Respir Cell Mol Biol. 2002; 26(2): 202-8.
Vladich FD, Brazille SM, Stern D, Peck ML, Ghittoni R, Vercelli D. IL-13 R130Q, a common variant associated with allergy and asthma, enhances effector mechanisms essential for human allergic inflammation. J Clin Invest. 2005; 115(3): 747-54.
Wills-Karp M, Luyimbazi J, Xu X, Schofield B, Neben TY, Karp CL, Donaldson D. Interleukin-13: Central mediator of allergic asthma Science 1998; 282 : 2258-2261.
Zhu Z, Homer RJ, Wang Z, Chen Q, Geba GP, Wang J, et al.. Pulmonary expression of interleukin-13 causes inflammation, mucus hypersecretion, subepithelial fibrosis, physiologic abnormalities, and eotaxin production. J Clin Invest 1999; 103: 779-88.

### Sequence Listing Information

### Tralokinumab (BAK502G9, CAT-354):

SEQ ID NO: 7 is HCDR1;
SEQ ID NO: 8 is HCDR2;
SEQ ID NO: 9 is HCDR3;
SEQ ID NO: 10 is LCDR1;
SEQ ID NO: 11 is LCDR2; and
SEQ ID NO: 12 is LCDR3.
SEQ ID NO: 15 is VH fragment
SEQ ID NO: 16 is VL fragment

### Variants of Tralokinumab (HCDR1, 2, 3, LCDR1, 2, 3)

BAK278D6 (SEQ ID NOS: 1, 2, 3, 4, 5, 6)
BAK1183H4 (SEQ ID NOS: 97, 98, 99, 100, 101, 102),
BAK1167F02 (SEQ ID NOS: 64, 65, 66, 67, 68, 69),
BAKllllD10 (SEQ ID NOS: 91, 92, 93, 94, 95, 96),
BAK1166G02 (SEQ ID NOS: 67, 68, 69, 70, 71, 72),
BAK1167F04 (SEQ ID NOS: 85, 86, 87, 88, 89, 90),
BAK1184C8 (SEQ ID NOS: 73, 74, 75, 76, 77, 78),
BAK1185E1 (SEQ ID NOS: 79, 80, 81, 82, 83, 84), and
BAK1185F8(SEQ ID NOS: 103, 104, 105, 106, 107, 108).

### Variants of tralokinumab (VH, VL)

BAK278D6 (VH SEQ ID NO: 13, VL SEQ ID NO; 14)
BAK1183H4 (VH SEQ ID NO: 37, VL SEQ ID NO: 38),
BAK1167F02 (VH SEQ ID NO: 35, VL SEQ ID NO: 36),
BAKllllD10 (VH SEQ ID NO: 41, VL SEQ ID NO: 42),
BAK1166G02 (VH SEQ ID NO: 53, VL SEQ ID NO: 54),
BAK1167F04 (VH SEQ ID NO: 43, VL SEQ ID NO: 44),
BAK1184C8 (VH SEQ ID NO: 45, VL SEQ ID NO: 46),
BAK1185E1 (VH SEQ ID NO: 47, VL SEQ ID NO: 48),
BAK1185F8 (VH SEQ ID NO: 49, VL SEQ ID NO: 50).

### BAK278D6

### HEAVY CHAIN

CDR1- SEQ ID NO 1: NYGLS
CDR2- SEQ ID NO 2: WISANNGDTNYGQEFQG
CDR3- SEQ ID NO 3: DSSSNWARWFFDL

### BAK278D6

### LIGHT CHAIN

CDR1- SEQ ID NO 4: GGNNIGSKLVH
CDR2- SEQ ID NO 5: DDGDRPS
CDR3- SEQ ID NO 6: QVWDTGSDPVV

### BAK502G9

### HEAVY CHAIN

CDR1-SEQ ID NO 7: NYGLS
CDR2-SEQ ID NO 8: WISANNGDTNYGQEFQG
CDR3-SEQ ID NO 9: DSSSSWARWFFDL

### LIGHT CHAIN

CDR1-SEQ ID NO 10: GGNIIGSKLVH
CDR2-SEQ ID NO 11: DDGDRPS
CDR3-SEQ ID NO 12: QVWDTGSDPVV

### BAK278D6

### HEAVY CHAIN DOMAIN

SEQ ID NO 13:

EVQLVQSGAEVKKPGASVKVSCKASGYTFRNYGLSWVRQAPGQGLEWM
GWISANNGDTNYGQEFQGRITMTTETSTNTAHMELRSLRSDDTAVYYCV
RDSSSNWARWFFDLWGKGTMVTVSS

### BAK278D6

### LIGHT CHAIN DOMAIN

SEQ ID NO 14:

SYVLTQPPSVSVAPGQTARIPCGGNNIGSKLVHWYQQKPGQAPVLVVYD
DGDRPSGIPERFSGSNSGNTATLTISRIDAGDEADYYCQVWDTGSDPVVF
GGGTKLTVL

### BAK502G9

### HEAVY CHAIN DOMAIN

SEQ ID NO 15:

QVQLVQSGAEVKKPGASVKVSCKASGYTFTNYGLSWVRQAPGQGLEWM
GWISANNGDTNYGQEFQGRVTMTTDTSTSTAYMELRSLRSDDTAVYYCA
RDSSSSWARWFFDLWGRGTLVTVSS

### BAK502G9

### LIGHT CHAIN DOMAIN

SEQ ID NO 16:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK278D6

### HEAVY CHAIN

FR1- SEQ ID NO 17: EVQLVQSGAEVKKPGASVKVSCKASGYTFR
FR2- SEQ ID NO 18: WVRQAPGQGLEWMG
FR3- SEQ ID NO 19: RITMTTETSTNTAHMELRSLRSDDTAVYYCVR

### BAK278D6

### LIGHT CHAIN

FR1- SEQ ID NO 20: SYVLTQPPSVSVAPGQTARIPC
FR2- SEQ ID NO 21: WYQQKPGQAPVLVVY
FR3- SEQ ID NO 22: GIPERFSGSNSGNTATLTISRIDAGDEADYYC

### BAK167A11

### HEAVY CHAIN DOMAIN

SEQ ID NO 23:

EVQLLESGGGLVQPGGSLRLSCAASGFTFSSYAMSWVRQAPGKGLEWV
SAISGSGGSTYYADSVKGRFTISRDNSKNTLYLQMNSLRAEDTAVYYCAR
VGAAGEGYYGYWGRGTLVTVSS

### BAK167A11

### LIGHT CHAIN DOMAIN

SEQ ID NO 24:

NFMLTQPHSVSESPGKTVTISCTRSSGSIASNYVQWYQQRPGSAPTTVIY
DDNQRPSGVPDRFSGSIDSSSNSASLTISGLKTEDEADYYCQSYDSNND
VFGGGTKVTVL

### BAK209B11

### HEAVY CHAIN DOMAIN

SEQ ID NO 25:

QVQLQESGGGGLVQPGGSLRLSCAASGFTFSSYGMSWVRQAPGKGLE
WVSSISASGDSTFYADSVKGRFTISRDNNKNMVFLQVNSLRADDTAVYF
CAKDWSQWLVGDAFDVWGRGTTVTVSS

### BAK209B11

### LIGHT CHAIN DOMAIN

SEQ ID NO 26:

DIQLTQSPSTLSASVGDRVTITCRASQSVSLWVAWYQQRPGKAPKLLIYD
GSTLQSGVPARFSGSGSGTEFTLTISSLQPDDFATYYCQQYKTFSTFGQG
TKVEIKRA

### BAK502G9

### HEAVY CHAIN

FR1- SEQ ID NO 27: QVQLVQSGAEVKKPGASVKVSCKASGYTFT
FR2- SEQ ID NO 28: WYRQAPGQGLEWMG
FR3- SEQ ID NO 29: RVTMTTDTSTSTAYMELRSLRSDDTAVYYCAR

### BAK502G9

### LIGHT CHAIN

FR1- SEQ ID NO 30: SYVLTQPPSVSVAPGKTARITC
FR2- SEQ ID NO 31: WYQQKPGQAPVLVIY
FR3- SEQ ID NO 32: GIPERFSGSNSGNTATLTISRVEAGDEADYYC

### BAK615E3

### HEAVY CHAIN DOMAIN

SEQ ID NO 33:

EVQLLESGGGLVQPGGSLRLSCAASGFTFSSYAMSWVRQAPGKGLEWV
SAISGSGGSTYYADSVKGRFTISRDNSKNTLYLQMNSLRAEDTAVYYCAR
VGKATTEEGYYGYWGRGTLVTVSS

### BAK615E3

### LIGHT CHAIN DOMAIN

SEQ ID NO 34:

NFMLTQPHSVSESPGKTVTISCTRSSGSIASNYVQWYQQRPGSAPTTVIY
DDNQRPSGVPDRFSGSIDSSSNSASLTISGLKTEDEADYYCQSYDSNND
VFGGGTKVTVL

### BAK1167F2

### HEAVY CHAIN DOMAIN

SEQ ID NO 35:

QVQLVQSGAEVKKPGASVKVSCKASGYTFEQTGVSWVRQAPGQGLEW
MGWISANNGDTNYGQEFQGRVTMTTDTSTSTAYMELRSLRSDDTAVYY
CARDSSSSWARWFFDLWGRGTLVTVSS

### BAK1167F2

### LIGHT CHAIN DOMAIN

SEQ ID NO 36:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1183H4

### HEAVY CHAIN DOMAIN

SEQ ID NO 37:

QVQLVQSGAEVKKPGASVKVSCKASGYTFTNYGLSWVRQAPGQGLEWM
GWINYDGGNTQYGQEFQGRVTMTTDTSTSTAYMELRSLRSDDTAVYYC
ARDSSSSWARWFFDLWGRGTLVTVSS

### BAK1183H4

### LIGHT CHAIN DOMAIN

SEQ ID NO 38:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1105H3

### HEAVY CHAIN DOMAIN

SEQ ID NO 39:

QVQLVQSGAEVKKPGASVKVSCKASGYTFTNYGLSWVRQAPGQGLEWM
GWISGLNGETLYGQEFQGRVTMTTDTSTSTAYMELRSLRSDDTAVYYCA
RDSSSSWARWFFDLWGRGTLVTVSS

### BAK1105H3

### LIGHT CHAIN DOMAIN

SEQ ID NO 40:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1111D10

### HEAVY CHAIN DOMAIN

SEQ ID NO 41:

QVQLVQSGAEVKKPGASVKVSCKASGYTFTNYGLSWVRQAPGQGLEWM
GWIATPDGQTSYGQEFQGRVTMTTDTSTSTAYMELRSLRSDDTAVYYCA
RDSNSSWARWFFDLWGRGTLVTVSS

### BAK1111D10

### LIGHT CHAIN DOMAIN

SEQ ID NO 42:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1167F4

### HEAVY CHAIN DOMAIN

SEQ ID NO 43:

QVQLVQSGAEVKKPGASVKVSCKASGYTFIDTGVSWVRQAPGQGLEWM
GWISANNGDTNYGQEFQGRVTMTTDTSTSTAYMELRSLRSDDTAVYYCA
RDSSSSWARWFFDLWGRGTLVTVSS

### BAK1167F4

### LIGHT CHAIN DOMAIN

SEQ ID NO 44:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1184C8

### HEAVY CHAIN DOMAIN

SEQ ID NO 45:

QVQLVQSGAEVKKPGASVKVSCKASGYTFTNYGLSWVRQAPGQGLEWM
GWISGSNGYTSYGQEFQGRVTMTTDTSTSTAYMELRSLRSDDTAVYYCA
RDSSSSWARWFFDLWGRGTLVTVSS

### BAK1184C8

### LIGHT CHAIN DOMAIN

SEQ ID NO 46:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1185E1

### HEAVY CHAIN DOMAIN

SEQ ID NO 47:

QVQLVQSGAEVKKPGASVKVSCKASGYTFTNYGLSWVRQAPGQGLEWM
GWINDATGDTQYGQEFQGRVTMTTDTSTSTAYMELRSLRSDDTAVYYCA
RDSSSSWARWFFDLWGRGTLVTVSS

### BAK1185E1

### LIGHT CHAIN DOMAIN

SEQ ID NO 48:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1185F8

### HEAVY CHAIN DOMAIN

SEQ ID NO 49:

QVQLVQSGAEVKKPGASVKVSCKASGYTFTDYGLSWVRQAPGQGLEW
MGWIRNIDGYTIYGQEFQGRVTMTTDTSTSTAYMELRSLRSDDTAVYYCA
RDSSSSWARWFFDLWGRGTLVTVSS

### BAK1185F8

### LIGHT CHAIN DOMAIN

SEQ ID NO 50:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1187B4

### HEAVY CHAIN DOMAIN

SEQ ID NO 51:

QVQLVQSGAEVKKPGASVKVSCKASGYTFTNYGLSWVRQAPGQGLEWM
GWIDDDSGTTIYGQEFQGRVTMTTDTSTSTAYMELRSLRSDDTAVYYCA
RDSSSSWARWFFDLWGRGTLVTVSS

### BAK1187B4

### LIGHT CHAIN DOMAIN

SEQ ID NO 52:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK1166G2

### HEAVY CHAIN DOMAIN

SEQ ID NO 53:

QVQLVQSGAEVKKPGASVKVSCKASGYTFANTGISWVRQAPGQGLEWM
GWISANNGDTNYGQEFQGRVTMTTDTSTSTAYMELRSLRSDDTAVYYCA
RDSSSSWARWFFDLWGRGTLVTVSS

### BAK1166G2

### LIGHT CHAIN DOMAIN

SEQ ID NO 54:

SYVLTQPPSVSVAPGKTARITCGGNIIGSKLVHWYQQKPGQAPVLVIYDD
GDRPSGIPERFSGSNSGNTATLTISRVEAGDEADYYCQVWDTGSDPVVF
GGGTKLTVL

### BAK167A11

### HEAVY CHAIN

CDR1- SEQ ID NO 55: SYAMS
CDR2- SEQ ID NO 56: AISGSGGSTYYADSVKG
CDR3- SEQ ID NO 57: VGAAGEGYYGY

### BAK167A11

### LIGHT CHAIN

CDR1- SEQ ID NO 58: TRSSGSIASNYVQ
CDR2- SEQ ID NO 59: DDNQRPS
CDR3- SEQ ID NO 60: QSYDSNNDV

### BAK1167F2

### HEAVY CHAIN

CDR1- SEQ ID NO 61: QTGVS
CDR2- SEQ ID NO 62: WISANNGDTNYGQEFQG
CDR3- SEQ ID NO 63: DSSSSWARWFFDL

### BAK1167F2

### LIGHT CHAIN

CDR1- SEQ ID NO 64: GGNIIGSKLVH
CDR2- SEQ ID NO 65: DDGDRPS
CDR3- SEQ ID NO 66: QVWDTGSDPVV

### BAK1166G2

### HEAVY CHAIN

CDR1- SEQ ID NO 67: NTGIS
CDR2- SEQ ID NO 68: WISANNGDTNYGQEFQG
CDR3- SEQ ID NO 69: DSSSSWARWFFDL

### BAK1166G2

### LIGHT CHAIN

CDR1- SEQ ID NO 70: GGNIIGSKLVH
CDR2- SEQ ID NO 71: DDGDRPS
CDR3- SEQ ID NO 72: QVWDTGSDPVV

### BAK1184C8

### HEAVY CHAIN

CDR1- SEQ ID NO 73: NYGLS
CDR2- SEQ ID NO 74: WISGSNGYTSYGQEFQG
CDR3- SEQ ID NO 75: DSSSSWARWFFDL

### BAK1184C8

### LIGHT CHAIN

CDR1- SEQ ID NO 76: GGNIIGSKLVH
CDR2- SEQ ID NO 77: DDGDRPS
CDR3- SEQ ID NO 78: QVWDTGSDPVV

### BAK1185E1

### HEAVY CHAIN

CDR1- SEQ ID NO 79: NYGLS
CDR2- SEQ ID NO 80: WINDATGDTQYGQEFQG
CDR3- SEQ ID NO 81: DSSSSWARWFFDL

### BAK1185E1

### LIGHT CHAIN

CDR1- SEQ ID NO 82: GGNIIGSKLVH
CDR2- SEQ ID NO 83: DDGDRPS
CDR3- SEQ ID NO 84: QVWDTGSDPVV

### BAK1167F4

### HEAVY CHAIN

CDR1- SEQ ID NO 85: DTGVS
CDR2- SEQ ID NO 86: WISANNGDTNYGQEFQG
CDR3- SEQ ID NO 87: DSSSSWARWFFDL

### BAK1167F4

### LIGHT CHAIN

CDR1- SEQ ID NO 88: GGNIIGSKLVH
CDR2- SEQ ID NO 89: DDGDRPS
CDR3- SEQ ID NO 90: QVWDTGSDPVV

### BAK1111D10

### HEAVY CHAIN

CDR1- SEQ ID NO 91: NYGLS
CDR2- SEQ ID NO 92: WIATPDGQTSYGQEFQG
CDR3- SEQ ID NO 93: DSNSSWARWFFDL

### BAK1111D10

### LIGHT CHAIN

CDR1- SEQ ID NO 94: GGNIIGSKLVH
CDR2- SEQ ID NO 95: DDGDRPS
CDR3- SEQ ID NO 96: QVWDTGSDPVV

### BAK1183H4

### HEAVY CHAIN

CDR1- SEQ ID NO 97: NYGLS
CDR2- SEQ ID NO 98: WINYDGGNTQYGQEFQG
CDR3- SEQ ID NO 99: DSSSSWARWFFDL

### BAK1183H4

### LIGHT CHAIN

CDR1- SEQ ID NO 100: GGNIIGSKLVH
CDR2- SEQ ID NO 101: DDGDRPS
CDR3- SEQ ID NO 102: QVWDTGSDPVV

### BAK1185H8

### HEAVY CHAIN

CDR1- SEQ ID NO 103: DYGLS
CDR2- SEQ ID NO 104: WRINDGYTIYGQEFQG
CDR3- SEQ ID NO 105: DSSSSWARWFFDL

### BAK1185H8

### LIGHT CHAIN

CDR1- SEQ ID NO 106: GGNIIGSKLVH
CDR2- SEQ ID NO 107: DDGDRPS
CDR3- SEQ ID NO 108: QVWDTGSDPVV

### BAK278D6

HEAVY CHAIN- SEQ ID NO: 109

CAGGTGCAGCTGGTGCAGTCTGGGGCTGAGGTGAAGAAGCCTGGGG
CCTCAGTGAAGGTCTCCTGCAAGGCTTCTGGTTACACCTTTACAAATTA
TGGTCTCAGCTGGGTGCGACAGGCCCCTGGACAAGGGCTTGAGTGGA
TGGGATGGATCAGCGCTAATAATGGCGACACAAATTATGGACAGGAAT
TCCAGGGCAGAGTCACCATGACCACAGATACATCCACGAGCACAGCCT
ACATGGAGTTGAGGAGCCTGAGATCTGACGACACGGCCGTTTATTACT
GTGCGAGAGACTCCAGCAGCAACTGGGCCCGCTGGTTTTCGATCTCT
GGGGCCGGGGGACACTGGTCACCGTCTCCTCA

### BAK278D6

LIGHT CHAIN- SEQ ID NO:110

TCCTATGTGCTGACTCAGCCACCCTCGGTGTCAGTGGCCCCAGGTAAG
ACGGCCAGGATTACCTGTGGGGGAAACAACATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK502G9

HEAVY CHAIN- SEQ ID NO: 111

CAGGTCCAGCTGGTGCAGTCTGGGGCTGAGGTGAAGAAGCCTGGGGC
CTCAGTGAAGGTCTCCTGCAAGGCTTCTGGTTACACCTTTACAAATTAT

GGTCTCAGCTGGGTGCGACAGGCCCCTGGACAAGGGCTTGAGTGGAT
GGGATGGATCAGCGCTAATAATGGCGACACAAATTATGGACAGGAATT
CCAGGGCAGAGTCACCATGACCACAGATACATCCACGAGCACAGCCTA
CATGGAGTTGAGGAGCCTGAGATCTGACGACACGGCCGTTTATTACTG
TGCGAGAGACTCCAGCAGCAGCTGGGCCCGCTGGTTTTCGATCTCTG
GGGCCGGGGGACACTGGTCACCGTCTCCTCA

### BAK502G9

LIGHT CHAIN- SEQ ID NO:112

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK1105H03

HEAVY CHAIN- SEQ ID NO: 113

CAGGTCCAGCTGGTGCAGTCTGGGGCTGAGGTGAAGAAGCCTGGGGC
CTCAGTGAAGGTCTCCTGCAAGGCTTCTGGTTACACCTTTACAAATTAT
GGTCTCAGCTGGGTGCGACAGGCCCCTGGACAAGGGCTTGAGTGGAT
GGGATGGATCTCCGGCTTGAACGGCGAGACATTGTATGGACAGGAATT
CCAGGGCAGAGTCACCATGACCACAGATACATCCACGAGCACAGCCTA
CATGGAGTTGAGGAGCCTGAGATCTGACGACACGGCCGTTTATTACTG
TGCGAGAGACTCCAGCAGCAGCTGGGCCCGCTGGTTTTTCGATCTCTG
GGGCCGGGGGACACTGGTCACCGTCTCCTCA

### BAK1105H03

LIGHT CHAIN- SEQ ID NO: 114

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK1111D10

HEAVY CHAIN- SEQ ID NO:115

CAGGTGCAGCTGGTGCAGTCTGGGGCTGAGGTGAAGAAGCCTGGGG
CCTCAGTGAAGGTCTCCTGCAAGGCTTCTGGTTACACCTTTACAAATTA
TGGTCTCAGCTGGGTGCGACAGGCCCCTGGACAAGGGCTTGAGTGGA
TGGGATGGATCGCAACCCCAGACGGCCAGACAAGCTATGGACAGGAA
TTCCAGGGCAGAGTCACCATGACCACAGATACATCCACGAGCACAGCC
TACATGGAGTTGAGGAGCCTGAGATCTGACGACACGGCCGTTTATTAC
TGTGCGAGAGACTCCAACAGCAGCTGGGCCCGCTGGTTTTTCGATCTC
TGGGGCCGGGGGACACTGGTCACCGTCTCCTCA

### BAK1111D10

LIGHT CHAIN- SEQ ID NO:116

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK 1167F2

HEAVY CHAIN- SEQ ID NO: 117

CAAGTGCAGCTGGTGCAGTCTGGGGCTGAGGTGAAGAAGCCTGGGGC
CTCAGTGAAGGTCTCCTGCAAGGCTTCTGGTTACACCTTTGAGCAGAC
CGGCGTCTCCTGGGTGCGACAGGCCCCTGGACAAGGGCTTGAGTGGA
TGGGATGGATCAGCGCTAATAATGGCGACACAAATTATGGACAGGAAT
TCCAGGGCAGAGTCACCATGACCACAGATACATCCACGAGCACAGCCT
ACATGGAGTTGAGGAGCCTGAGATCTGACGACACGGCCGTTTATTACT
GTGCGAGAGACTCCAGCAGCAGCTGGGCCCGCTGGTTTTTCGATCTCT
GGGGCCGGGGGACACTGGTCACCGTCTCCTCA

### BAK 1167F2

LIGHT CHAIN- SEQ ID NO:118

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK 1167F04

HEAVY CHAIN- SEQ ID NO:119

CAGGTGCAGCTGGTGCAGTCTGGGGCTGAGGTGAAGAAGCCTGGGG
CCTCAGTGAAGGTCTCCTGCAAGGCTTCTGGTTACACCTTTATCGACAC
CGGGGTCTCCTGGGTGCGACAGGCCCCTGGACAAGGGCTTGAGTGGA
TGGGATGGATCAGCGCTAATAATGGCGACACAAATTATGGACAGGAAT
TCCAGGGCAGAGTCACCATGACCACAGATACATCCACGAGCACAGCCT
ACATGGAGTTGAGGAGCCTGAGATCTGACGACACGGCCGTTTATTACT
GTGCGAGAGACTCCAGCAGCAGCTGGGCCCGCTGGTTTTCGATCTCT
GGGGCCGGGGGACACTGGTCACCGTCTCCTCA

### BAK 1167F04

LIGHT CHAIN- SEQ ID NO:120

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC

CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK 1183H4

HEAVY CHAIN- SEQ ID NO:121

CAGGTGCAGCTGGTGCAGTCTGGGGCTGAGGTGAAGAAGCCTGGGG
CCTCAGTGAAGGTCTCCTGCAAGGCTTCTGGTTACACCTTTACAAATTA
TGGTCTCAGCTGGGTGCGACAGGCCCCTGGACAAGGGCTTGAGTGGA
TGGGATGGATCAACTACGACGGCGGCAACACACAGTATGGACAGGAAT
TCCAGGGCAGAGTCACCATGACCACAGATACATCCACGAGCACAGCCT
ACATGGAGTTGAGGAGCCTGAGATCTGACGACACGGCCGTTTATTACT
GTGCGAGAGACTCCAGCAGCAGCTGGGCCCGCTGGTTTTCGATCTCT
GGGGCCGGGGGACACTGGTCACCGTCTCCTCA

### BAK 1183H4

LIGHT CHAIN- SEQ ID NO: 122

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK1184C8

HEAVY CHAIN- SEQ ID NO:123

CAGGTGCAGCTGGTGCAGTCTGGGGCTGAGGTGAAGAAGCCTGGGG
CCTCAGTGAAGGTCTCCTGCAAGGCTTCTGGTTACACCTTTACAAATTA
TGGTCTCAGCTGGGTGCGACAGGCCCCTGGACAAGGGCTTGAGTGGA
TGGGATGGATCAGCGGGAGCAACGGCTACACATCTTATGGACAGGAAT
TCCAGGGCAGAGTCACCATGACCACAGATACGTCCACGAGCACAGCCT
ACATGGAGTTGAGGAGCCTGAGATCTGACGACACGGCCGTTTATTACT
GTGCGAGAGACTCCAGCAGCAGCTGGGCCCGCTGGTTTTTCGATCTCT
GGGGCCGGGGGACACTGGTCACCGTCTCCTCA

### BAK1184C8

LIGHT CHAIN- SEQ ID NO: 124

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK1185E1

HEAVY CHAIN- SEQ ID NO:125

CAGGTGCAGCTGGTGCAGTCTGGGGCTGAGGTGAAGAAGCCTGGGG
CCTCAGTGAAGGTCTCCTGCAAGGCTTCTGGTTACACCTTTACAAATTA
TGGTCTCAGCTGGGTGCGACAGGCCCCTGGACAAGGGCTTGAGTGGA
TGGGATGGATCAACGACGCCACCGGCGACACACAGTATGGACAGGAA
TTCCAGGGCAGAGTCACCATGACCACAGATACATCCACGAGCACAGCC
TACATGGAGTTGAGGAGCCTGAGATCTGACGACACGGCCGTTTATTAC
TGTGCGAGAGACTCCAGCAGCAGCTGGGCCCGCTGGTTTTTCGATCTC
TGGGGCCGGGGGACACTGGTCACCGTCTCCTCA

### BAK1185E1

LIGHT CHAIN- SEQ ID NO: 126

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK1185F8

HEAVY CHAIN- SEQ ID NO:127

CAGGTGCAGCTGGTGCAGTCTGGGGCTGAGGTGAAGAAGCCTGGGG
CCTCAGTGAAGGTCTCCTGCAAGGCTTCTGGTTACACCTTTACAGATTA
TGGTCTCAGCTGGGTGCGACAGGCCCCTGGACAAGGGCTAGAGTGGA
TGGGATGGATCCGCAACATCGACGGCTACACAATTTATGGACAGGAAT
TCCAGGGCAGAGTCACCATGACCACAGATACATCCACGAGCACAGCCT
ACATGGAGTTGAGGAGCCTGAGATCTGACGACACGGCCGTTTATTACT
GTGCGAGAGACTCCAGCAGCAGCTGGGCCCGCTGGTTTTCGATCTCT
GGGGCCGGGGGACACTGGTCACCGTCTCCTCA

### BAK1185F8

LIGHT CHAIN- SEQ ID NO: 128

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK1187B4

HEAVY CHAIN- SEQ ID NO:129

CAGGTCCAGCTGGTGCAGTCTGGGGCTGAGGTGAAGAAGCCTGGGGC
CTCAGTGAAGGTCTCCTGCAAGGCTTCTGGTTACACCTTTACAAATTAT
GGTCTCAGCTGGGTGCGACAGGCCCCTGGACAAGGGCTTGAGTGGAT
GGGATGGATCGACGACGACAGCGGCACGACAATATATGGACAGGAAT
TCCAGGGCAGAGTCACCATGACCACAGATACATCCACGAGCACAGCCT
ACATGGAGTTGAGGAGCCTGAGATCTGACGACACGGCCGTTTATTACT

GTGCGAGAGACTCCAGCAGCAGCTGGGCCCGCTGGTTTTCGATCTCT
GGGGCCGGGGGACACTGGTCACCGTCTCCTCA

### BAK1187B4

LIGHT CHAIN- SEQ ID NO:130

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK1166G02

HEAVY CHAIN- SEQ ID NO: 131

CAAGTGCAGTTGGTGCAGTCTGGGGCTGAGGTGAAGAAGCCTGGGGC
CTCAGTGAAGGTCTCCTGCAAGGCTTCTGGTTACACCTTTGCGAACAC
CGGGATCTCGTGGGTGCGACAGGCCCCTGGACAAGGGCTTGAGTGGA
TGGGATGGATCAGCGCTAATAATGGCGACACAAATTATGGACAGGAAT
TCCAGGGCAGAGTCACCATGACCACAGATACATCCACGAGCACAGCCT
ACATGGAGTTGAGGAGCCTGAGATCTGACGACACGGCCGTTTATTACT
GTGCGAGAGACTCCAGCAGCAGCTGGGCCCGCTGGTTTTCGATCTCT
GGGGTCGGGGGACACTGGTCACCGTCTCCTCA

### BAK1166G02

LIGHT CHAIN- SEQ ID NO:132

TCCTATGTGCTGACTCAGCCACCCTCGGTGTCAGTGGCCCCAGGAAAG
ACGGCCAGGATTACCTGTGGGGGAAACATCATTGGAAGTAAACTTGTA
CACTGGTACCAGCAGAAGCCAGGCCAGGCCCCTGTGCTGGTCATCTAT
GATGATGGCGACCGGCCCTCAGGGATCCCTGAGCGATTCTCTGGCTC
CAACTCTGGGAACACGGCCACCCTGACCATCAGCAGGGTCGAGGCCG
GGGATGAGGCCGACTATTATTGTCAGGTGTGGGATACTGGTAGTGATC
CCGTGGTATTCGGCGGAGGGACCAAGCTGACCGTCCTAGGT

### BAK165E7

HEAVY CHAIN- SEQ ID NO: 133

EVQLVQSGAEVKKPGASVKVSCKASGYTFTNYGLSWVRQAPGQGLEWM
GWISANNGETNYGQEFQGRVTMTTETPTNTAHMELRSLTSDDTAVYYCV
RDSSSNWARWYFDLWGQGTLVTVSS

### BAK165E7

LIGHT CHAIN- SEQ ID NO: 134

SYVLTQPPSVSVAPGQTARIPCGGNNIGSKLVHWYQQKPGQAPVLVVYD
DGDRPSGIPERFSGSNSGNTATLTISRIDAGDEADYYCQVWDTGSDPVVF
GGGTKLTVLG

### BAK165E7

### HEAVY CHAIN

CDR1- SEQ ID NO: 135 NYGLS
CDR2- SEQ ID NO: 136 WISANNGETNYGQEFQG
CDR3- SEQ ID NO: 137 DSSSNWARWYFDL

### BAK165E7

### LIGHT CHAIN

CDR1- SEQ ID NO: 138 GGNNIGSKLVH
CDR2- SEQ ID NO: 139 DDGDRPS
CDR3- SEQ ID NO:140 QVWDTGSDPVV

### BAK582 F7

### HEAVY CHAIN

CDR1- SEQ ID NO 141: SYAMS
CDR2- SEQ ID NO 142: AISGSGGSTYYADSVKG
CDR3- SEQ ID NO 143: VGAAGEGYYGY

### BAK582 F7

### LIGHT CHAIN

CDR1-SEQ ID NO 144: TRSSGSIASNYVE
CDR2-SEQ ID NO 145: DDNQRPS
CDR3-SEQ ID NO 146: QSYDSNNDV

### BAK612B5

### HEAVY CHAIN

CDR1- SEQ ID NO 147: SYAMS
CDR2- SEQ ID NO 148: AISGSGGSTYYADSVKG
CDR3- SEQ ID NO 149: VGRATTDEGYYGY

### BAK612B5

### LIGHT CHAIN

CDR1- SEQ ID NO 150: TRSSGSIASNYVQ
CDR2- SEQ ID NO 151: DDNQRPS
CDR3- SEQ ID NO 152: QSYDSNNDV

### BAK615E3

### HEAVY CHAIN

CDR1- SEQ ID NO 153: SYAMS
CDR2- SEQ ID NO 154: AISGSGGSTYYADSVKG
CDR3- SEQ ID NO 155: VGKATTEEGYY

### BAK615E3

### LIGHT CHAIN

CDR1- SEQ ID NO 156: TRSSGSIASNYVQ
CDR2- SEQ ID NO 157: DDNQRPS
CDR3- SEQ ID NO 158: QSYDSNNDV

### BAK0278D6

### HEAVY CHAIN

CDR1- SEQ ID NO 159: AATTATGGTCTCAGC
CDR2- SEQ ID NO 160: 
TGGATCAGCGCTAATAATGGCGACACAAATTATGGACAGGAATTCCAG
GGC
CDR3- SEQ ID NO 161:
   GACTCCAGCAGCAACTGGGCCCGCTGGTTTTTCGATCTC

### BAK278D6

### LIGHT CHAIN

CDR1- SEQ ID NO 162:
   GGGGGAAACAACATTGGAAGTAAACTTGTACAC
CDR2- SEQ ID NO 163: GATGATGGCGACCGGCCCTCA
CDR3- SEQ ID NO 164:
   CAGGTGTGGGATACTGGTAGTGATCCCGTGGTA

### BAK502G9

### HEAVY CHAIN

CDR1- SEQ ID NO 165: AATTATGGTCTCAGC
CDR2- SEQ ID NO 166: 
TGGATCAGCGCTAATAATGGCGACACAAATTATGGACAGGAATTCCAG
GGC
CDR3- SEQ ID NO 167:
   GACTCCAGCAGCAGCTGGGCCCGCTGGTTTTTCGATCTC

### BAK502G9

### LIGHT CHAIN

CDR1- SEQ ID NO 168:
   GGGGGAAACATCATTGGAAGTAAACTTGTACAC
CDR2- SEQ ID NO 169: GATGATGGCGACCGGCCCTCA
CDR3- SEQ ID NO 170:
   CAGGTGTGGGATACTGGTAGTGATCCCGTGGTA

### CH Domains- SEQ ID NO: 171

ASTKGPSVFPLAPCSRSTSESTAALGCLVKDYFPEPVTVSWNSGALTSGV
HTFPAVLQSSGLYSLSSVVTVPSSSLGTKTYTCNVDHKPSNTKVDKRVES
KYGPPCPSCPAPEFLGGPSVFLFPPKPKDTLMISRTPEVTCVVVDVSQED
PEVQFNWYVDGVEVHNAKTKPREEQFNSTYRVVSVLTVLHQDWLNGKE
YKCKVSNKGLPSSIEKTISKAKGQPREPQVYTLPPSQEEMTKNQVSLTCL
VKGFYPSDIAVEWESNGQPENNYKTTPPVLDSDGSFFLYSRLTVDKSRW
QEGNVFSCSVMHEALHNHYTQKSLSLSLGK

### CL Domain- SEQ ID NO: 172

QPKAAPSVTLFPPSSEELQANKATLVCLISDFYPGAVTVAWKADSSPVKA
GVETTTPSKQSNNKYAASSYLSLTPEQWKSHRSYSCQVTHEGSTVEKTV
APTECS

SEQUENCE LISTING<110> MedImmune Limited<120>
Therapies for improving pulmonary function<130> MED0593.PRO<140> <141>
<150> <151> <150> <151> <150> <151> <150> <151>
<160> 249 <170> PatentIn version 3.1<210> 1<211> 5<212> PRT<213>
Homo sapiens<400> 1

Asn Tyr Gly Leu Ser
1               5

<210> 2<211> 17<212> PRT<213> Homo sapiens<400> 2

Trp Ile Ser Ala Asn Asn Gly Asp Thr Asn Tyr Gly Gln Glu Phe Gln Gly
1               5                   10                  15

<210> 3<211> 13<212> PRT<213> Homo sapiens<400> 3

Asp Ser Ser Ser Asn Trp Ala Arg Trp Phe Phe Asp Leu
1               5                   10

<210> 4<211> 11<212> PRT<213> Homo sapiens<400> 4

Gly Gly Asn Asn Ile Gly Ser Lys Leu Val His
1               5                   10

<210> 5<211> 7<212> PRT<213> Homo sapiens<400> 5

Asp Asp Gly Asp Arg Pro Ser
1               5

<210> 6<211> 11<212> PRT<213> Homo sapiens<400> 6

Gln Val Trp Asp Thr Gly Ser Asp Pro Val Val
1               5                   10

<210> 7<211> 5<212> PRT<213> Homo sapiens<400> 7

Asn Tyr Gly Leu Ser
1               5

<210> 8<211> 17<212> PRT<213> Homo sapiens<400> 8

Trp Ile Ser Ala Asn Asn Gly Asp Thr Asn Tyr Gly Gln Glu Phe Gln Gly
1               5                   10                  15

<210> 9<211> 13<212> PRT<213> Homo sapiens<400> 9

Asp Ser Ser Ser Ser Trp Ala Arg Trp Phe Phe Asp Leu
1               5                   10

<210> 10<211> 11<212> PRT<213> Homo sapiens<400> 10

Gly Gly Asn Ile Ile Gly Ser Lys Leu Val His
1               5                   10

<210>  11<211>  7<212>  PRT<213>  Homo sapiens<400>  11

Asp Asp Gly Asp Arg Pro Ser
1               5

<210>  12<211>  11<212>  PRT<213>  Homo sapiens<400>  12

Gln Val Trp Asp Thr Gly Ser Asp Pro Val Val
1               5                   10

<210>  13<211>  122<212>  PRT<213>  Homo sapiens<400>  13

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Arg Asn Tyr
            20                  25                  30

Gly Leu Ser Trp Val Arg Gln Ala Pro Gly Gln Gly Leu Glu Trp Met
        35                  40                  45

Gly Trp Ile Ser Ala Asn Asn Gly Asp Thr Asn Tyr Gly Gln Glu Phe
    50                  55                  60

Gln Gly Arg Ile Thr Met Thr Thr Glu Thr Ser Thr Asn Thr Ala His
65                  70                  75                  80

Met Glu Leu Arg Ser Leu Arg Ser Asp Asp Thr Ala Val Tyr Tyr Cys
                85                  90                  95

Val Arg Asp Ser Ser Ser Asn Trp Ala Arg Trp Phe Phe Asp Leu Trp
            100                 105                 110

Gly Lys Gly Thr Met Val Thr Val Ser Ser
        115                 120

<210>  14<211>  108<212>  PRT<213>  Homo sapiens<400>  14

Ser Tyr Val Leu Thr Gln Pro Pro Ser Val Ser Val Ala Pro Gly Gln
1               5                   10                  15

Thr Ala Arg Ile Pro Cys Gly Gly Asn Asn Ile Gly Ser Lys Leu Val
            20                  25                  30

His Trp Tyr Gln Gln Lys Pro Gly Gln Ala Pro Val Leu Val Val Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Ile Asp Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>  15<211>  122<212>  PRT<213>  Homo sapiens<400>  15

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Ser Ala Asn Asn Gly Asp Thr Asn Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210>  16<211>  108<212>  PRT<213>  Homo sapiens<400>  16

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser

50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>  17<211>  30<212>  PRT<213>  Homo sapiens<400>  17

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Arg
            20                  25                  30

<210>  18<211>  14<212>  PRT<213>  Homo sapiens<400>  18

Trp Val Arg Gln Ala Pro Gly Gln Gly Leu Glu Trp Met Gly
1               5                   10

<210>  19<211>  32<212>  PRT<213>  Homo sapiens<400>  19

Arg Ile Thr Met Thr Thr Glu Thr Ser Thr Asn Thr Ala His Met Glu
1               5                   10                  15

Leu Arg Ser Leu Arg Ser Asp Asp Thr Ala Val Tyr Tyr Cys Val Arg
            20                  25                  30

<210>  20<211>  22<212>  PRT<213>  Homo sapiens<400>  20

Ser Tyr Val Leu Thr Gln Pro Pro Ser Val Ser Val Ala Pro Gly Gln
1               5                   10                  15

Thr Ala Arg Ile Pro Cys
            20

<210>  21<211>  15<212>  PRT<213>  Homo sapiens<400>  21

Trp Tyr Gln Gln Lys Pro Gly Gln Ala Pro Val Leu Val Val Tyr
1               5                   10                  15

<210>  22<211>  32<212>  PRT<213>  Homo sapiens<400>  22

Gly Ile Pro Glu Arg Phe Ser Gly Ser Asn Ser Gly Asn Thr Ala Thr
1               5                   10                  15

Leu Thr Ile Ser Arg Ile Asp Ala Gly Asp Glu Ala Asp Tyr Tyr Cys
            20                  25                  30

<210> 23<211> 120<212> PRT<213> Homo sapiens<400> 23

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Gly Ala Ala Gly Glu Gly Tyr Tyr Gly Tyr Trp Gly Arg
            100                 105                 110

Gly Thr Leu Val Thr Val Ser Ser
        115                 120

<210> 24<211> 110<212> PRT<213> Homo sapiens<400> 24

Asn Phe Met Leu Thr Gln Pro His Ser Val Ser Glu Ser Pro Gly Lys
1               5                   10                  15

Thr Val Thr Ile Ser Cys Thr Arg Ser Ser Gly Ser Ile Ala Ser Asn
            20                  25                  30

Tyr Val Gln Trp Tyr Gln Gln Arg Pro Gly Ser Ala Pro Thr Thr Val
        35                  40                  45

Ile Tyr Asp Asp Asn Gln Arg Pro Ser Gly Val Pro Asp Arg Phe Ser
    50                  55                  60

Gly Ser Ile Asp Ser Ser Ser Asn Ser Ala Ser Leu Thr Ile Ser Gly
65                  70                  75                  80

Leu Lys Thr Glu Asp Glu Ala Asp Tyr Tyr Cys Gln Ser Tyr Asp Ser
                85                  90                  95

Asn Asn Asp Val Phe Gly Gly Gly Thr Lys Val Thr Val Leu
            100                 105                 110

<210> 25<211> 123<212> PRT<213> Homo sapiens<400> 25

Gln Val Gln Leu Gln Glu Ser Gly Gly Gly Gly Leu Val Gln Pro Gly
1               5                   10                  15

Gly Ser Leu Arg Leu Ser Cys Ala Ala Ser Gly Phe Thr Phe Ser Ser
            20                  25                  30

Tyr Gly Met Ser Trp Val Arg Gln Ala Pro Gly Lys Gly Leu Glu Trp
        35                  40                  45

Val Ser Ser Ile Ser Ala Ser Gly Asp Ser Thr Phe Tyr Ala Asp Ser
    50                  55                  60

Val Lys Gly Arg Phe Thr Ile Ser Arg Asp Asn Asn Lys Asn Met Val
65                  70                  75                  80

Phe Leu Gln Val Asn Ser Leu Arg Ala Asp Asp Thr Ala Val Tyr Phe
                85                  90                  95

Cys Ala Lys Asp Trp Ser Gln Trp Leu Val Gly Asp Ala Phe Asp Val
            100                 105                 110

Trp Gly Arg Gly Thr Thr Val Thr Val Ser Ser
        115                 120

<210> 26<211> 108<212> PRT<213> Homo sapiens<400> 26

Asp Ile Gln Leu Thr Gln Ser Pro Ser Thr Leu Ser Ala Ser Val Gly
1               5                   10                  15

Asp Arg Val Thr Ile Thr Cys Arg Ala Ser Gln Ser Val Ser Leu Trp
            20                  25                  30

Val Ala Trp Tyr Gln Gln Arg Pro Gly Lys Ala Pro Lys Leu Leu Ile
        35                  40                  45

Tyr Asp Gly Ser Thr Leu Gln Ser Gly Val Pro Ala Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Lys Thr Phe Ser Thr
                85                  90                  95

Phe Gly Gln Gly Thr Lys Val Glu Ile Lys Arg Ala
            100                 105

<210>  27<211>  30<212>  PRT<213>  Homo sapiens<400>  27

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr
            20                  25                  30

<210>  28<211>  14<212>  PRT<213>  Homo sapiens<400>  28

Trp Tyr Arg Gln Ala Pro Gly Gln Gly Leu Glu Trp Met Gly
1               5                   10

<210>  29<211>  32<212>  PRT<213>  Homo sapiens<400>  29

Arg Val Thr Met Thr Thr Asp Thr Ser Thr Ser Thr Ala Tyr Met Glu
1               5                   10                  15

Leu Arg Ser Leu Arg Ser Asp Asp Thr Ala Val Tyr Tyr Cys Ala Arg
            20                  25                  30

<210>  30<211>  22<212>  PRT<213>  Homo sapiens<400>  30

Ser Tyr Val Leu Thr Gln Pro Pro Ser Val Ser Val Ala Pro Gly Lys
1               5                   10                  15

Thr Ala Arg Ile Thr Cys
            20

<210>  31<211>  15<212>  PRT<213>  Homo sapiens<400>  31

Trp Tyr Gln Gln Lys Pro Gly Gln Ala Pro Val Leu Val Ile Tyr
1               5                   10                  15

<210>  32<211>  32<212>  PRT<213>  Homo sapiens<400>  32

Gly Ile Pro Glu Arg Phe Ser Gly Ser Asn Ser Gly Asn Thr Ala Thr
1               5                   10                  15

Leu Thr Ile Ser Arg Val Glu Ala Gly Asp Glu Ala Asp Tyr Tyr Cys
            20                  25                  30

<210>  33<211>  122<212>  PRT<213>  Homo sapiens<400>  33

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Gly Lys Ala Thr Thr Glu Glu Gly Tyr Tyr Gly Tyr Trp
            100                 105                 110

Gly Arg Gly Thr Leu Val Thr Val Ser Ser
        115                 120

<210> 34<211> 110<212> PRT<213> Homo sapiens<400> 34

Asn Phe Met Leu Thr Gln Pro His Ser Val Ser Glu Ser Pro Gly Lys
1               5                   10                  15

Thr Val Thr Ile Ser Cys Thr Arg Ser Ser Gly Ser Ile Ala Ser Asn
            20                  25                  30

Tyr Val Gln Trp Tyr Gln Gln Arg Pro Gly Ser Ala Pro Thr Thr Val
        35                  40                  45

Ile Tyr Asp Asp Asn Gln Arg Pro Ser Gly Val Pro Asp Arg Phe Ser
    50                  55                  60

Gly Ser Ile Asp Ser Ser Ser Asn Ser Ala Ser Leu Thr Ile Ser Gly
65                  70                  75                  80

Leu Lys Thr Glu Asp Glu Ala Asp Tyr Tyr Cys Gln Ser Tyr Asp Ser
                85                  90                  95

Asn Asn Asp Val Phe Gly Gly Gly Thr Lys Val Thr Val Leu
            100                 105                 110

<210> 35<211> 122<212> PRT<213> Homo sapiens<400> 35

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Glu Gln Thr
            20                  25                  30

Gly Val Ser Trp Val Arg Gln Ala Pro Gly Gln Gly Leu Glu Trp Met
        35                  40                  45

Gly Trp Ile Ser Ala Asn Asn Gly Asp Thr Asn Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210> 36<211> 108<212> PRT<213> Homo sapiens<400> 36

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210> 37<211> 122<212> PRT<213> Homo sapiens<400> 37

Gln Val Gln Leu Val Gln Ser Gly Ala Glu Val Lys Lys Pro Gly Ala

1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Asn Tyr Asp Gly Gly Asn Thr Gln Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210>  38<211>  108<212>  PRT<213>  Homo sapiens<400>  38

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>  39<211>  122<212>  PRT<213>  Homo sapiens<400>  39

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Ser Gly Leu Asn Gly Glu Thr Leu Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210>  40<211>  108<212>  PRT<213>  Homo sapiens<400>  40

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>  41<211>  122<212>  PRT<213>  Homo sapiens<400>  41

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Ala Thr Pro Asp Gly Gln Thr Ser Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Asn Ser Ser Trp Ala Arg Trp Phe Phe Asp Leu Trp
            100                 105                 110

Gly Arg Gly Thr Leu Val Thr Val Ser Ser
        115                 120

<210>  42<211>  108<212>  PRT<213>  Homo sapiens<400>  42

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>  43<211>  122<212>  PRT<213>  Homo sapiens<400>  43

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ile Asp Thr
            20                  25                  30

Gly Val Ser Trp Val Arg Gln Ala Pro Gly Gln Gly Leu Glu Trp Met
        35                  40                  45

Gly Trp Ile Ser Ala Asn Asn Gly Asp Thr Asn Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210>  44<211>  108<212>  PRT<213>  Homo sapiens<400>  44

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>  45<211>  122<212>  PRT<213>  Homo sapiens<400>  45

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Ser Gly Ser Asn Gly Tyr Thr Ser Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210>  46<211>  108<212>  PRT<213>  Homo sapiens<400>  46

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>   47<211>   122<212>   PRT<213>   Homo sapiens<400>   47

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Asn Asp Ala Thr Gly Asp Thr Gln Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210>   48<211>   108<212>   PRT<213>   Homo sapiens<400>   48

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu

            100                 105

<210> 49<211> 122<212> PRT<213> Homo sapiens<400> 49

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Gly Leu Ser Trp Val Arg Gln Ala Pro Gly Gln Gly Leu Glu Trp Met
        35                  40                  45

Gly Trp Ile Arg Asn Ile Asp Gly Tyr Thr Ile Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210> 50<211> 108<212> PRT<213> Homo sapiens<400> 50

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210> 51<211> 122<212> PRT<213> Homo sapiens<400> 51

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Asp Asp Asp Ser Gly Thr Thr Ile Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210> 52<211> 108<212> PRT<213> Homo sapiens<400> 52

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210> 53<211> 122<212> PRT<213> Homo sapiens<400> 53

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ala Asn Thr
            20                  25                  30

Gly Ile Ser Trp Val Arg Gln Ala Pro Gly Gln Gly Leu Glu Trp Met
        35                  40                  45

Gly Trp Ile Ser Ala Asn Asn Gly Asp Thr Asn Tyr Gly Gln Glu Phe
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp Ser Ser Ser Ser Trp Ala Arg Trp Phe Phe Asp Leu Trp
            100                 105                 110

Gly Arg Gly Thr Leu Val Thr Val Ser Ser
        115                 120

<210> 54<211> 108<212> PRT<213> Homo sapiens<400> 54

Ser Tyr Val Leu Thr Gln Pro Pro Ser Val Ser Val Ala Pro Gly Lys
1               5                   10                  15

Thr Ala Arg Ile Thr Cys Gly Gly Asn Ile Ile Gly Ser Lys Leu Val
            20                  25                  30

His Trp Tyr Gln Gln Lys Pro Gly Gln Ala Pro Val Leu Val Ile Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Val Glu Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu
            100                 105

<210>  55<211>  5<212>  PRT<213>  Homo sapiens<400>  55

Ser Tyr Ala Met Ser
1               5

<210>  56<211>  17<212>  PRT<213>  Homo sapiens<400>  56

Ala Ile Ser Gly Ser Gly Gly Ser Thr Tyr Tyr Ala Asp Ser Val Lys
1               5                   10                  15

Gly

<210>  57<211>  11<212>  PRT<213>  Homo sapiens<400>  57

Val Gly Ala Ala Gly Glu Gly Tyr Tyr Gly Tyr
1               5                   10

<210>  58<211>  13<212>  PRT<213>  Homo sapiens<400>  58

Thr Arg Ser Ser Gly Ser Ile Ala Ser Asn Tyr Val Gln
1               5                   10

<210>  59<211>  7<212>  PRT<213>  Homo sapiens<400>  59

Asp Asp Asn Gln Arg Pro Ser
1               5

<210>  60<211>  9<212>  PRT<213>  Homo sapiens<400>  60

Gln Ser Tyr Asp Ser Asn Asn Asp Val
1               5

<210>  61<211>  5<212>  PRT<213>  Homo sapiens<400>  61

Gln Thr Gly Val Ser
1               5

<210>  62<211>  17<212>  PRT<213>  Homo sapiens<400>  62

Trp Ile Ser Ala Asn Asn Gly Asp Thr Asn Tyr Gly Gln Glu Phe Gln Gly
1               5                   10                  15

<210>  63<211>  13<212>  PRT<213>  Homo sapiens<400>  63

Asp Ser Ser Ser Ser Trp Ala Arg Trp Phe Phe Asp Leu
1               5                   10

<210>  64<211>  11<212>  PRT<213>  Homo sapiens<400>  64

Gly Gly Asn Ile Ile Gly Ser Lys Leu Val His
1               5                   10

<210>  65<211>  7<212>  PRT<213>  Homo sapiens<400>  65

Asp Asp Gly Asp Arg Pro Ser
1               5

<210>  66<211>  11<212>  PRT<213>  Homo sapiens<400>  66

Gln Val Trp Asp Thr Gly Ser Asp Pro Val Val
1               5                   10

<210>  67<211>  5<212>  PRT<213>  Homo sapiens<400>  67

Asn Thr Gly Ile Ser
1               5

<210>  68<211>  17<212>  PRT<213>  Homo sapiens<400>  68

Trp Ile Ser Ala Asn Asn Gly Asp Thr Asn Tyr Gly Gln Glu Phe Gln Gly
1               5                   10                  15

<210>  69<211>  13<212>  PRT<213>  Homo sapiens<400>  69

Asp Ser Ser Ser Ser Trp Ala Arg Trp Phe Phe Asp Leu
1               5                   10

<210>  70<211>  11<212>  PRT<213>  Homo sapiens<400>  70

Gly Gly Asn Ile Ile Gly Ser Lys Leu Val His
1               5                   10

<210>  71<211>  7<212>  PRT<213>  Homo sapiens<400>  71

Asp Asp Gly Asp Arg Pro Ser
1               5

<210>  72<211>  11<212>  PRT<213>  Homo sapiens<400>  72

Gln Val Trp Asp Thr Gly Ser Asp Pro Val Val
1               5                   10

<210>  73<211>  5<212>  PRT<213>  Homo sapiens<400>  73

Asn Tyr Gly Leu Ser
1               5

<210>  74<211>  17<212>  PRT<213>  Homo sapiens<400>  74

Trp Ile Ser Gly Ser Asn Gly Tyr Thr Ser Tyr Gly Gln Glu Phe Gln Gly
1               5                   10                  15

<210>  75<211>  13<212>  PRT<213>  Homo sapiens<400>  75

Asp Ser Ser Ser Ser Trp Ala Arg Trp Phe Phe Asp Leu
1               5                   10

<210>  76<211>  11<212>  PRT<213>  Homo sapiens<400>  76

Gly Gly Asn Ile Ile Gly Ser Lys Leu Val His
1               5                   10

<210>  77<211>  7<212>  PRT<213>  Homo sapiens<400>  77

Asp Asp Gly Asp Arg Pro Ser
1               5

<210>  78<211>  11<212>  PRT<213>  Homo sapiens<400>  78

Gln Val Trp Asp Thr Gly Ser Asp Pro Val Val
1               5                   10

<210>  79<211>  5<212>  PRT<213>  Homo sapiens<400>  79

Asn Tyr Gly Leu Ser
1               5

<210>  80<211>  17
<212>  PRT<213>  Homo sapiens<400>  80

Trp Ile Asn Asp Ala Thr Gly Asp Thr Gln Tyr Gly Gln Glu Phe Gln Gly
1               5                   10                  15

<210>  81<211>  13<212>  PRT<213>  Homo sapiens<400>  81

Asp Ser Ser Ser Ser Trp Ala Arg Trp Phe Phe Asp Leu
1               5                   10

<210>  82<211>  11<212>  PRT<213>  Homo sapiens<400>  82

Gly Gly Asn Ile Ile Gly Ser Lys Leu Val His
1               5                   10

<210>  83<211>  7<212>  PRT<213>  Homo sapiens<400>  83

Asp Asp Gly Asp Arg Pro Ser
1               5

<210>  84<211>  11<212>  PRT<213>  Homo sapiens<400>  84

Gln Val Trp Asp Thr Gly Ser Asp Pro Val Val
1               5                   10

<210>  85<211>  5<212>  PRT<213>  Homo sapiens<400>  85

Asp Thr Gly Val Ser
1               5


<210>  86<211>  17<212>  PRT<213>  Homo sapiens<400>  86

Trp Ile Ser Ala Asn Asn Gly Asp Thr Asn Tyr Gly Gln Glu Phe Gln Gly
1               5                   10                  15


<210>  87<211>  13<212>  PRT<213>  Homo sapiens<400>  87

Asp Ser Ser Ser Ser Trp Ala Arg Trp Phe Phe Asp Leu
1               5                   10


<210>  88<211>  11<212>  PRT<213>  Homo sapiens<400>  88

Gly Gly Asn Ile Ile Gly Ser Lys Leu Val His
1               5                   10


<210>  89<211>  7<212>  PRT<213>  Homo sapiens<400>  89

Asp Asp Gly Asp Arg Pro Ser
1               5


<210>  90<211>  11<212>  PRT<213>  Homo sapiens<400>  90

Gln Val Trp Asp Thr Gly Ser Asp Pro Val Val
1               5                   10


<210>  91<211>  5<212>  PRT<213>  Homo sapiens<400>  91

Asn Tyr Gly Leu Ser
1               5


<210>  92<211>  17<212>  PRT<213>  Homo sapiens<400>  92

Trp Ile Ala Thr Pro Asp Gly Gln Thr Ser Tyr Gly Gln Glu Phe Gln Gly
1               5                   10                  15


<210>  93<211>  13<212>  PRT<213>  Homo sapiens<400>  93

Asp Ser Asn Ser Ser Trp Ala Arg Trp Phe Phe Asp Leu
1               5                   10


<210>  94<211>  11<212>  PRT<213>  Homo sapiens<400>  94

Gly Gly Asn Ile Ile Gly Ser Lys Leu Val His
1               5                   10


<210>  95<211>  7<212>  PRT<213>  Homo sapiens<400>  95

Asp Asp Gly Asp Arg Pro Ser
1               5

<210>  96<211>  11<212>  PRT<213>  Homo sapiens<400>  96

Gln Val Trp Asp Thr Gly Ser Asp Pro Val Val
1               5                   10

<210>  97<211>  5<212>  PRT<213>  Homo sapiens<400>  97

Asn Tyr Gly Leu Ser
1               5

<210>  98<211>  17<212>  PRT<213>  Homo sapiens<400>  98

Trp Ile Asn Tyr Asp Gly Gly Asn Thr Gln Tyr Gly Gln Glu Phe Gln Gly
1               5                   10                  15

<210>  99<211>  13<212>  PRT<213>  Homo sapiens<400>  99

Asp Ser Ser Ser Ser Trp Ala Arg Trp Phe Phe Asp Leu
1               5                   10

<210>  100<211>  11<212>  PRT<213>  Homo sapiens<400>  100

Gly Gly Asn Ile Ile Gly Ser Lys Leu Val His
1               5                   10

<210>  101<211>  7<212>  PRT<213>  Homo sapiens<400>  101

Asp Asp Gly Asp Arg Pro Ser
1               5

<210>  102<211>  11<212>  PRT<213>  Homo sapiens<400>  102

Gln Val Trp Asp Thr Gly Ser Asp Pro Val Val
1               5                   10

<210>  103<211>  5<212>  PRT<213>  Homo sapiens<400>  103

Asp Tyr Gly Leu Ser
1               5

<210>  104<211>  16<212>  PRT<213>  Homo sapiens<400>  104

Trp Arg Ile Asn Asp Gly Tyr Thr Ile Tyr Gly Gln Glu Phe Gln Gly
1               5                   10                  15

<210>  105<211>  13<212>  PRT<213>  Homo sapiens<400>  105

Asp Ser Ser Ser Ser Trp Ala Arg Trp Phe Phe Asp Leu
1               5                   10

<210>  106<211>  11<212>  PRT<213>  Homo sapiens<400>  106

Gly Gly Asn Ile Ile Gly Ser Lys Leu Val His
1               5                   10


<210>  107<211>  7<212>  PRT<213>  Homo sapiens<400>  107

Asp Asp Gly Asp Arg Pro Ser
1               5


<210>  108<211>  11<212>  PRT<213>  Homo sapiens<400>  108

Gln Val Trp Asp Thr Gly Ser Asp Pro Val Val
1               5                   10


<210>  109<211>  366<212>  DNA<213>  Homo sapiens<400>  109
caggtgcagc tggtgcagtc tggggctgag gtgaagaagc ctggggcctc agtgaaggtc      60

tcctgcaagg cttctggtta cacctttaca aattatggtc tcagctgggt gcgacaggcc     120

cctggacaag ggcttgagtg gatgggatgg atcagcgcta ataatggcga cacaaattat     180

ggacaggaat tccagggcag agtcaccatg accacagata catccacgag cacagcctac     240

atggagttga ggagcctgag atctgacgac acggccgttt attactgtgc gagagactcc     300

agcagcaact gggcccgctg gtttttcgat ctctggggcc gggggacact ggtcaccgtc     360

tcctca                                                                366


<210>  110<211>  327<212>  DNA<213>  Homo sapiens<400>  110
tcctatgtgc tgactcagcc accctcggtg tcagtggccc caggtaagac ggccaggatt      60

acctgtgggg gaaacaacat tggaagtaaa cttgtacact ggtaccagca gaagccaggc     120

caggcccctg tgctggtcat ctatgatgat ggcgaccggc cctcagggat ccctgagcga     180

ttctctggct ccaactctgg gaacacggcc accctgacca tcagcagggt cgaggccggg     240

gatgaggccg actattattg tcaggtgtgg gatactggta gtgatcccgt ggtattcggc     300

ggagggacca agctgaccgt cctaggt                                         327


<210>  111<211>  366<212>  DNA<213>  Homo sapiens<400>  111
caggtccagc tggtgcagtc tggggctgag gtgaagaagc ctggggcctc agtgaaggtc      60

tcctgcaagg cttctggtta cacctttaca aattatggtc tcagctgggt gcgacaggcc     120

cctggacaag ggcttgagtg gatgggatgg atcagcgcta ataatggcga cacaaattat     180

ggacaggaat tccagggcag agtcaccatg accacagata catccacgag cacagcctac     240

atggagttga ggagcctgag atctgacgac acggccgttt attactgtgc gagagactcc     300

agcagcagct gggcccgctg gtttttcgat ctctggggcc gggggacact ggtcaccgtc     360

tcctca                                                                366

<210>  112<211>  327<212>  DNA<213>  Homo sapiens<400>  112
tcctatgtgc tgactcagcc accctcggtg tcagtggccc caggaaagac ggccaggatt      60

acctgtgggg gaaacatcat tggaagtaaa cttgtacact ggtaccagca gaagccaggc     120

caggcccctg tgctggtcat ctatgatgat ggcgaccggc cctcagggat ccctgagcga     180

ttctctggct ccaactctgg gaacacggcc accctgacca tcagcagggt cgaggccggg     240

gatgaggccg actattattg tcaggtgtgg gatactggta gtgatcccgt ggtattcggc     300

ggagggacca agctgaccgt cctaggt                                         327

<210>  113<211>  366<212>  DNA<213>  Homo sapiens<400>  113
caggtccagc tggtgcagtc tggggctgag gtgaagaagc ctggggcctc agtgaaggtc      60

tcctgcaagg cttctggtta cacctttaca aattatggtc tcagctgggt gcgacaggcc     120

cctggacaag ggcttgagtg gatgggatgg atctccggct tgaacggcga gacattgtat     180

ggacaggaat tccagggcag agtcaccatg accacagata catccacgag cacagcctac     240

atggagttga ggagcctgag atctgacgac acggccgttt attactgtgc gagagactcc     300

agcagcagct gggcccgctg gtttttcgat ctctggggcc gggggacact ggtcaccgtc     360

tcctca                                                                366

<210>  114<211>  327<212>  DNA<213>  Homo sapiens<400>  114
tcctatgtgc tgactcagcc accctcggtg tcagtggccc caggaaagac ggccaggatt      60

acctgtgggg gaaacatcat tggaagtaaa cttgtacact ggtaccagca gaagccaggc     120

caggcccctg tgctggtcat ctatgatgat ggcgaccggc cctcagggat ccctgagcga     180

ttctctggct ccaactctgg gaacacggcc accctgacca tcagcagggt cgaggccggg     240

gatgaggccg actattattg tcaggtgtgg gatactggta gtgatcccgt ggtattcggc     300

ggagggacca agctgaccgt cctaggt                                         327

<210>  115<211>  366<212>  DNA<213>  Homo sapiens<400>  115
caggtgcagc tggtgcagtc tggggctgag gtgaagaagc ctggggcctc agtgaaggtc      60

tcctgcaagg cttctggtta cacctttaca aattatggtc tcagctgggt gcgacaggcc     120

cctggacaag ggcttgagtg gatgggatgg atcgcaaccc cagacggcca gacaagctat     180

ggacaggaat tccagggcag agtcaccatg accacagata catccacgag cacagcctac     240

atggagttga ggagcctgag atctgacgac acggccgttt attactgtgc gagagactcc     300

aacagcagct gggcccgctg gtttttcgat ctctggggcc gggggacact ggtcaccgtc     360

tcctca                                                                366

<210>  116<211>  327<212>  DNA<213>  Homo sapiens<400>  116
tcctatgtgc tgactcagcc accctcggtg tcagtggccc caggaaagac ggccaggatt      60

acctgtgggg gaaacatcat tggaagtaaa cttgtacact ggtaccagca gaagccaggc     120

caggcccctg tgctggtcat ctatgatgat ggcgaccggc cctcagggat ccctgagcga     180

ttctctggct ccaactctgg gaacacggcc accctgacca tcagcagggt cgaggccggg     240

gatgaggccg actattattg tcaggtgtgg gatactggta gtgatcccgt ggtattcggc     300

ggagggacca agctgaccgt cctaggt                                         327

<210>  117<211>  366<212>  DNA<213>  Homo sapiens<400>  117
caagtgcagc tggtgcagtc tggggctgag gtgaagaagc ctggggcctc agtgaaggtc      60

tcctgcaagg cttctggtta cacctttgag cagaccggcg tctcctgggt gcgacaggcc     120

cctggacaag ggcttgagtg gatgggatgg atcagcgcta ataatggcga cacaaattat     180

ggacaggaat tccagggcag agtcaccatg accacagata catccacgag cacagcctac     240

atggagttga ggagcctgag atctgacgac acggccgttt attactgtgc gagagactcc     300

agcagcagct gggcccgctg gtttttcgat ctctggggcc gggggacact ggtcaccgtc     360

tcctca                                                                366

<210>  118<211>  327<212>  DNA<213>  Homo sapiens<400>  118
tcctatgtgc tgactcagcc accctcggtg tcagtggccc caggaaagac ggccaggatt      60

acctgtgggg gaaacatcat tggaagtaaa cttgtacact ggtaccagca gaagccaggc     120

caggcccctg tgctggtcat ctatgatgat ggcgaccggc cctcagggat ccctgagcga     180

ttctctggct ccaactctgg gaacacggcc accctgacca tcagcagggt cgaggccggg     240

gatgaggccg actattattg tcaggtgtgg gatactggta gtgatcccgt ggtattcggc     300

ggagggacca agctgaccgt cctaggt                                         327

<210>  119<211>  366<212>  DNA<213>  Homo sapiens<400>  119
caggtgcagc tggtgcagtc tggggctgag gtgaagaagc ctggggcctc agtgaaggtc      60

tcctgcaagg cttctggtta cacctttatc gacaccgggg tctcctgggt gcgacaggcc     120

cctggacaag ggcttgagtg gatgggatgg atcagcgcta ataatggcga cacaaattat     180

ggacaggaat tccagggcag agtcaccatg accacagata catccacgag cacagcctac     240

atggagttga ggagcctgag atctgacgac acggccgttt attactgtgc gagagactcc     300

agcagcagct gggcccgctg gtttttcgat ctctggggcc gggggacact ggtcaccgtc     360

tcctca                                                                366

<210>  120<211>  327<212>  DNA<213>  Homo sapiens<400>  120
tcctatgtgc tgactcagcc accctcggtg tcagtggccc caggaaagac ggccaggatt      60

acctgtgggg gaaacatcat tggaagtaaa cttgtacact ggtaccagca gaagccaggc 120

caggcccctg tgctggtcat ctatgatgat ggcgaccggc cctcagggat ccctgagcga 180

ttctctggct ccaactctgg gaacacggcc accctgacca tcagcagggt cgaggccggg 240

gatgaggccg actattattg tcaggtgtgg gatactggta gtgatcccgt ggtattcggc 300

ggagggacca agctgaccgt cctaggt 327

<210> 121<211> 366<212> DNA<213> Homo sapiens<400> 121
caggtgcagc tggtgcagtc tggggctgag gtgaagaagc ctggggcctc agtgaaggtc 60

tcctgcaagg cttctggtta cacctttaca aattatggtc tcagctgggt gcgacaggcc 120

cctggacaag ggcttgagtg gatgggatgg atcaactacg acggcggcaa cacacagtat 180

ggacaggaat tccagggcag agtcaccatg accacagata catccacgag cacagcctac 240

atggagttga ggagcctgag atctgacgac acggccgttt attactgtgc gagagactcc 300

agcagcagct gggcccgctg gtttttcgat ctctggggcc gggggacact ggtcaccgtc 360

tcctca 366

<210> 122<211> 327<212> DNA<213> Homo sapiens<400> 122
tcctatgtgc tgactcagcc accctcggtg tcagtggccc caggaaagac ggccaggatt 60

acctgtgggg gaaacatcat tggaagtaaa cttgtacact ggtaccagca gaagccaggc 120

caggcccctg tgctggtcat ctatgatgat ggcgaccggc cctcagggat ccctgagcga 180

ttctctggct ccaactctgg gaacacggcc accctgacca tcagcagggt cgaggccggg 240

gatgaggccg actattattg tcaggtgtgg gatactggta gtgatcccgt ggtattcggc 300

ggagggacca agctgaccgt cctaggt 327

<210> 123<211> 366<212> DNA<213> Homo sapiens<400> 123
caggtgcagc tggtgcagtc tggggctgag gtgaagaagc ctggggcctc agtgaaggtc 60

tcctgcaagg cttctggtta cacctttaca aattatggtc tcagctgggt gcgacaggcc 120

cctggacaag ggcttgagtg gatgggatgg atcagcggga gcaacggcta cacatcttat 180

ggacaggaat tccagggcag agtcaccatg accacagata cgtccacgag cacagcctac 240

atggagttga ggagcctgag atctgacgac acggccgttt attactgtgc gagagactcc 300

agcagcagct gggcccgctg gtttttcgat ctctggggcc gggggacact ggtcaccgtc 360

tcctca 366

<210> 124<211> 327<212> DNA<213> Homo sapiens<400> 124
tcctatgtgc tgactcagcc accctcggtg tcagtggccc caggaaagac ggccaggatt 60

acctgtgggg gaaacatcat tggaagtaaa cttgtacact ggtaccagca gaagccaggc 120

caggcccctg tgctggtcat ctatgatgat ggcgaccggc cctcagggat ccctgagcga 180

ttctctggct ccaactctgg gaacacggcc accctgacca tcagcagggt cgaggccggg 240

gatgaggccg actattattg tcaggtgtgg gatactggta gtgatcccgt ggtattcggc 300

ggagggacca agctgaccgt cctaggt 327

<210> 125<211> 366<212> DNA<213> Homo sapiens<400> 125
caggtgcagc tggtgcagtc tggggctgag gtgaagaagc ctggggcctc agtgaaggtc 60

tcctgcaagg cttctggtta cacctttaca aattatggtc tcagctgggt gcgacaggcc 120

cctggacaag ggcttgagtg gatgggatgg atcaacgacg ccaccggcga cacacagtat 180

ggacaggaat tccagggcag agtcaccatg accacagata catccacgag cacagcctac 240

atggagttga ggagcctgag atctgacgac acggccgttt attactgtgc gagagactcc 300

agcagcagct gggcccgctg gtttttcgat ctctggggcc gggggacact ggtcaccgtc 360

tcctca 366

<210> 126<211> 327<212> DNA<213> Homo sapiens<400> 126
tcctatgtgc tgactcagcc accctcggtg tcagtggccc caggaaagac ggccaggatt 60

acctgtgggg gaaacatcat tggaagtaaa cttgtacact ggtaccagca gaagccaggc 120

caggcccctg tgctggtcat ctatgatgat ggcgaccggc cctcagggat ccctgagcga 180

ttctctggct ccaactctgg gaacacggcc accctgacca tcagcagggt cgaggccggg 240

gatgaggccg actattattg tcaggtgtgg gatactggta gtgatcccgt ggtattcggc 300

ggagggacca agctgaccgt cctaggt 327

<210> 127<211> 366<212> DNA<213> Homo sapiens<400> 127
caggtgcagc tggtgcagtc tggggctgag gtgaagaagc ctggggcctc agtgaaggtc 60

tcctgcaagg cttctggtta cacctttaca gattatggtc tcagctgggt gcgacaggcc 120

cctggacaag ggctagagtg gatgggatgg atccgcaaca tcgacggcta cacaatttat 180

ggacaggaat tccagggcag agtcaccatg accacagata catccacgag cacagcctac 240

atggagttga ggagcctgag atctgacgac acggccgttt attactgtgc gagagactcc 300

agcagcagct gggcccgctg gtttttcgat ctctggggcc gggggacact ggtcaccgtc 360

tcctca 366

<210> 128<211> 327<212> DNA<213> Homo sapiens<400> 128
tcctatgtgc tgactcagcc accctcggtg tcagtggccc caggaaagac ggccaggatt 60

acctgtgggg gaaacatcat tggaagtaaa cttgtacact ggtaccagca gaagccaggc 120

caggcccctg tgctggtcat ctatgatgat ggcgaccggc cctcagggat ccctgagcga 180

ttctctggct ccaactctgg gaacacggcc accctgacca tcagcagggt cgaggccggg 240

gatgaggccg actattattg tcaggtgtgg gatactggta gtgatcccgt ggtattcggc 300

ggagggacca agctgaccgt cctaggt 327

<210> 129<211> 366<212> DNA<213> Homo sapiens<400> 129
caggtccagc tggtgcagtc tggggctgag gtgaagaagc ctggggcctc agtgaaggtc 60

tcctgcaagg cttctggtta cacctttaca aattatggtc tcagctgggt gcgacaggcc 120

cctggacaag ggcttgagtg gatgggatgg atcgacgacg acagcggcac gacaatatat 180

ggacaggaat tccagggcag agtcaccatg accacagata catccacgag cacagcctac 240

atggagttga ggagcctgag atctgacgac acggccgttt attactgtgc gagagactcc 300

agcagcagct gggcccgctg gtttttcgat ctctggggcc gggggacact ggtcaccgtc 360

tcctca 366

<210> 130<211> 327<212> DNA<213> Homo sapiens<400> 130
tcctatgtgc tgactcagcc accctcggtg tcagtggccc caggaaagac ggccaggatt 60

acctgtgggg gaaacatcat tggaagtaaa cttgtacact ggtaccagca gaagccaggc 120

caggcccctg tgctggtcat ctatgatgat ggcgaccggc cctcagggat ccctgagcga 180

ttctctggct ccaactctgg gaacacggcc accctgacca tcagcagggt cgaggccggg 240

gatgaggccg actattattg tcaggtgtgg gatactggta gtgatcccgt ggtattcggc 300

ggagggacca agctgaccgt cctaggt 327

<210> 131<211> 366<212> DNA<213> Homo sapiens<400> 131
caagtgcagt tggtgcagtc tggggctgag gtgaagaagc ctggggcctc agtgaaggtc 60

tcctgcaagg cttctggtta cacctttgcg aacaccggga tctcgtgggt gcgacaggcc 120

cctggacaag ggcttgagtg gatgggatgg atcagcgcta ataatggcga cacaaattat 180

ggacaggaat tccagggcag agtcaccatg accacagata catccacgag cacagcctac 240

atggagttga ggagcctgag atctgacgac acggccgttt attactgtgc gagagactcc 300

agcagcagct gggcccgctg gtttttcgat ctctggggtc gggggacact ggtcaccgtc 360

tcctca 366

<210> 132<211> 327<212> DNA<213> Homo sapiens<400> 132
tcctatgtgc tgactcagcc accctcggtg tcagtggccc caggaaagac ggccaggatt 60

acctgtgggg gaaacatcat tggaagtaaa cttgtacact ggtaccagca gaagccaggc 120

caggcccctg tgctggtcat ctatgatgat ggcgaccggc cctcagggat ccctgagcga 180

ttctctggct ccaactctgg gaacacggcc accctgacca tcagcagggt cgaggccggg 240

gatgaggccg actattattg tcaggtgtgg gatactggta gtgatcccgt ggtattcggc 300

ggagggacca agctgaccgt cctaggt 327

<210>  133<211>  122<212>  PRT<213>  Homo sapiens<400>  133

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Trp Ile Ser Ala Asn Asn Gly Glu Thr Asn Tyr Gly Gln Glu Phe
    50                  55                  60

Gln Gly Arg Val Thr Met Thr Thr Glu Thr Pro Thr Asn Thr Ala His
65                  70                  75                  80

Met Glu Leu Arg Ser Leu Thr Ser Asp Asp Thr Ala Val Tyr Tyr Cys
                85                  90                  95

Val Arg Asp Ser Ser Ser Asn Trp Ala Arg Trp Tyr Phe Asp Leu Trp
            100                 105                 110

Gly Gln Gly Thr Leu Val Thr Val Ser Ser
        115                 120

<210>  134<211>  109<212>  PRT<213>  Homo sapiens<400>  134

Ser Tyr Val Leu Thr Gln Pro Pro Ser Val Ser Val Ala Pro Gly Gln
1               5                   10                  15

Thr Ala Arg Ile Pro Cys Gly Gly Asn Asn Ile Gly Ser Lys Leu Val
            20                  25                  30

His Trp Tyr Gln Gln Lys Pro Gly Gln Ala Pro Val Leu Val Val Tyr
        35                  40                  45

Asp Asp Gly Asp Arg Pro Ser Gly Ile Pro Glu Arg Phe Ser Gly Ser
    50                  55                  60

Asn Ser Gly Asn Thr Ala Thr Leu Thr Ile Ser Arg Ile Asp Ala Gly
65                  70                  75                  80

Asp Glu Ala Asp Tyr Tyr Cys Gln Val Trp Asp Thr Gly Ser Asp Pro
                85                  90                  95

Val Val Phe Gly Gly Gly Thr Lys Leu Thr Val Leu Gly

100                 105

<210>  135<211>  5<212>  PRT<213>  Homo sapiens<400>  135

Asn Tyr Gly Leu Ser
1               5

<210>  136<211>  17<212>  PRT<213>  Homo sapiens<400>  136

Trp Ile Ser Ala Asn Asn Gly Glu Thr Asn Tyr Gly Gln Glu Phe Gln
1               5                   10                  15

Gly

<210>  137<211>  13<212>  PRT<213>  Homo sapiens<400>  137

Asp Ser Ser Ser Asn Trp Ala Arg Trp Tyr Phe Asp Leu
1               5                   10

<210>  138<211>  11<212>  PRT<213>  Homo sapiens<400>  138

Gly Gly Asn Asn Ile Gly Ser Lys Leu Val His
1               5                   10

<210>  139<211>  7<212>  PRT<213>  Homo sapiens<400>  139

Asp Asp Gly Asp Arg Pro Ser
1               5

<210>  140<211>  11<212>  PRT<213>  Homo sapiens<400>  140

Gln Val Trp Asp Thr Gly Ser Asp Pro Val Val
1               5                   10

<210>  141<211>  5<212>  PRT<213>  Homo sapiens<400>  141

Ser Tyr Ala Met Ser
1               5

<210>  142<211>  17<212>  PRT<213>  Homo sapiens<400>  142

Ala Ile Ser Gly Ser Gly Gly Ser Thr Tyr Tyr Ala Asp Ser Val Lys
1               5                   10                  15

Gly

<210>  143<211>  11<212>  PRT<213>  Homo sapiens<400>  143

Val Gly Ala Ala Gly Glu Gly Tyr Tyr Gly Tyr
1               5                   10

<210>  144<211>  13<212>  PRT<213>  Homo sapiens<400>  144

Thr Arg Ser Ser Gly Ser Ile Ala Ser Asn Tyr Val Glu
1               5                   10

<210>  145<211>  7<212>  PRT<213>  Homo sapiens<400>  145

Asp Asp Asn Gln Arg Pro Ser
1               5

<210>  146<211>  9<212>  PRT<213>  Homo sapiens<400>  146

Gln Ser Tyr Asp Ser Asn Asn Asp Val
1               5

<210>  147<211>  5<212>  PRT<213>  Homo sapiens<400>  147

Ser Tyr Ala Met Ser
1               5

<210>  148<211>  17<212>  PRT<213>  Homo sapiens<400>  148

Ala Ile Ser Gly Ser Gly Gly Ser Thr Tyr Tyr Ala Asp Ser Val Lys
1               5                   10                  15

Gly

<210>  149<211>  13<212>  PRT<213>  Homo sapiens<400>  149

Val Gly Arg Ala Thr Thr Asp Glu Gly Tyr Tyr Gly Tyr
1               5                   10

<210>  150<211>  13<212>  PRT<213>  Homo sapiens<400>  150

Thr Arg Ser Ser Gly Ser Ile Ala Ser Asn Tyr Val Gln
1               5                   10

<210>  151<211>  7<212>  PRT<213>  Homo sapiens<400>  151

Asp Asp Asn Gln Arg Pro Ser
1               5

<210>  152<211>  9<212>  PRT<213>  Homo sapiens<400>  152

Gln Ser Tyr Asp Ser Asn Asn Asp Val
1               5

<210>  153<211>  5<212>  PRT<213>  Homo sapiens<400>  153

Ser Tyr Ala Met Ser

1               5

<210>  154<211>  17<212>  PRT<213>  Homo sapiens<400>  154

Ala Ile Ser Gly Ser Gly Gly Ser Thr Tyr Tyr Ala Asp Ser Val Lys
1               5                   10                  15

Gly

<210>  155<211>  11<212>  PRT<213>  Homo sapiens<400>  155

Val Gly Lys Ala Thr Thr Glu Glu Gly Tyr Tyr
1               5                   10

<210>  156<211>  13<212>  PRT<213>  Homo sapiens<400>  156

Thr Arg Ser Ser Gly Ser Ile Ala Ser Asn Tyr Val Gln
1               5                   10

<210>  157<211>  7<212>  PRT<213>  Homo sapiens<400>  157

Asp Asp Asn Gln Arg Pro Ser
1               5

<210>  158<211>  9<212>  PRT<213>  Homo sapiens<400>  158

Gln Ser Tyr Asp Ser Asn Asn Asp Val
1               5

<210>  159<211>  15<212>  DNA<213>  Homo sapiens<400>  159
aattatggtc tcagc                                                        15

<210>  160<211>  51<212>  DNA<213>  Homo sapiens<400>  160
tggatcagcg ctaataatgg cgacacaaat tatggacagg aattccaggg c                51

<210>  161<211>  39<212>  DNA<213>  Homo sapiens<400>  161
gactccagca gcaactgggc ccgctggttt ttcgatctc                              39

<210>  162<211>  33<212>  DNA<213>  Homo sapiens<400>  162
gggggaaaca acattggaag taaacttgta cac                                    33

<210>  163<211>  21<212>  DNA<213>  Homo sapiens<400>  163
gatgatggcg accggccctc a                                                 21

<210>  164<211>  33<212>  DNA<213>  Homo sapiens<400>  164
caggtgtggg atactggtag tgatcccgtg gta                                    33

<210>  165<211>  15<212>  DNA<213>  Homo sapiens<400>  165
aattatggtc tcagc                                                        15

<210>  166<211>  51<212>  DNA<213>  Homo sapiens<400>  166
tggatcagcg ctaataatgg cgacacaaat tatggacagg aattccaggg c                51

<210>  167<211>  39<212>  DNA<213>  Homo sapiens<400>  167
gactccagca gcagctgggc ccgctggttt ttcgatctc                              39

<210>  168<211>  33<212>  DNA<213>  Homo sapiens<400>  168
gggggaaaca tcattggaag taaacttgta cac                                    33

<210>  169<211>  21<212>  DNA<213>  Homo sapiens<400>  169
gatgatggcg accggccctc a                                                 21

<210>  170<211>  33<212>  DNA<213>  Homo sapiens<400>  170
caggtgtggg atactggtag tgatcccgtg gta                                    33

<210>  171<211>  327<212>  PRT<213>  Homo sapiens<400>  171

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  172<211>  105<212>  PRT<213>  Homo sapiens<400>  172

Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Thr Val Ala Trp Lys Ala Asp Ser Ser Pro Val
        35                  40                  45

Lys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
            100                 105

<210>  173<211>  132<212>  PRT<213>  Homo sapiens<400>  173

Met Ala Leu Leu Leu Thr Thr Val Ile Ala Leu Thr Cys Leu Gly Gly
1               5                   10                  15

Phe Ala Ser Pro Gly Pro Val Pro Pro Ser Thr Ala Leu Arg Glu Leu
            20                  25                  30

Ile Glu Glu Leu Val Asn Ile Thr Gln Asn Gln Lys Ala Pro Leu Cys
        35                  40                  45

Asn Gly Ser Met Val Trp Ser Ile Asn Leu Thr Ala Gly Met Tyr Cys
    50                  55                  60

Ala Ala Leu Glu Ser Leu Ile Asn Val Ser Gly Cys Ser Ala Ile Glu
65                  70                  75                  80

Lys Thr Gln Arg Met Leu Ser Gly Phe Cys Pro His Lys Val Ser Ala
                85                  90                  95

Gly Gln Phe Ser Ser Leu His Val Arg Asp Thr Lys Ile Glu Val Ala
            100                 105                 110

Gln Phe Val Lys Asp Leu Leu Leu His Leu Lys Lys Leu Phe Arg Glu
        115                 120                 125

Gly Arg Phe Asn
    130

<210>  174<211>  132<212>  PRT<213>  Macaca fascicularis<400>  174

Met Ala Leu Leu Leu Thr Thr Val Ile Ala Leu Thr Cys Leu Gly Gly
1               5                   10                  15

Phe Ala Ser Pro Ser Pro Val Pro Pro Ser Thr Ala Leu Lys Glu Leu
            20                  25                  30

Ile Glu Glu Leu Val Asn Ile Thr Gln Asn Gln Lys Ala Pro Leu Cys
        35                  40                  45

Asn Gly Ser Met Val Trp Ser Ile Asn Leu Thr Ala Gly Val Tyr Cys
    50                  55                  60

Ala Ala Leu Glu Ser Leu Ile Asn Val Ser Gly Cys Ser Ala Ile Glu
65                  70                  75                  80

Lys Thr Gln Arg Met Leu Asn Gly Phe Cys Pro His Lys Val Ser Ala
                85                  90                  95

Gly Gln Phe Ser Ser Leu Arg Val Arg Asp Thr Lys Ile Glu Val Ala
            100                 105                 110

Gln Phe Val Lys Asp Leu Leu Val His Leu Lys Lys Leu Phe Arg Glu
        115                 120                 125

Gly Gln Phe Asn
    130

<210>  175<211>  131<212>  PRT<213>  Mus sp.<400>  175

Met Ala Leu Trp Val Thr Ala Val Leu Ala Leu Ala Cys Leu Gly Gly
1               5                   10                  15

Leu Ala Ala Pro Gly Pro Val Pro Arg Ser Val Ser Leu Pro Leu Thr
            20                  25                  30

Leu Lys Glu Leu Ile Glu Glu Leu Ser Asn Ile Thr Gln Asp Gln Thr
        35                  40                  45

Pro Leu Cys Asn Gly Ser Met Val Trp Ser Val Asp Leu Ala Ala Gly
    50                  55                  60

Gly Phe Cys Val Ala Leu Asp Ser Leu Thr Asn Ile Ser Asn Cys Asn
65                  70                  75                  80

Ala Ile Tyr Arg Thr Gln Arg Ile Leu His Gly Leu Cys Asn Arg Lys
                85                  90                  95

Ala Pro Thr Thr Val Ser Ser Leu Pro Asp Thr Lys Ile Glu Val Ala
            100                 105                 110

His Phe Ile Thr Lys Leu Leu Ser Tyr Thr Lys Gln Leu Phe Arg His
        115                 120                 125

Gly Pro Phe
    130

<210>  176<211>  5<212>  PRT<213>  Artificial sequence<220><223>  HCDR1
formula

<220><221>  MISC_FEATURE<222>  (1)..(1)<223>  Xaa is selected from the group
consisting of Asn, Gln, Asp, Leu,
       Gly and Glu

<220><221>  MISC_FEATURE<222>  (2)..(2)<223>  Xaa is selected from the group
consisting of Tyr and Thr

<220><221>  MISC_FEATURE<222>  (4)..(4)<223>  Xaa is selected from the group
consisting of Val, Ile, Phe and Le
       u

<400>  176

Xaa Xaa Gly Xaa Ser
1               5


<210>  177<211>  17<212>  PRT<213>  Artificial sequence<220><223>  HCDR2
formula

<220><221>  MISC_FEATURE<222>  (3)..(3)<223>  Xaa is selected from the group
consisting of Ser, Asp, Asn, Ala,
       Arg, Gly and Glu

<220><221>  MISC_FEATURE<222>  (4)..(4)<223>  Xaa is selected from the group
consisting of Ala, Asp, Gly, Thr,
       Pro, Asn and Tyr

<220><221>  MISC_FEATURE<222>  (5)..(5)<223>  Xaa is selected from the group
consisting of Asn, Asp, Leu, Ala,
       Pro, Thr, Ser, Ile and Arg

<220><221>  MISC_FEATURE<222>  (6)..(6)<223>  Xaa is selected from the group
consisting of Asn, Ser, Thr, Asp,
       Gly, Lys and Ile

<220><221>  MISC_FEATURE<222>  (8)..(8)<223>  Xaa is selected from the group
consisting of Asp, Thr, Glu, Gln,
       Leu, Tyr, Asn, Val, Ala, Met and Gly

<220><221>  MISC_FEATURE<222>  (10)..(10)<223>  Xaa is selected from the
group consisting of Asn, Ile, Leu, Gln,
       Ser, Met, His, Asp and Lys

<220><221>  MISC_FEATURE<222>  (12)..(12)<223>  Xaa is selected from the
group consisting of Gly and Arg

<220><221>  MISC_FEATURE<222>  (13)..(13)<223>  Xaa is selected from the
group consisting of Gln and Arg

<220><221>  MISC_FEATURE<222>  (14)..(14)<223>  Xaa is selected from the
group consisting of Glu, Lys and Gly

<220><221>  MISC_FEATURE<222>  (16)..(16)<223>  Xaa is selected from the
group consisting of Gln and Arg

<220><221>  MISC_FEATURE<222>  (17)..(17)<223>  Xaa is selected from the
group consisting of Gly and Lys

<400>  177

Trp Ile Xaa Xaa Xaa Xaa Gly Xaa Thr Xaa Tyr Xaa Xaa Xaa Phe Xaa
1               5                   10                  15

Xaa

<210>  178<211>  13<212>  PRT<213>  Artificial sequence<220><223>  HCDR3
formula

<220><221>  MISC_FEATURE<222>  (2)..(2)<223>  Xaa is selected from the group
consisting of Ser, Arg and Asp

<220><221>  MISC_FEATURE<222>  (3)..(3)<223>  Xaa is selected from the group
consisting of Ser, Asn, Asp, Thr a
       nd Pro

<220><221>  MISC_FEATURE<222>  (4)..(4)<223>  Xaa is selected from the group
consisting of Ser and Arg

<220><221>  MISC_FEATURE<222>  (5)..(5)<223>  Xaa is selected from the group
consisting of Ser, Asn, Ala, Ile,
       Arg, Pro and Lys

<220><221>  MISC_FEATURE<222>  (10)..(10)<223>  Xaa is selected from the
group consisting of Phe and Tyr

<220><221>  MISC_FEATURE<222>  (12)..(12)<223>  Xaa is selected from the
group consisting of Asp and Tyr

<400>  178

Asp Xaa Xaa Xaa Xaa Trp Ala Arg Trp Xaa Phe Xaa Leu
1               5                   10

<210>  179<211>  11<212>  PRT<213>  Artificial sequence<220><223>  LCDR1
formula

<220><221>  MISC_FEATURE<222>  (3)..(3)<223>  Xaa is selected from the group
consisting of Asn, Asp and Ser

<220><221>  MISC_FEATURE<222>  (4)..(4)<223>  Xaa is selected from the group
consisting of Asn, Ile, Leu, Met,
       Cys, Val, Lys, Tyr, Phe, Arg, Thr, Ser, Ala, His and Gly

<220><221>  MISC_FEATURE<222>  (5)..(5)<223>  Xaa is selected from the group
consisting of Ile and Val

<220><221>  MISC_FEATURE<222>  (7)..(7)<223>  Xaa is selected from the group
consisting of Ser and Gly

<220><221>  MISC_FEATURE<222>  (8)..(8)<223>  Xaa is selected from the group
consisting of Lys and Arg

<400>  179

Gly Gly Xaa Xaa Xaa Gly Xaa Xaa Leu Val His
1               5                   10

<210>  180<211>  7<212>  PRT<213>  Artificial sequence<220><223>  LCDR2
formula

<220><221>  MISC_FEATURE<222>  (7)..(7)<223>  Xaa is selected from the group
consisting of Ser and Thr

<400>  180

Asp Asp Gly Asp Arg Pro Xaa
1               5


<210>  181<211>  11<212>  PRT<213>  Artificial sequence<220><223>  LCDR3
formula

<220><221>  MISC_FEATURE<222>  (8)..(8)<223>  Xaa is selected from the group
consisting of Asp and Asn

<220><221>  MISC_FEATURE<222>  (11)..(11)<223>  Xaa is selected from the
group consisting of Val and Ile

<400>  181

Gln Val Trp Asp Thr Gly Ser Xaa Pro Val Xaa
1               5                   10


<210>  182<211>  5<212>  PRT<213>  Homo sapiens<400>  182

Leu Thr Gly Val Ser
1               5


<210>  183<211>  5<212>  PRT<213>  Homo sapiens<400>  183

Gly Thr Gly Val Ser
1               5


<210>  184<211>  5<212>  PRT<213>  Homo sapiens<400>  184

Glu Thr Gly Ile Ser
1               5


<210>  185<211>  5<212>  PRT<213>  Homo sapiens<400>  185

Asp Thr Gly Ile Ser
1               5


<210>  186<211>  5<212>  PRT<213>  Homo sapiens<400>  186

Gly Thr Gly Ile Ser
1               5


<210>  187<211>  5<212>  PRT<213>  Homo sapiens<400>  187

Asn Tyr Gly Phe Ser
1               5

<210>  188<211>  17<212>  PRT<213>  Homo sapiens<400>  188

Trp Ile Arg Pro Thr Asp Gly Leu Thr Met Tyr Gly Gln Glu Phe Gln
1               5                   10                  15

Gly

<210>  189<211>  17<212>  PRT<213>  Homo sapiens<400>  189

Trp Ile Asp Asp Arg Thr Gly Thr Thr Gln Tyr Gly Gln Glu Phe Gln
1               5                   10                  15

Gly

<210>  190<211>  17<212>  PRT<213>  Homo sapiens<400>  190

Trp Ile Arg Ala Ser Asp Gly Gln Thr Ile Tyr Gly Gln Glu Phe Gln
1               5                   10                  15

Gly

<210>  191<211>  17<212>  PRT<213>  Homo sapiens<400>  191

Trp Ile Ser Gly Ile Asp Gly Val Thr Leu Tyr Gly Gln Glu Phe Gln
1               5                   10                  15

Gly

<210>  192<211>  17<212>  PRT<213>  Homo sapiens<400>  192

Trp Ile Arg Ala Ala Asp Gly Glu Thr His Tyr Gly Gln Glu Phe Gln
1               5                   10                  15

Gly

<210>  193<211>  17<212>  PRT<213>  Homo sapiens<400>  193

Trp Ile Gly Asn Asn Asn Gly Asp Thr Leu Tyr Gly Gln Glu Phe Gln
1               5                   10                  15

Gly

<210>  194<211>  17<212>  PRT<213>  Homo sapiens<400>  194

Trp Ile Gly Pro Ser Lys Gly Glu Thr Ser Tyr Gly Gln Glu Phe Gln

1               5                   10                  15

Gly

<210>  195<211>  17<212>  PRT<213>  Homo sapiens<400>  195

Trp Ile Arg Pro Arg Asp Gly Thr Thr His Tyr Gly Gln Glu Phe Gln
1               5                   10                  15

Gly

<210>  196<211>  17<212>  PRT<213>  Homo sapiens<400>  196

Trp Ile Ser Gly Arg Ser Gly Ala Thr Leu Tyr Gly Gln Glu Phe Gln
1               5                   10                  15

Gly

<210>  197<211>  17<212>  PRT<213>  Homo sapiens<400>  197

Trp Ile Glu Gly Ser Thr Gly Asn Thr Ile Tyr Gly Gln Glu Phe Gln
1               5                   10                  15

Gly

<210>  198<211>  17<212>  PRT<213>  Homo sapiens<400>  198

Trp Ile Gly Pro Ile Asn Gly Met Thr His Tyr Gly Gln Glu Phe Gln
1               5                   10                  15

Gly

<210>  199<211>  17<212>  PRT<213>  Homo sapiens<400>  199

Trp Ile Ser Ala Asn Asn Gly Asp Thr Asn Tyr Gly Gln Lys Phe Gln
1               5                   10                  15

Gly

<210>  200<211>  17<212>  PRT<213>  Homo sapiens<400>  200

Trp Ile Ser Ala Asn Asn Gly Asp Thr Asp Tyr Gly Gln Glu Phe Gln
1               5                   10                  15

Gly

<210>  201<211>  17<212>  PRT<213>  Homo sapiens<400>  201

Trp Ile Ser Ala Asn Asn Gly Gly Thr Asn Tyr Gly Gln Glu Phe Gln
1               5                   10                  15

Gly

<210>  202<211>  17<212>  PRT<213>  Homo sapiens<400>  202

Trp Ile Ser Ala Asn Asn Gly Asp Thr Asn Tyr Arg Gln Glu Phe Gln
1               5                   10                  15

Gly

<210>  203<211>  17<212>  PRT<213>  Homo sapiens<400>  203

Trp Ile Gly Ala Asn Asn Gly Asp Thr Asn Tyr Gly Gln Glu Phe Gln
1               5                   10                  15

Gly

<210>  204<211>  17<212>  PRT<213>  Homo sapiens<400>  204

Trp Ile Ser Ala Asn Asn Gly Asp Thr Asn Tyr Gly Gln Glu Phe Arg
1               5                   10                  15

Gly

<210>  205<211>  17<212>  PRT<213>  Homo sapiens<400>  205

Trp Ile Ser Ala Asn Asn Gly Asp Thr Ile Tyr Gly Gln Glu Phe Gln
1               5                   10                  15

Gly

<210>  206<211>  17<212>  PRT<213>  Homo sapiens<400>  206

Trp Ile Ser Thr Asn Asn Gly Asp Thr Asn Tyr Gly Gln Glu Phe Gln
1               5                   10                  15

Gly

<210>  207<211>  17<212>  PRT<213>  Homo sapiens<400>  207

Trp Ile Ser Ala Asn Asn Gly Asp Thr Ile Tyr Arg Gln Glu Phe Gln
1               5                   10                  15

Gly

<210>  208<211>  17<212>  PRT<213>  Homo sapiens<400>  208

Trp Ile Ser Ala Asn Asn Gly Asp Thr Ser Tyr Gly Gln Glu Phe Gln
1               5                   10                  15

Gly

<210>  209<211>  17<212>  PRT<213>  Homo sapiens<400>  209

Trp Ile Ser Ala Asn Asn Gly Asp Thr Lys Tyr Gly Gln Glu Phe Gln
1               5                   10                  15

Gly

<210>  210<211>  17<212>  PRT<213>  Homo sapiens<400>  210

Trp Ile Gly Ala Asn Asn Gly Asp Thr Asp Tyr Gly Gln Glu Phe Gln
1               5                   10                  15

Gly

<210>  211<211>  17<212>  PRT<213>  Homo sapiens<400>  211

Trp Ile Ser Ala Asn Ile Gly Asp Thr Asn Tyr Gly Gln Glu Phe Gln
1               5                   10                  15

Gly

<210>  212<211>  17<212>  PRT<213>  Homo sapiens<400>  212

Trp Ile Ser Ala Asn Asn Gly Asp Thr Asn Tyr Gly Gln Gly Phe Gln
1               5                   10                  15

Gly

<210>  213<211>  17<212>  PRT<213>  Homo sapiens<400>  213

Trp Ile Ser Thr Asn Asn Gly Asp Thr Asn Tyr Gly Arg Glu Phe Gln
1               5                   10                  15

Gly

<210>  214<211>  17<212>  PRT<213>  Homo sapiens<400>  214

Trp Ile Ser Ala Asn Asn Gly Asp Thr Asn Tyr Gly Gln Glu Phe Gln
1               5                   10                  15

Lys

<210>  215<211>  13<212>  PRT<213>  Homo sapiens<400>  215

Asp Ser Asp Ser Ser Trp Ala Arg Trp Phe Phe Asp Leu
1               5                   10

<210>  216<211>  13<212>  PRT<213>  Homo sapiens<400>  216

Asp Ser Thr Ser Ala Trp Ala Arg Trp Phe Phe Asp Leu
1               5                   10

<210>  217<211>  13<212>  PRT<213>  Homo sapiens<400>  217

Asp Ser Asn Ser Ala Trp Ala Arg Trp Phe Phe Asp Leu
1               5                   10

<210>  218<211>  13<212>  PRT<213>  Homo sapiens<400>  218

Asp Ser Ser Ser Ile Trp Ala Arg Trp Phe Phe Asp Leu
1               5                   10

<210>  219<211>  13<212>  PRT<213>  Homo sapiens<400>  219

Asp Ser Thr Ser Arg Trp Ala Arg Trp Phe Phe Asp Leu
1               5                   10

<210>  220<211>  13<212>  PRT<213>  Homo sapiens<400>  220

Asp Asp Pro Arg Pro Trp Ala Arg Trp Phe Phe Asp Leu
1               5                   10

<210>  221<211>  13<212>  PRT<213>  Homo sapiens<400>  221

Asp Ser Ser Ser Lys Trp Ala Arg Trp Phe Phe Asp Leu
1               5                   10

<210>  222<211>  13<212>  PRT<213>  Homo sapiens<400>  222

Asp Ser Asn Ser Asn Trp Ala Arg Trp Phe Phe Tyr Leu

1               5                   10

<210>  223<211>  13<212>  PRT<213>  Homo sapiens<400>  223

Asp Ser Asn Ser Ser Trp Ala Arg Trp Phe Phe Asp Leu
1               5                   10

<210>  224<211>  13<212>  PRT<213>  Homo sapiens<400>  224

Asp Arg Asp Ser Ser Trp Ala Arg Trp Phe Phe Asp Leu
1               5                   10

<210>  225<211>  11<212>  PRT<213>  Homo sapiens<400>  225

Gly Gly Asn Leu Ile Gly Ser Lys Leu Val His
1               5                   10

<210>  226<211>  11<212>  PRT<213>  Homo sapiens<400>  226

Gly Gly Asn Met Ile Gly Ser Lys Leu Val His
1               5                   10

<210>  227<211>  11<212>  PRT<213>  Homo sapiens<400>  227

Gly Gly Asn Cys Ile Gly Ser Lys Leu Val His
1               5                   10

<210>  228<211>  11<212>  PRT<213>  Homo sapiens<400>  228

Gly Gly Asn Val Ile Gly Ser Lys Leu Val His
1               5                   10

<210>  229<211>  11<212>  PRT<213>  Homo sapiens<400>  229

Gly Gly Asn Lys Ile Gly Ser Lys Leu Val His
1               5                   10

<210>  230<211>  11<212>  PRT<213>  Homo sapiens<400>  230

Gly Gly Asn Tyr Ile Gly Ser Lys Leu Val His
1               5                   10

<210>  231<211>  11<212>  PRT<213>  Homo sapiens<400>  231

Gly Gly Asn Phe Ile Gly Ser Lys Leu Val His
1               5                   10

<210>  232<211>  11<212>  PRT<213>  Homo sapiens<400>  232

Gly Gly Asn Arg Ile Gly Ser Lys Leu Val His
1               5                   10

<210>  233<211>  11<212>  PRT<213>  Homo sapiens<400>  233

Gly Gly Asn Thr Ile Gly Ser Lys Leu Val His
1               5                   10

<210>  234<211>  11<212>  PRT<213>  Homo sapiens<400>  234

Gly Gly Asn Asn Ile Gly Gly Lys Leu Val His
1               5                   10

<210>  235<211>  11<212>  PRT<213>  Homo sapiens<400>  235

Gly Gly Asn Ser Ile Gly Ser Arg Leu Val His
1               5                   10

<210>  236<211>  11<212>  PRT<213>  Homo sapiens<400>  236

Gly Gly Asp Asn Ile Gly Gly Lys Leu Val His
1               5                   10

<210>  237<211>  11<212>  PRT<213>  Homo sapiens<400>  237

Gly Gly Asn Ser Ile Gly Gly Lys Leu Val His
1               5                   10

<210>  238<211>  11<212>  PRT<213>  Homo sapiens<400>  238

Gly Gly Asn Ala Ile Gly Ser Lys Leu Val His
1               5                   10

<210>  239<211>  11<212>  PRT<213>  Homo sapiens<400>  239

Gly Gly Asn Ser Ile Gly Ser Lys Leu Val His
1               5                   10

<210>  240<211>  11<212>  PRT<213>  Homo sapiens<400>  240

Gly Gly Asn His Ile Gly Ser Lys Leu Val His
1               5                   10

<210>  241<211>  11<212>  PRT<213>  Homo sapiens<400>  241

Gly Gly Asn Gly Ile Gly Ser Lys Leu Val His
1               5                   10

<210>  242<211>  11<212>  PRT<213>  Homo sapiens<400>  242

Gly Gly Ser Asn Ile Gly Gly Lys Leu Val His
1               5                   10

<210>  243<211>  11<212>  PRT<213>  Homo sapiens<400>  243

Gly Gly Asn Asn Val Gly Gly Lys Leu Val His

1               5                   10

<210>  244<211>  11<212>  PRT<213>  Homo sapiens<400>  244

Gly Gly Asn Asn Ile Gly Ser Arg Leu Val His
1               5                   10

<210>  245<211>  7<212>  PRT<213>  Homo sapiens<400>  245

Asp Asp Gly Asp Arg Pro Thr
1               5

<210>  246<211>  11<212>  PRT<213>  Homo sapiens<400>  246

Gln Val Trp Asp Thr Gly Ser Asn Pro Val Val
1               5                   10

<210>  247<211>  11<212>  PRT<213>  Homo sapiens<400>  247

Gln Val Trp Asp Thr Gly Ser Asp Pro Val Ile
1               5                   10

<210>  248<211>  132<212>  PRT<213>  Artificial sequence<220><223>
Consensus between human IL-3 and Cynomolgus IL-3

<220><221>  MISC_FEATURE<222>  (21, 30, 62, 87, 103, 120, 130)<223>  Xaa =
no consensus

<400>  248

Met Ala Leu Leu Leu Thr Thr Val Ile Ala Leu Thr Cys Leu Gly Gly
1               5                   10                  15

Phe Ala Ser Pro Xaa Pro Val Pro Pro Ser Thr Ala Leu Xaa Glu Leu
            20                  25                  30

Ile Glu Glu Leu Val Asn Ile Thr Gln Asn Gln Lys Ala Pro Leu Cys
        35                  40                  45

Asn Gly Ser Met Val Trp Ser Ile Asn Leu Thr Ala Gly Xaa Tyr Cys
    50                  55                  60

Ala Ala Leu Glu Ser Leu Ile Asn Val Ser Gly Cys Ser Ala Ile Glu
65                  70                  75                  80

Lys Thr Gln Arg Met Leu Xaa Gly Phe Cys Pro His Lys Val Ser Ala
                85                  90                  95

Gly Gln Phe Ser Ser Leu Xaa Val Arg Asp Thr Lys Ile Glu Val Ala
            100                 105                 110

Gln Phe Val Lys Asp Leu Leu Xaa His Leu Lys Lys Leu Phe Arg Glu
        115                 120                 125

Gly Xaa Phe Asn
    130

<210>  249<211>  136<212>  PRT<213>  Artificial sequence<220><223>
Consensus between human IL-3 and murine IL-3

<220><221>  MISC_FEATURE<222>  (4, 5, 7, 9, 12, 17, 19, 25, 27..32, 34, 41,
46, 48)<223>  Xaa = no consensus

<220><221>  MISC_FEATURE<222>  (49, 60, 61, 63, 66, 67, 69, 72, 75, 77, 79,
81, 84)<223>  Xaa = no consensus

<220><221>  MISC_FEATURE<222>  (85, 89, 91, 93, 95, 96, 98..103, 107..109,
117)<223>  Xaa = no consensus

<220><221>  MISC_FEATURE<222>  (119..121, 124..126, 128, 132, 134, 136)<223>
Xaa = no consensus

<400>  249

Met Ala Leu Xaa Xaa Thr Xaa Val Xaa Ala Leu Xaa Cys Leu Gly Gly
1               5                   10                  15

Xaa Ala Xaa Pro Gly Pro Val Pro Xaa Ser Xaa Xaa Xaa Xaa Xaa Xaa
            20                  25                  30

Leu Xaa Glu Leu Ile Glu Glu Leu Xaa Asn Ile Thr Gln Xaa Gln Xaa
        35                  40                  45

Xaa Pro Leu Cys Asn Gly Ser Met Val Trp Ser Xaa Xaa Leu Xaa Ala
    50                  55                  60

Gly Xaa Xaa Cys Xaa Ala Leu Xaa Ser Leu Xaa Asn Xaa Ser Xaa Cys
65                  70                  75                  80

Xaa Ala Ile Xaa Xaa Thr Gln Arg Xaa Leu Xaa Gly Xaa Cys Xaa Xaa
                85                  90                  95

Lys Xaa Xaa Xaa Xaa Xaa Xaa Ser Ser Leu Xaa Xaa Xaa Asp Thr Lys
            100                 105                 110

Ile Glu Val Ala Xaa Phe Xaa Xaa Xaa Leu Leu Xaa Xaa Xaa Lys Xaa
        115                 120                 125

Leu Phe Arg Xaa Gly Xaa Phe Xaa
    130                 135

73

## Claims

1. An IL-13 antagonist for use in improving pulmonary function in a subject with impaired pulmonary function, wherein the IL-13 antagonist is an anti-human-IL-13 antibody or a human-IL-13-binding fragment thereof, selected from the group consisting of:
(a) an anti-human-IL-13 antibody or a human-IL-13 binding fragment thereof comprising a VH domain comprising HCDR1, HCDR2 and HCDR3 and a VL domain comprising LCDR1, LCDR2 and LCDR3, wherein HCDR1 has the amino acid sequence of SEQ ID NO: 7, HCDR2 has the amino acid sequence of SEQ ID NO: 8, HCDR3 has the amino acid sequence of SEQ ID NO: 9, LCDR1 has the amino acid sequence of SEQ ID NO: 10, LCDR2 has the amino acid sequence of SEQ ID NO: 11, and LCDR3 has the amino acid sequence of SEQ ID NO: 12;
(b) an anti-human-IL-13 antibody, or a human-IL-13-binding fragment thereof comprising a VH domain having the amino acid sequence of SEQ ID NO: 15 and a VL domain having the amino acid sequence of SEQ ID NO: 16; and
(c) an anti-human-IL-13 antibody or a human-IL-13-binding fragment thereof according to (a) or (b), further comprising a human IgG4 constant domain;
wherein improvement in pulmonary function is an improvement in Forced Expiratory Volume in One Second (FEV1) score of between 5% and 18% over a baseline FEV1 measurement taken before administering the anti-human-IL-13 antibody or a human-IL-13-binding fragment thereof, and wherein the IL-13 antibody or IL-13 binding fragment thereof is administered subcutaneously at a dose between 100 and 600 mg.

2. The IL-13 antagonist for use according to claim 1, wherein said antagonist is selected from the group consisting of; an immunoglobulin molecule, a monoclonal antibody, a chimeric antibody, a CDR-grafted antibody, a humanized antibody, a Fab, a Fab', a F(ab')2, a Fv, a disulfide linked Fv, a scFv, a diabody, a multispecific antibody, a dual-specific antibody, and a bispecific antibody.

3. The IL-13 antagonist for use according to any one of the preceding claims, wherein the subject has impaired pulmonary function associated with asthma, COPD, or idiopathic pulmonary fibrosis.

4. The IL-13 antagonist for use according to any one of the preceding claims, wherein the subject has impaired pulmonary function associated with asthma selected from atopic asthma, non-atopic asthma, moderate to severe asthma, moderate to severe asthma uncontrolled by inhaled or oral corticosteroids and asthma at GINA score 5, 4, 3, 2, or 1.

5. The IL-13 antagonist for use according to any one of the preceding claims, wherein the subject has impaired pulmonary function associated with asthma and is undergoing concomitant therapy with one or more further therapeutic selected from an inhaled corticosteroid, long-acting beta2 antagonist (LABA), theophylline, a leukotriene antagonist, and an oral corticosteroid.

6. The IL-13 antagonist for use according to any one of the preceding claims, wherein the IL-13 antibody or IL-13 binding fragment thereof is administered subcutaneously at a dose selected from the group consisting of 150, 300, and 600 mg.

7. The IL-13 antagonist for use according to any one of the preceding claims, wherein the IL-13 antibody or IL-13-binding fragment thereof is administered at a frequency selected from every 1, 2, 3, 4, 6, 8, 10 and 12 weeks.

8. The IL-13 antagonist for use according to any one of the preceding claims, wherein an improvement in pulmonary function in asthma subjects can be detected by 15 days following initiation of treatment.

9. The IL-13 antagonist for use according to any one of the preceding claims, wherein an improvement in pulmonary function in asthma subjects can be detected at least 12 weeks after cessation of treatment.

10. The IL-13 antagonist for use according to any one of claims 1 to 9, wherein the IL-13 antibody or IL-13-binding fragment thereof is administered by subcutaneous administration, at a dose of 300mg and at dosing intervals of 2 weeks or 4 weeks.

11. The IL-13 antagonist for use according to any one claims 1 to 9, wherein the improvement in pulmonary function is an improvement in FEV1 score of between 8% and 16% over a baseline FEV1 measurement taken before subcutaneously administering the anti-human-IL-13 antibody or a human-IL-13-binding fragment thereof at two week or four week intervals.

12. The IL-13 antagonist for use according to any one claims 1 to 9, wherein the improvement in pulmonary function is an improvement in FEV1 score of between 12% and 18% over a baseline FEV1 measurement taken before administering the anti-human-IL-13 antibody or a human-IL-13-binding fragment thereof.

13. The IL-13 antagonist for use according to any one of claims 1 to 9, wherein the improvement in pulmonary function is an improvement in FEV1 score of 8% over a baseline FEV1 measurement taken before subcutaneously administering 150mg of the anti-human-IL-13 antibody or a human-IL-13-binding fragment thereof at two week intervals; or
wherein the improvement in pulmonary function is an improvement in FEV1 score of 13.3% over a baseline FEV1 measurement taken before subcutaneously administering 300mg of the anti-human-IL-13 antibody or a human-IL-13-binding fragment thereof at two week intervals; or
wherein the improvement in pulmonary function is an improvement in FEV1 score of 15.2% over a baseline FEV1 measurement taken before subcutaneously administering 600mg of the anti-human-IL-13 antibody or a human-IL-13-binding fragment thereof at two week intervals.

14. The IL-13 antagonist for use according to any one of the preceding claims, wherein the IL-13 antagonist is an anti-human-IL-13 antibody or a human-IL-13 binding fragment thereof comprising a VH domain comprising HCDR1, HCDR2 and HCDR3 and a VL domain comprising LCDR1, LCDR2 and LCDR3, wherein HCDR1 has the amino acid sequence of SEQ ID NO: 7, HCDR2 has the amino acid sequence of SEQ ID NO: 8, HCDR3 has the amino acid sequence of SEQ ID NO: 9, LCDR1 has the amino acid sequence of SEQ ID NO: 10, LCDR2 has the amino acid sequence of SEQ ID NO: 11, and LCDR3 has the amino acid sequence of SEQ ID NO: 12.

15. The IL-13 antagonist for use according to any one of the preceding claims, wherein the IL-13 antagonist is an anti-human-IL-13 antibody or a human-IL-13 binding fragment thereof comprising a VH domain having the amino acid sequence of SEQ ID NO: 15 and a VL domain having the amino acid sequence of SEQ ID NO: 16.

16. The IL-13 antagonist for use according to any one of the preceding claims, wherein the improvement in pulmonary function is an improvement in FEV1 score of between 5% and 12% over a baseline FEV1 measurement taken before administering the anti-human-IL-13 antibody or a human-IL-13-binding fragment thereof.

## Patentansprüche

1. IL-13-Antagonist zur Verwendung bei der Verbesserung der Lungenfunktion bei einem Individuum mit beeinträchtigter Lungenfunktion, wobei es sich bei dem IL-13-Antagonisten um einen Antikörper gegen menschliches IL-13 oder ein menschliches IL-13 bindendes Fragment davon handelt, ausgewählt aus der Gruppe bestehend aus:
(a) einem Antikörper gegen menschliches IL-13 bzw. einem menschliches IL-13 bindenden Fragment, der bzw. das eine HCDR1, HCDR2 und HCDR3 umfassende VH-Domäne und eine LCDR1, LCDR2 und LCDR3 umfassende VL-Domäne umfasst, wobei HCDR1 die Aminosäuresequenz unter SEQ ID NO: 7, HCDR2 die Aminosäuresequenz unter SEQ ID NO: 8, HCDR3 die Aminosäuresequenz unter SEQ ID NO: 9, LCDR1 die Aminosäuresequenz unter SEQ ID NO: 10, LCDR2 die Aminosäuresequenz unter SEQ ID NO: 11 und LCDR3 die Aminosäuresequenz unter SEQ ID NO: 12 aufweist;
(b) einem Antikörper gegen menschliches IL-13 bzw. einem menschliches IL-13 bindenden Fragment davon, der bzw. das eine VH-Domäne mit der Aminosäuresequenz unter SEQ ID NO: 15 und eine VL-Domäne mit der Aminosäuresequenz unter SEQ ID NO: 16 umfasst; und
(c) einem Antikörper gegen menschliches IL-13 bzw. einem menschliches IL-13 bindenden Fragment davon gemäß (a) oder (b), der bzw. das ferner eine konstante Domäne von menschlichem IgG4 umfasst;
wobei es sich bei der Verbesserung der Lungenfunktion um eine Verbesserung des FEV1 (Forced Expiratory Volume in One Second)-Werts um zwischen 5% und 18% gegenüber einer FEV1-Basislinienmes-sung, die vor Verabreichen des Antikörpers gegen menschliches IL-13 bzw. eines menschliches IL-13 bindenden Fragments davon vorgenommen wurde, handelt und wobei der IL-13-Antikörper bzw. das IL-13 bindende Fragment davon in einer Dosis zwischen 100 und 600 mg subkutan verabreicht wird.

2. IL-13-Antagonist zur Verwendung nach Anspruch 1, wobei der Antagonist aus der aus einem Immunglobulinmolekül, einem monoklonalen Antikörper, einem chimären Antikörper, einem CDR-gepfropften Antikörper, einem humanisierten Antikörper, einem Fab, einem Fab', einem F(ab')2, einem Fv, einem disulfidverknüpften Fv, einem scFv, einem Diabody, einem multispezifischen Antikörper, einem Antikörper mit dualer Spezifität und einem bispezifischen Antikörper bestehenden Gruppe ausgewählt ist.

3. IL-13-Antagonist zur Verwendung nach einem der vorhergehenden Ansprüche, wobei das Individuum eine beeinträchtigte Lungenfunktion in Zusammenhang mit Asthma, COPD oder idiopathischer Lungenfibrose aufweist.

4. IL-13-Antagonist zur Verwendung nach einem der vorhergehenden Ansprüche, wobei das Individuum eine beeinträchtigte Lungenfunktion in Zusammenhang mit aus atopischem Asthma, nicht atopischem Asthma, mittelschwerem bis schwerem Asthma, mittelschwerem bis schwerem Asthma, das nicht unter Kontrolle durch inhalierte oder orale Corticosteroide steht, und Asthma mit einem GINA-Wert von 5, 4, 3, 2 oder 1 ausgewähltem Asthma aufweist.

5. IL-13-Antagonist zur Verwendung nach einem der vorhergehenden Ansprüche, wobei das Individuum eine beeinträchtigte Lungenfunktion in Zusammenhang mit Asthma aufweist und eine gleichzeitige Therapie mit einem oder mehreren weiteren Therapeutika durchmacht, die aus einem inhalierten Corticosteroid, Beta2-Antagonisten mit Langzeitwirkung (LABA), Theophyllin, einem Leukotrienantagonisten und einem oralen Corticosteroid ausgewählt sind.

6. IL-13-Antagonist zur Verwendung nach einem der vorhergehenden Ansprüche, wobei der IL-13-Antikörper bzw. das IL-13 bindende Fragment davon in einer aus der aus 150, 300 und 600 mg bestehenden Gruppe ausgewählten Dosis subkutan verabreicht wird.

7. IL-13-Antagonist zur Verwendung nach einem der vorhergehenden Ansprüche, wobei der IL-13-Antikörper bzw. das IL-13 bindende Fragment davon mit einer aus alle 1, 2, 3, 4, 6, 8, 10 und 12 Wochen ausgewählten Häufigkeit verabreicht wird.

8. IL-13-Antagonist zur Verwendung nach einem der vorhergehenden Ansprüche, wobei eine Verbesserung der Lungenfunktion bei Individuen mit Asthma 15 Tage nach Behandlungsbeginn nachgewiesen werden kann.

9. IL-13-Antagonist zur Verwendung nach einem der vorhergehenden Ansprüche, wobei eine Verbesserung der Lungenfunktion bei Individuen mit Asthma wenigstens 12 Wochen nach Behandlungsende nachgewiesen werden kann.

10. IL-13-Antagonist zur Verwendung nach einem der Ansprüche 1 bis 9, wobei der IL-13-Antikörper bzw. das IL-13 bindende Fragment davon durch subkutane Verabreichung bei einer Dosis von 300 mg und Dosierungsintervallen von 2 Wochen oder 4 Wochen verabreicht wird.

11. IL-13-Antagonist zur Verwendung nach einem der Ansprüche 1 bis 9, wobei es sich bei der Verbesserung der Lungenfunktion um eine Verbesserung des FEV1-Werts um zwischen 8% und 16% gegenüber einer FEV1-Basislinienmessung, die vor subkutanem Verabreichen des Antikörpers gegen menschliches IL-13 bzw. eines menschliches IL-13 bindenden Fragments davon in Zeitabständen von zwei oder vier Wochen vorgenommen wurde, handelt.

12. IL-13-Antagonist zur Verwendung nach einem der Ansprüche 1 bis 9, wobei es sich bei der Verbesserung der Lungenfunktion um eine Verbesserung des FEV1-Werts um zwischen 12% und 18% gegenüber einer FEV1-Basislinienmessung, die vor Verabreichen des Antikörpers gegen menschliches IL-13 bzw. eines menschliches IL-13 bindenden Fragments davon vorgenommen wurde, handelt.

13. IL-13-Antagonist zur Verwendung nach einem der Ansprüche 1 bis 9, wobei es sich bei der Verbesserung der Lungenfunktion um eine Verbesserung des FEV1-Werts um 8% gegenüber einer FEV1-Basis-linienmessung, die vor subkutanem Verabreichen von 150 mg des Antikörpers gegen menschliches IL-13 bzw. eines menschliches IL-13 bindenden Fragments davon in zweiwöchigen Zeitabständen vorgenommen wurde, handelt; oder
wobei es sich bei der Verbesserung der Lungenfunktion um eine Verbesserung des FEV1-Werts um 13,3% gegenüber einer FEV1-Basislinienmessung, die vor subkutanem Verabreichen von 300 mg des Antikörpers gegen menschliches IL-13 bzw. eines menschliches IL-13 bindenden Fragments davon in zweiwöchigen Zeitabständen vorgenommen wurde, handelt; oder
wobei es sich bei der Verbesserung der Lungenfunktion um eine Verbesserung des FEV1-Werts um 15,2% gegenüber einer FEV1-Basislinienmessung, die vor subkutanem Verabreichen von 600 mg des Antikörpers gegen menschliches IL-13 bzw. eines menschliches IL-13 bindenden Fragments davon in zweiwöchigen Zeitabständen vorgenommen wurde, handelt.

14. IL-13-Antagonist zur Verwendung nach einem der vorhergehenden Ansprüche, wobei es sich bei dem IL-13-Antagonisten um einen Antikörper gegen menschliches IL-13 bzw. ein menschliches IL-13 bindendes Fragment davon handelt, der bzw. das eine HCDR1, HCDR2 und HCDR3 umfassende VH-Domäne und eine LCDR1, LCDR2 und LCDR3 umfassende VL-Domäne umfasst, wobei HCDR1 die Aminosäuresequenz unter SEQ ID NO: 7, HCDR2 die Aminosäuresequenz unter SEQ ID NO: 8, HCDR3 die Aminosäuresequenz unter SEQ ID NO: 9, LCDR1 die Aminosäuresequenz unter SEQ ID NO: 10, LCDR2 die Aminosäuresequenz unter SEQ ID NO: 11 und LCDR3 die Aminosäuresequenz unter SEQ ID NO: 12 aufweist.

15. IL-13-Antagonist zur Verwendung nach einem der vorhergehenden Ansprüche, wobei es sich bei dem IL-13-Antagonisten um einen Antikörper gegen menschliches IL-13 bzw. ein menschliches IL-13 bindendes Fragment davon handelt, der bzw. das eine VH-Domäne mit der Aminosäuresequenz unter SEQ ID NO: 15 und eine VL-Domäne mit der Aminosäuresequenz unter SEQ ID NO: 16 umfasst.

16. IL-13-Antagonist zur Verwendung nach einem der vorhergehenden Ansprüche, wobei es sich bei der Verbesserung der Lungenfunktion um eine Verbesserung des FEV1-Werts um zwischen 5% und 12% gegenüber einer FEV1-Basislinienmessung, die vor Verabreichen des Antikörpers gegen menschliches IL-13 bzw. eines menschliches IL-13 bindenden Fragments davon vorgenommen wurde, handelt.

## Revendications

1. Antagoniste de l'IL-13 destiné à être utilisé dans une amélioration de la fonction pulmonaire chez un sujet atteint d'une fonction pulmonaire altérée, l'antagoniste de l'IL-13 étant un anticorps anti-IL-13 humaine ou un fragment de celui-ci de liaison à l'IL-13 humaine, choisi dans le groupe constitué par :
(a) un anticorps anti-IL-13 humaine ou un fragment de celui-ci de liaison à l'IL-13 humaine comprenant un domaine VH, qui comprend des HCDR1, HCDR2 et HCDR3, ainsi qu'un domaine VL qui comprend des LCDR1, LCDR2 et LCDR3, dans lequel le HCDR1 a la séquence d'acides aminés de SEQ ID n° : 7, le HCDR2 a la séquence d'acides aminés de SEQ ID n° : 8, le HCDR3 a la séquence d'acides aminés de SEQ ID n° : 9, le LCDR1 a la séquence d'acides aminés de SEQ ID n° : 10, le LCDR2 a la séquence d'acides aminés de SEQ ID n° : 11 et le LCDR3 a la séquence d'acides aminés de SEQ ID n° : 12 ;
(b) un anticorps anti-IL-13 humaine ou un fragment de celui-ci de liaison à l'IL-13 humaine comprenant un domaine VH, ayant la séquence d'acides aminés de SEQ ID n° : 15, et un domaine VL ayant la séquence d'acides aminés de SEQ ID n° : 16 ; et
(c) un anticorps anti-IL-13 humaine ou un fragment de celui-ci de liaison à l'IL-13 humaine, selon le paragraphe (a) ou (b), comprenant en outre un domaine constant d'IgG4 humaine ;
dans lequel une amélioration de la fonction pulmonaire est une amélioration du score du Volume Expiratoire Maximal par Seconde (VEMS1), comprise entre 5% et 18% par rapport à une mesure de base de la VEMS1 prise avant l'administration de l'anticorps anti-IL-13 humaine ou d'un fragment de celui-ci de liaison à l'IL-13 humaine, et l'anticorps anti-IL-13 ou un fragment de celui-ci de liaison à l'IL-13 étant administré par voie sous-cutanée en une dose comprise entre 100 et 600 mg.

2. Antagoniste de l'IL-13 destiné à être utilisé selon la revendication 1, ledit antagoniste étant choisi dans le groupe constitué par : une molécule d'immunoglobuline, un anticorps monoclonal, un anticorps chimère, un anticorps à CDR greffé, un anticorps humanisé, un Fab, un Fab', un F(ab')2, un Fv, un Fv lié par liaison disulfure, un Fvsc, un diabody, un anticorps multi-spécifique, un anticorps double spécifique et un anticorps bispécifique.

3. Antagoniste de l'IL-13 destiné à être utilisé selon l'une quelconque des revendications précédentes, dans lequel le sujet a une fonction pulmonaire altérée associée à l'asthme, à une MPOC ou à une fibrose pulmonaire idiopathique.

4. Antagoniste de l'IL-13 destiné à être utilisé selon l'une quelconque des revendications précédentes, dans lequel le sujet a une fonction pulmonaire altérée associée à l'asthme, choisie parmi un asthme atopique, un asthme non atopique, un asthme modéré à grave, un asthme modéré à grave non contrôlé par des corticostéroïdes inhalés ou oraux, ainsi qu'un asthme étant à un niveau du score GINA de 5, 4, 3, 2 ou 1.

5. Antagoniste de l'IL-13 destiné à être utilisé selon l'une quelconque des revendications précédentes, dans lequel le sujet a une fonction pulmonaire altérée associée à l'asthme et subit une thérapie concomitante avec une ou plusieurs substance(s) thérapeutique(s) supplémentaire(s), choisie(s) parmi un corticostéroïde inhalé, un antagoniste bêta2 à action prolongée (LABA), la théophylline, un antagoniste des leucotriènes et un corticostéroïde oral.

6. Antagoniste de l'IL-13 destiné à être utilisé selon l'une quelconque des revendications précédentes, dans lequel l'anticorps anti-IL-13 ou un fragment de celui-ci de liaison à l'IL-13 est administré par voie sous-cutanée en une dose choisie dans le groupe constitué par 150, 300 et 600 mg.

7. Antagoniste de l'IL-13 destiné à être utilisé selon l'une quelconque des revendications précédentes, dans lequel l'anticorps anti-IL-13 ou un fragment de celui-ci de liaison à l'IL-13 est administré à une fréquence choisie parmi chaque 1, 2, 3, 4, 6, 8, 10 et 12 semaines.

8. Antagoniste de l'IL-13 destiné à être utilisé selon l'une quelconque des revendications précédentes, dans lequel une amélioration de la fonction pulmonaire chez des sujets atteints d'asthme peut être détectée 15 jours après le début du traitement.

9. Antagoniste de l'IL-13 destiné à être utilisé selon l'une quelconque des revendications précédentes, dans lequel une amélioration de la fonction pulmonaire chez des sujets atteints d'asthme peut être détectée au moins 12 semaines après un arrêt du traitement.

10. Antagoniste de l'IL-13 destiné à être utilisé selon l'une quelconque des revendications 1 à 9, dans lequel l'anticorps anti-IL-13 ou un fragment de celui-ci de liaison à l'IL-13 est administré par une administration sous-cutanée, en une dose de 300 mg et à des intervalles de doses de 2 semaines ou de 4 semaines.

11. Antagoniste de l'IL-13 destiné à être utilisé selon l'une quelconque des revendications 1 à 9, dans lequel l'amélioration de la fonction pulmonaire est une amélioration du score du VEMS1 comprise entre 8% et 16% par rapport à une mesure de base de la VEMS1 prise avant une administration sous-cutanée de l'anticorps anti-IL-13 humaine ou d'un fragment de celui-ci de liaison à l'IL-13 humaine à des intervalles de deux semaines ou de quatre semaines.

12. Antagoniste de l'IL-13 destiné à être utilisé selon l'une quelconque des revendications 1 à 9, dans lequel l'amélioration de la fonction pulmonaire est une amélioration du score du VEMS1 comprise entre 12% et 18% par rapport à une mesure de base de la VEMS1 prise avant une administration de l'anticorps anti-IL-13 humaine ou d'un fragment de celui-ci de liaison à l'IL-13 humaine.

13. Antagoniste de l'IL-13 destiné à être utilisé selon l'une quelconque des revendications 1 à 9, dans lequel l'amélioration de la fonction pulmonaire est une amélioration du score du VEMS1 de 8% par rapport à une mesure de base de la VEMS1 prise avant une administration sous-cutanée de 150 mg de l'anticorps anti-IL-13 humaine ou d'un fragment de celui-ci de liaison à l'IL-13 humaine à des intervalles de deux semaines ; ou
dans lequel l'amélioration de la fonction pulmonaire est une amélioration du score du VEMS1 de 13,3% par rapport à une mesure de base de la VEMS1 prise avant une administration sous-cutanée de 300 mg de l'anticorps anti-IL-13 humaine ou d'un fragment de celui-ci de liaison à l'IL-13 humaine à des intervalles de deux semaines ; ou
dans lequel l'amélioration de la fonction pulmonaire est une amélioration du score du VEMS1 de 15,2% par rapport à une mesure de base de la VEMS1 prise avant une administration sous-cutanée de 600 mg de l'anticorps anti-IL-13 humaine ou d'un fragment de celui-ci de liaison à l'IL-13 humaine à des intervalles de deux semaines.

14. Antagoniste de l'IL-13 destiné à être utilisé selon l'une quelconque des revendications précédentes, l'antagoniste de l'IL-13 étant un anticorps anti-IL-13 humaine ou un fragment de celui-ci de liaison à l'IL-13 humaine comprenant un domaine VH, qui comprend des HCDR1, HCDR2 et HCDR3, ainsi qu'un domaine VL qui comprend des LCDR1, LCDR2 et LCDR3, dans lequel le HCDR1 a la séquence d'acides aminés de SEQ ID n° : 7, le HCDR2 a la séquence d'acides aminés de SEQ ID n° : 8, le HCDR3 a la séquence d'acides aminés de SEQ ID n° : 9, le LCDR1 a la séquence d'acides aminés de SEQ ID n° : 10, le LCDR2 a la séquence d'acides aminés de SEQ ID n° : 11 et le LCDR3 a la séquence d'acides aminés de SEQ ID n° : 12.

15. Antagoniste de l'IL-13 destiné à être utilisé selon l'une quelconque des revendications précédentes, l'antagoniste de l'IL-13 étant un anticorps anti-IL-13 humaine ou un fragment de celui-ci de liaison à l'IL-13 humaine comprenant un domaine VH, ayant la séquence d'acides aminés de SEQ ID n° : 15, et un domaine VL ayant la séquence d'acides aminés de SEQ ID n° : 16.

16. Antagoniste de l'IL-13 destiné à être utilisé selon l'une quelconque des revendications précédentes, dans lequel l'amélioration de la fonction pulmonaire est une amélioration du score du VEMS1 comprise entre 5% et 12% par rapport à une mesure de base de la VEMS1 prise avant une administration de l'anticorps anti-IL-13 humaine ou d'un fragment de celui-ci de liaison à l'IL-13 humaine.
